(19)

Europäisches
Patentamt

European
Patent Office

Office européen
des brevets

(11) **EP 4 623 819 A1**

(12) **EUROPEAN PATENT APPLICATION**

(43) Date of publication:
**01.10.2025 Bulletin 2025/40**

(21) Application number: **25160661.2**

(22) Date of filing: **27.02.2025**

(51) International Patent Classification (IPC):
**A61B 5/11** *(2006.01)* **A61B 5/103** *(2006.01)*
**A61B 5/00** *(2006.01)*

(52) Cooperative Patent Classification (CPC):
**A61B 5/112; A61B 5/6807; A61B 5/7267;**
**G16H 40/67;** A61B 5/1038; G16H 50/20

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **28.03.2024 US 202418619845**

(71) Applicant: **The University of Ottawa**
**Ottawa, Ontario K1N 6N5 (CA)**

(72) Inventors:
• **GRAHAM, Ryan**
**Ottawa, ON K1N 6N5 (CA)**
• **MAVOR, Matthew**
**Ottawa, ON K1N 6N5 (CA)**
• **CHAN, Victor**
**Ottawa, ON K1N 6N5 (CA)**
• **MIR-OREFICE, Alexandre**
**Ottawa, ON K1N 6N5 (CA)**

(74) Representative: **August Debouzy**
**7, rue de Téhéran**
**75008 Paris (FR)**

(54) **SYSTEMS AND METHODS FOR EVALUATING GAIT**

(57) Disclosed herein are systems and methods for monitoring and evaluating a user's gait. In one embodiment, the method comprises training one or more human activity recognition (HAR) models, each HAR model comprising at least one artificial neural network (ANN) trained on a general or phenotype-specific population. The HAR models are used to identify one or more ambulatory activities in sensor data measured by one or more sensors from a pair of smart insoles worn by the individual. The data is segmented into one or more segments in accordance with the identified ambulatory activities. A gait detection algorithm is used to characterize a gait event with one or more spatiotemporal metrics. The spatiotemporal metrics are classified via one or more machine learning algorithms to produce a gait quality index (CI) score.

**EP 4 623 819 A1**

**Description**

FIELD OF THE DISCLOSURE

The present disclosure pertains to systems and methods of monitoring and evaluating gait.

BACKGROUND

The majority of people with multiple sclerosis (PwMS) may experience a walking impairment, which can lead to falls, and have collectively expressed that mobility is a significant contributor to their quality of life (Bethoux and Bennett, 2011; Heesen et al., 2008).

Clinicians employ various tests to assess a PwMS's gait quality, based on clinical outcome parameters measured by the tests that include, but are not limited to, ambulation fatigability, ambulatory index, and the like, which can aggregate to assess a patient in terms of scores such as the expanded disability status scale (EDSS) scores.

Amongst a battery of neurological tests, clinicians may employ clinical walking tests (e.g., the 500-metre walk test, the six-/two-minute walk test, and the timed 25-foot walk and questionnaires (e.g., MS walking scale 12 (MSWS12)) to longitudinally track the progression of MS and evaluate the effectiveness of interventions, including, but not limited to: exercise, assistive device, surgery, pharmacological (Kurtzke, 1983; Phan-Ba et al., 2012; Chen et al., 2021; Decavel et al., 2019; Bethoux et al., 2016; Hobart et al., 2003).

These walking tests are quick to administer and require minimal equipment while providing meaningful outcome measures and generally valid results (Learmonth et al., 2013; Motl et al., 2017; Stellmann et al., 2015).

However, these tests can be burdensome on a patient, as they must be done in-person, causing the patient to travel to a clinician's (or clinicians') offices. The act of visually observing the participant inherently makes some of these metrics subjective; although objective measurements are recorded (e.g., travel time, distance), there may be more objective information that could be collected and used to inform the clinician on their patient's ambulatory status and thus disease progression/regression.

Moreover, these walking tests are only designed to evaluate gross walking ability (i.e., maximum velocity, distance travelled, travel time, perception), not to analyze the many kinematic, kinetic, and spatiotemporal metrics that characterize the human gait pattern.

Existing methods of objective tests for monitoring and assessing gait in a lab, requiring motion cameras and complex equipment, which may not be easily translated to a mobile or portable method which can be run reliably and continuously, without human intervention.

Understandably, most clinicians may not be able to access the prohibitively expensive and sophisticated equipment found in motion capture laboratories, which may be able to capture the above missing data.

Without this access, clinicians cannot evaluate important gait metrics that have been shown to characterize disease progression and significantly differentiate PwMS from healthy participants: velocity, step length, step time, cadence, double support time, swing time, stance time, step width, and gait variability (Givon et al., 2009; Preiningerova et al., 2015; Severini et al., 2017; Shah et al., 2020, Socie et al., 2013; Chitnis et al., 2019; Filli et al., 2018; Monaghan et al., 2021; Sehle et al., 2011).

However, with the emergence of wearable technology, spatiotemporal gait evaluation, once bound to laboratory settings, is becoming increasingly more accessible to clinicians to objectively evaluate their patients' gait patterns during clinical walking tests (e.g. Cheng et al., 2021; Shema-Shiratzky et al., 2019) and daily life (e.g., Block et al., 2017; Chitnis et al., 2019).

Wearable devices are body-worn equipment typically in the form of an inertial measurement unit (IMU; accelerometer, gyroscope, magnetometer (not always present)) and optionally combined with other sensors (e.g., pressure, heart rate, blood oxygen, etc.).

In some cases, pressure sensor data can be leveraged to detect gait events, be used as a substitute for ground reaction forces, measure balance variables (e.g., centre of pressure (COP) area, COP displacement), and be used to measure temporal events (e.g., stance time, double support time, single support time, sway time, cadence).

An accelerometer may allow for the calculation of spatial variables (e.g., step length, step width, step height, distance travelled), and combined with gait events to calculate velocity.

Gyroscope data can be used to calculate turning variables (e.g., mean, max, min turning angle/velocity) at the level of the foot.

By fusing accelerometer, and gyroscope data, (e.g., using an Unscented Kalman Filter or a Madgwick filter), some methods have demonstrated a framework for determining three dimensional angles of the foot, and/or the position and orientation of the foot in space, which can be calculated through all aspects of the gait cycle. From that orientation, distance travelled (i.e., stride length) and foot angle can be understood.

However, the participant must first perform a calibration procedure to orient the sensor to the foot and the fusion algorithm must be robust enough to control for drift over time, possibly by reorienting the sensor during quiet stance.

New and emerging short-range wireless communications technologies such as UWB can be integrated into sensor network systems. In addition to their low cost, low complexity, and lack of interference with other wireless systems, they provide precise self-location information, making this technology useful for tracking. In the context of gait analysis, knowing the location of one sensor relative to another allows for the calculation of foot-to-foot location and variability between gait cycles, step width, step length, and base of support, among others. These variables are important for assessing fall risk as the mediolateral and anteroposterior foot location directly influence a person's base of support, and when combined with force/pressure data, the centre of mass can be derived, which provides useful information for assessing fall risk.

Studies have shown that that spatiotemporal variables are significantly different within the multiple sclerosis (MS) population with respect to EDSS ranges and compared to the healthy populations.

There is less evidence that angle-based metrics are important when assessing PWMS's gait_pattern(s). Additionally, when angle-based, or turning variables, are identified as important, the sensors are located on the trunk.

For PwMS Shah et al. (2020) noted that the most important variables to discriminate from healthy persons are activity variables (i.e., strides per walking bout, strides per hour) and spatiotemporal variables, including gait speed, double support %, swing %, stride duration, cadence, step duration, and stride length (Shah V V., McNames J, Mancini M, Carlson-Kuhta P, Spain RI, Nutt JG, et al. Quantity and quality of gait and turning in people with multiple sclerosis, Parkinson's disease and matched controls during daily living. J Neurol. 2020;267(4):1188-96.).

Previous studies have also employed wearable devices for tracking basic motion trends in PwMS, demonstrating moderate to strong trends for daily step count, mobility, and sleep compared to clinically derived disability metrics (Block et al., 2019; Sun et al., 2022; Chitnis et al., 2019; Shah et al., 2020; Supratak et al., 2018).

This work is not limited to MS; similar success has been demonstrated in people with Parkinson's disease (e.g., Salis et al., 2023) and cardiovascular disease (e.g., Del Din et al., 2017).

Using wearable devices and other laboratory-grade motion capture technologies, researchers have also been able to implement machine learning (ML) architectures to identify MS disease progression, classify walking phenotypes, identify fall risk, and classify between healthy and PwMS. By frequently capturing a large dataset of PwMS, many of these developed ML architectures can be matured to provide meaningful results and eventually be implemented into clinical practice (Trentzsch et al., 2021; Filli et al., 2018; Meyer et al., 2021; Schumann et al., 2022).

However, a key aspect of any solution is utility. Not all clinicians have a biomechanical or data analysis background that they can rely on to interpret dozens of gait metrics to identify trends of improvement or worsening. Further, patients cannot be actively involved in their healthcare when provided with information they do not understand.

Furthermore, in terms of processing gait data measured by such equipment or by more accessible equipment, there are numerous technical challenges to the processing and identifying trends in the variable mobility data belonging to a person with one or more neurological disorders (the neurological disorders including, but not limited to: Multiple Sclerosis (MS), Parkinson's disease, Alzheimer's disease, Cerebral Palsy (CP), Epilepsy, Motor Neurone Disease (MND), Neurofibromatosis, and the like), or elderly persons, or persons with injuries, walking assisting devices, and the like. These challenges may affect accuracy of computed assessments, and/or the robustness of the algorithms to perform just as well under uncontrolled conditions - "in the wild".

Diseases affecting the central nervous system (CNS), impairments to the person's mobility are largely dependent on where the scarring is within the CNS, resulting in no single gait description to characterize this population, and resulting in technical challenges in developing algorithms and models that can characterize many different gaits.

In addition, studies have been reporting the measurement of some spatiotemporal variable, such as velocity, step length, step time, cadence, double support time, swing time, stance time, step width, and gait variability, that may contribute to distinguishing between healthy participants and PwMS.

Gait spatiotemporal and balance variables significantly change as PwMS become progressively fatigued, and may be correlated with EDSS scores, and other gait-relevant clinical outcome scoring methods (e.g., Multiple Sclerosis Functional Composite- MSFC, or Artificial Intelligence methods- AI). Other variables, which may include local dynamic stability, sway displacement and distance, stability, turning velocity, turning angle, ground reaction forces, lower limb kinetics, may also be important measurements to obtain when characterizing PwMS's gait patterns.

Data may further be obtained by other variables such as smartwatches or activity trackers, but may not provide the same accuracy and/or may not be conducive to building a robust evaluative framework for persons with abnormal gaits.

Notably, there is no known method of providing frameworks for aggregating and translating at home or in-lab patient motion data to provide clinicians and patients with an easily understandable overview of the data, in the form of a novel composite index (CI) or composite movement quality score, coupled with actionable feedback triggered by the automatic processing and conversion of data to the score.

Therefore, developing a framework which can identify the metrics that are significant, vs. insignificant, and to what extent, and use this information to accurately assess a user's gait, as well as diagnose the assessment in terms of the user's disorder, progression of their disorder, injury, and progression of injury/ recovery from injury, are generally not technically feasible with traditional methods of data processing, given the wide spectrum of variables present between patient's having neurological disorders and healthy patients.

Furthermore, once the framework is developed, transforming that framework to an understandable and actionable platform having a useful user interface or graphical user interface (GUI) for both clinicians and patients, is just as variable, and requires significant technical improvements over known methods.

Further, algorithms can be created which may develop a framework for the processing of collected data to provide clinicians with meaningful objective data with minimal human intervention. In this regard, ML can be leveraged to provide composite index (CI) scores from 0-100% to represent a high-level measure of their walking quality and provide data points from which trends can be quickly observed. Similar methods have been successfully implemented in sports research to characterize athletic ability (Ross et al., 2018).

Such technology can provide clinicians with an enhanced ability to intervene (medical or exercise-based) in the progression of the disease to lessen the impact on patients' quality of life. Creating such algorithms that can assess abnormal gaits, or widely varying gaits, is a technical challenge, as there may not be predictable trends in a user's gait (for example, people with MS, people with Parkinson's, and other diseases that may exhibit neurological biomarkers affecting gait, or variability due to people's use of assistive devices such as walkers, canes, and the like). Furthermore, the variability of gait events "in the wild", or outside of a lab, can be significant roadblocks to providing an accurate assessment of gait.

This background information is provided to reveal information believed by the applicant to be of possible relevance. No admission is necessarily intended, nor should be construed, that any of the preceding information constitutes prior art or forms part of the general common knowledge in the relevant art.

SUMMARY

The following presents a simplified summary of the general inventive concept(s) described herein to provide a basic understanding of some aspects of the disclosure. This summary is not an extensive overview of the disclosure. It is not intended to restrict key or critical elements of embodiments of the disclosure or to delineate their scope beyond that which is explicitly or implicitly described by the following description and claims.

A need exists for a system and method for monitoring and evaluating gait.

In accordance with an aspect of the disclosure, there is provided a computer implemented method for evaluating a user's gait, the method comprising: receiving, by a computing device having a memory and a processor, from a pair of smart insoles communicatively coupled to the computing device and worn by the user, data measured by one or more types of sensors; segmenting, by the processor of the computing device, the data into gait-related segmented data comprising one or more of: gait cycle segmentation or activity type; processing the gait-related segmented data, via one or more algorithms stored in the memory of the computing device, to determine one or more of: gait patterns associated with the segmented data; gait parameters associated with the segmented data; gait phenotypes associated with the segmented data; calculating, via one or more algorithms a composite gait quality score, based on a combination and interaction of at least two of: the segmented data, the gait patterns, the gait parameters, and the gait phenotypes.

In some embodiments, the computer-implemented method further comprises: displaying, on a graphical user interface of a user device communicatively coupled to the computing device, the composite gait quality score.

In some embodiments, the graphical user interface comprises one or more of: a clinician portal and a patient smart phone app; wherein the clinician portal and patient app are further configured to receive raw and processed patient data from the system.

In some embodiments, the determining, via one or more algorithms a composite gait quality score, further comprises: training a support vector machine (SVM), of the one or more algorithms, to classify walking data associated with a plurality of participants in one or more groups.

In some embodiments, training the SVM comprises a feature selection step to remove redundant features and identify one or more designated metrics for evaluating gait in a specific patient population.

In some embodiments, the one or more sensor types comprise at least one of: an accelerometer, a gyroscope, a magnetometer, a pressure sensor, and a temperature sensor.

In some embodiments, the parameters comprise gait metrics from said heel strike, foot on floor, heel raise and toe off data.

In some embodiments, the gait patterns comprise a gait signature of the individual from said gait metrics.

In some embodiments, determining the composite gait quality score further comprises: determining a progression over time of the combination and interaction of at least two of: the segmented data, the gait patterns, the gait parameters, and the gait phenotypes.

In some embodiments, determining the composite gait quality score further comprises: an assessment of the user's gait signature and recommendations for improvements.

In some embodiments, determining the composite gait quality score further comprises: an assessment of a change in a patient's neurological disease condition based on measured changes to the patient's gait.

In some embodiments, determining the composite gait quality score further comprises: determining a baseline objective walking quality score of the individual obtained prior to start of a rehabilitation program or immediately following an injury; determining, based on the identified changes in gait pattern, an effectiveness of said rehabilitation program.

In some embodiments, the processing is optimized, by the processor, based on a type of assistive device being used by the user.

In accordance with another aspect, there is provided a system for evaluating a user's gait, the system comprising: a processor; and a memory comprising instructions stored thereon, which when executed by the processor, causes the processor to: receive data measured by one or more types of sensors of a pair of smart insoles worn by the user, the smart insoles in communication with the computing device, segment the data into gait-related segmented data comprising one or more of: gait cycle segmentation or activity type; process the gait-related segmented data, via one or more algorithms stored in the memory of the computing device, to determine one or more of: gait patterns associated with the segmented data; gait parameters associated with the segmented data; gait phenotypes associated with the segmented data; calculate, via one or more algorithms, a composite gait quality score, based on a combination and interaction of at least two of: the segmented data, the gait patterns, the gait parameters, and the gait phenotypes.

In some embodiments, the system further comprises a user device communicatively coupled to the processor, and operable to display on a graphical user interface the composite gait quality score.

In some embodiments, determining, via one or more algorithms a composite gait quality score, further comprises: training a support vector machine (SVM), of the one or more algorithms, to classify walking data associated with a plurality of participants in one or more groups.

In some embodiments, training the SVM comprises a feature selection step to remove redundant features and identify one or more designated metrics for evaluating gait.

In some embodiments, determining is optimized based on a type of assistive device used by the user.

In accordance with another aspect, there is provided a non-transitory computer-readable storage medium comprising instructions stored thereon, which when executed by one or more processors, cause the one or more processors to perform operations for evaluating a user's gait, the operations comprising: receiving data, measured by one or more sensors of a pair of smart insoles worn by the user, the smart insoles in communication with the computing device, segmenting, by a processor of the computing device, the data into gait-related segmented data comprising one or more of: heel strike, foot on floor, heel raise, toe off data, and activity type; processing the gait-related segmented data, via one or more algorithms stored in the memory of the computing device, to determine one or more of: gait patterns associated with the segmented data; gait parameters associated with the segmented data; gait phenotypes associated with the segmented data; calculating, via one or more algorithms a composite gait quality score, based on a combination and interaction of at least two of: the segmented data, the gait patterns, the gait parameters, and the gait phenotypes.

In some embodiments, determining, via one or more algorithms a composite gait quality score, further comprises: training a support vector machine (SVM), of the one or more algorithms, to classify walking data associated with a plurality of participants in one or more groups.

In accordance with another aspect of the disclosure, there is provided a method for monitoring gait quality of an individual, comprising the steps of: training one or more human activity recognition (HAR) models, each HAR model comprising at least one artificial neural network (ANN) trained on a general or phenotype-specific population; using the one or more HAR models to identify one or more ambulatory activities in sensor data measured by one or more sensors from a pair of smart insoles worn by the individual; segmenting said data into one or more segments in accordance with the identified ambulatory activities; analyzing, via a gait detection algorithm, segments only related to an ambulatory activity to characterize a gait event with one or more spatiotemporal metrics; classifying, via one or more machine learning algorithms, the spatiotemporal metrics to produce a gait quality composite index (CI) score; computing a change between the CI score relative to a previously produced CI score; and provide a notification of an improvement or degradation in said gait quality based on said change.

Other aspects, features and/or advantages will become more apparent upon reading of the following non-restrictive description of specific embodiments thereof, given by way of example only with reference to the accompanying drawings.

BRIEF DESCRIPTION OF THE DRAWINGS

Several embodiments of the present disclosure will be provided, by way of examples only, with reference to the appended drawings, wherein:

FIG. 1 illustrates a block flow diagram of a system and method in accordance with an embodiment of the disclosure;

FIG. 2 provides a block flow diagram of a system and method in accordance with an embodiment of the disclosure;

FIG. 3A provides details with respect to determining a composite gait quality score;

FIG. 3B illustrates how a person's gait quality fits within a population, in accordance with one embodiment;

FIG. 4 illustrates a flow diagram of methods for monitoring gait, in accordance with one embodiment;

FIG. 5 illustrates a flow diagram detailing sensor inputs, outputs, algorithms and clinician portal and patient app, in accordance with one embodiment;

FIG. 6 illustrates a flow diagram illustrating algorithms and progression algorithms, in accordance with one embodiment;

FIG. 7A provides a non-limiting illustrative example of the difference in gait pattern of unassisted vs assisted walking measured with the system of an embodiment of the disclosure;

FIG. 7B illustrates a dysfunctional gait pattern measured with the system of an embodiment of the present disclosure. The gait detection method can segment the data into distinct gait cycles, and each gait cycle into the different phases (heel strike, foot on floor, heel raise, and toe off) based on the measured, raw plantar pressure, accelerometer, and gyroscope data retrieved from the instrumented insoles.

FIG. 8 illustrates a visual representation of the difference in the patterns of the gyroscope pitch between the left (circumduction) and right feet (see area circled);

FIG. 9A illustrates unassisted (left circumduction) gait pattern of an individual whose left leg expresses circumduction (i.e., rotates about the hip to clear their foot as they do not have sufficient dorsi flexion strength) determined using a method in accordance with an embodiment.

FIG. 9B illustrates the difference in patterns of the Yaw gyroscope;

FIG. 10A illustrates ground truth whole-body motion capture, in accordance with an embodiment of the disclosure;

FIG. 10B illustrates assisted gait pattern determined using a method of an embodiment of the present disclosure;

FIG. 11 illustrates graphically balance analysis using a method of an embodiment of the disclosure. Balance metrics can identify changes due to eyes being open and closed (i.e., reducing feedback to the central nervous system to maintain balance). A larger area is indicative of a reduced balance quality;

FIG. 12 illustrates balance analysis with the system of an embodiment of the present disclosure. By extracting and processing the same data as illustrated in FIG. 11, the velocity of the centre of pressure displacement was assessed. Higher values are correlated with an increase in fall risk;

FIG. 13A illustrates the ground truth method used (MVN Link, Xsens, Netherlands) when outside of the motion capture laboratory;

FIG. 13B illustrates the foot position of an instrumented insole. The participant performs a ~125 metre walk with a stair ascend and descend portion. Foot position is calculated using a Madgwick sensor fusion algorithm;

FIG. 14 illustrates provides an illustrative example of a composite score of gait quality calculated over time of an individual, in accordance with one embodiment;

FIG. 15 depicts the raw sensor data from the instrumented shoe insole and the gait event overlay of a participant performing an ~125 metre walk with a stair ascend and descend portion, in accordance with one embodiment;

FIG. 16 illustrates depicts a hypothetical machine learning clustering result of principal component scores to identify gait phenotypes (e.g., healthy, ataxic, hemipelagic, etc.), in accordance with one embodiment;

FIG. 17A and FIG. 17B and FIG. 17C illustrate one or more steps set forth by a user interface of a mobile app of an embodiment of the present disclosure;

FIG. 18 illustrates graphically, one or more aspects of a composite movement or gait quality score in accordance with one embodiment of the present disclosure;

FIG. 19 illustrates a box diagram of one or more aspects of a composite movement or gait quality score, in accordance with one embodiment of the present disclosure;

FIG. 20A illustrates an aspect of a user interface in accordance with an embodiment of the disclosure; and

FIG. 20B illustrates an aspect of a user interface in accordance with an embodiment of the disclosure;

FIG. 22 is a schematic diagram illustrating an improved machine learning comprising an artificial neural network with four-fully connected dense layers, in accordance with one embodiment;

FIGS. 23A, 23B, 23C and 23D illustrate tables of confusion matrices for predictions on test sets for different ANNs, in accordance one embodiment;

FIG. 24 is a graphical plot that illustrates ICC values for the reliable metrics, in accordance with one embodiment;

FIG. 25 is a graphical plot that illustrate MSWS-12 question correlation values, in accordance with one embodiment;

FIG. 26 is a graphical plot that illustrates the mean values of dependent variables in a walking trial, in accordance with one embodiment;

FIG. 27A, 27B, 27C, 27D, 27E, 27F, 27G and 27H are graphical plots illustrating the results of linear mixed models, in accordance with one embodiment.

DETAILED DESCRIPTION

Various implementations and aspects of the specification will be described with reference to details discussed below. The following description and drawings are illustrative of the specification and are not to be construed as limiting the specification. Numerous specific details are described to provide a thorough understanding of various implementations of the present specification. However, in certain instances, well-known or conventional details are not described in order to provide a concise discussion of implementations of the present specification.

Furthermore, numerous specific details are set forth in order to provide a thorough understanding of the implementations described herein. However, it will be understood by those skilled in the relevant arts that the implementations described herein may be practiced without these specific details. In other instances, well-known methods, procedures and components have not been described in detail so as not to obscure the implementations described herein.

In this specification, elements may be described as "configured to" perform one or more functions or "configured for" such functions. In general, an element that is configured to perform or configured for performing a function is enabled to perform the function, or is suitable for performing the function, or is adapted to perform the function, or is operable to perform the function, or is otherwise capable of performing the function.

When introducing elements of aspects of the disclosure or the examples thereof, the articles "a," "an," "the," and "said" are intended to mean that there are one or more of the elements. The terms "comprising," "including," and "having" are intended to be inclusive and mean that there may be additional elements other than the listed elements. The term "exemplary" is intended to mean "an example of." The phrase "one or more of the following: A, B, and C" means "at least one of A and/or at least one of B and/or at least one of C."

The expressions "gait pattern" as used herein and throughout this disclosure, refer to a general high-level explanation of someone's walking movement on a stereotypical/population level. Certain general information may be obtained from the gait pattern of an individual, such as and without limitation: gender, body size, emotion, disability status, etc. However, a gait pattern may not be used to identify the individual.

The expression "gait signature" as used herein and throughout this disclosure, refer to a specific representation of an individual's walking movement that can be used to identify an individual (i.e., can be used as a form of biometric identification). Changes to the gait signature in response to stimuli (e.g., perturbation, speed change) are specific to that individual.

Systems and methods are disclosed herein which may address the technical difficulties in collecting the data as described above with short-range wireless communications coupled with insole sensors, processing the data collected via these methods, and developing frameworks for converting the collected data into gait assessments and actionable insights derived from gait assessments.

Systems and methods disclosed herein may address technical challenges in the development of a framework which may collect gait patterns using instrumented shoe insoles, analyze those data to calculate spatiotemporal gait metrics, and provide an interpretable overarching CI score (0-100%) that clinicians can use to identify trends and responses to interventions, and that patients can use to objectively observe their walking quality trends and advocate for their healthcare.

Systems and methods disclosed herein may address technical challenges in the tools currently available for monitoring and/or evaluating a user's gait, by employing short-range wireless communications coupled with insole sensors, in order to provide users with a platform to evaluate their symptoms at home.

Systems and Methods for Monitoring Gait (2023)

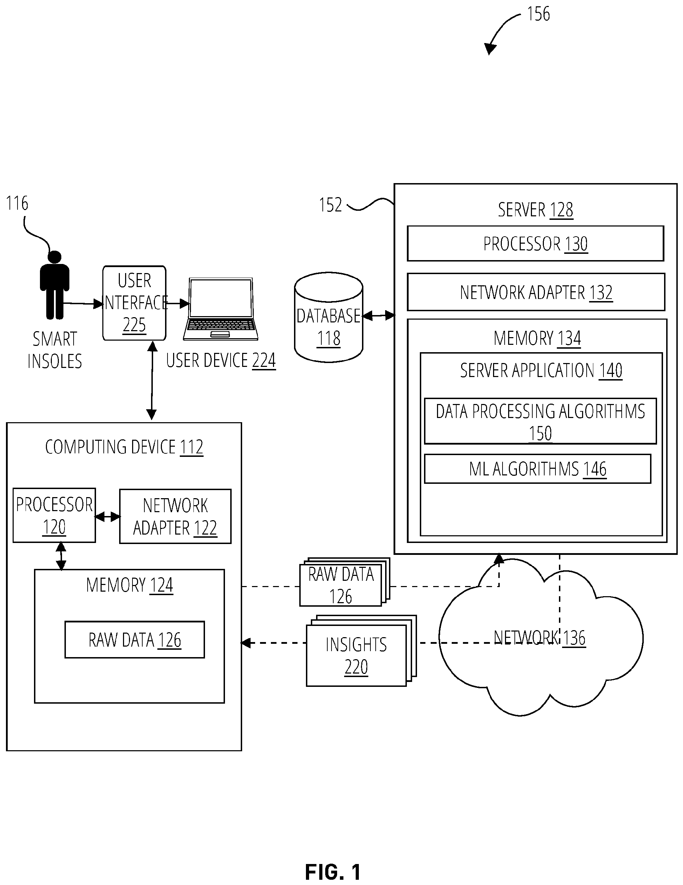

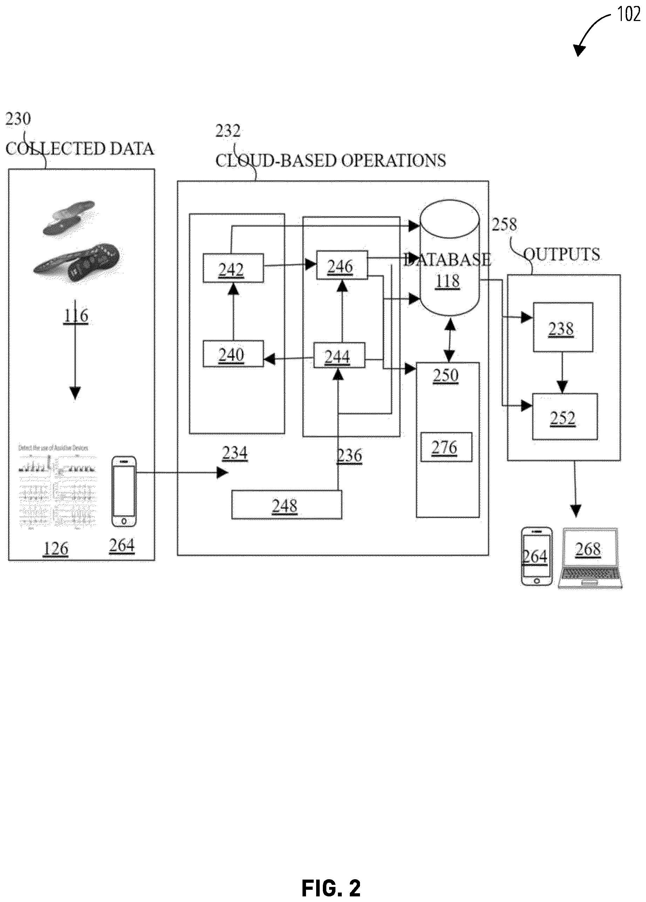

FIG. 1 and FIG. 2 illustrate block flow diagrams of a system 102 and method for analyzing gait and MS progression, in accordance with an embodiment of the disclosure.

An object of the present disclosure is to provide a system and method of monitoring gait.

In accordance with another aspect of the present disclosure, there is provided a system comprising a computing device configured to perform the methods of the present disclosure.

In accordance with another aspect of the present disclosure, there is provided a non-transitory, computer readable memory and/or medium having recorded thereon statements and instructions for execution by a computer the methods of the present disclosure.

As illustrated, a user wearing smart insoles 116 performs walking motions that are recorded as raw data 126 by one or more sensors embedded in the smart insoles 116. In certain embodiments, the smart insoles are in wireless communication, e.g., via Bluetooth, with a user device 224, such as a smart mobile device such as a smart phone, tablet or smart watch comprising a mobile application.

In such embodiments, the smart mobile device receives the raw data 126 collected by the smart insoles and sends the data and optionally other data such as data related to cognitive and dexterity tests to a computing device 112, optionally a cloud-based computing device 152, for analysis with one or more algorithms 146, 150, and optionally storage in a database 118, optionally a cloud-based database 118.

Following analysis, the analyzed data and/or other output may be saved to a database 118, optionally a cloud-based database 118 and/or feedback and insights 220 may be provided to the user and/or other parties such as the user's medical providers. The feedback may be displayed via the mobile application and/or web portal via the user device 224.

Exemplary feedback and insights 220 include but are not limited to alerts or notifications, for example alerts or notifications related to increased fall risk or changes in gait or disease progression, recommendations with respect to treatment programs, rehabilitation programs and/or assistive devices etc.

The raw data 126 collected by the insoles 116 may be received by the computing device in real-time or in batches.

In certain embodiments, the method further comprises one or more steps of collecting the 126 with smart insoles 116. In certain embodiments, the insole data collection is automatic when the insoles are being worn and paired with a smart device comprising the mobile app 1772 of the present disclosure.

In certain embodiments, the insole data collection is in response to user input or at predetermined times. The data collection may be continuous when the insoles are in use or intermittent. In certain embodiments, the method further comprises collecting other data related to cognitive and/or dexterity assessments. For example, cognitive and dexterity tests may be collected through a mobile app 1772 and/or user interface 225 on a user's device 224, by completing surveys, cognitive assessments and upper limb dexterity tests. In certain embodiments, upper limb dexterity tasks and hand to eye coordination is measured using in app 1772 games. In certain embodiments of the system and methods of the present disclosure, a user interface 225 of the user device 224 sends a notification to the user, optionally at predetermined time periods, for example scheduled by a healthcare provider, to complete walking, cognitive and/or dexterity tasks for data collection.

FIG. 2 illustrates in greater detail one or more algorithms that may be employed by the systems and methods of the present disclosure, in order to synthesize a framework for evaluating an individual's gait signature/pattern, and/or in evaluating changes to an individual's gait pattern over time may be used to monitor the progression of a neurological disorder, such as MS, which impacts gait; monitoring for increased risk of falls; and/or the monitoring effectiveness of rehabilitation program/post injury recovery.

In certain embodiments, the raw data 126 collected by the 116 is sent to a cloud-based computing device 232.

Broadly, there are four different kinds of algorithms employed by the systems and methods of the disclosure. Raw data processing algorithms 248 take raw data and segments it. Algorithms for gait pattern classification 236 takes segmented data and classifies it based detected patterns. Algorithms for gait parameters 234 receive classified data and segments it further based on the type of gait, then assess the gate in terms of its performance against key metrics, such as stride, time, stride length, asymmetry, cadence, and the like. Gait pattern classification 236 may also receive raw data or data sorted by the gait parameters 234 algorithm, to phenotype the gait using AI and pattern classification. A database 118 then receives classified data from the gait pattern classification 236 algorithms, and sorts it using a fourth type of algorithm-composite score, which is a novel method of aggregating the outputs to score a user's gait data from a given session in terms of movement quality. The score can be displayed in outputs 258 to a user device in the form of progression graphing (showing the change in gait or gait events over time), and insights 220 or feedback, geared selectively to either a user (patient) or a clinician.

Raw data processing algorithms may comprise: a group 1 algorithm: raw data processing 248), wherein raw data 126 may be segmented, by the computing device, into different activities or tasks of interest. Non-limiting examples of activities include but are not limited to standing, walking, turning, stair ascend/descend, etc. In certain embodiments, the machine learning algorithms employ a sliding window approach. A window refers to a predetermined time period (determine based on the nature of the data; e.g., 1.5 seconds). Then, all data from a window is input to an algorithm, and the activity the data corresponds to is determined (i.e., the output). This window will "slide" forward over the data with a pre-set, constant step size (e.g., 0.5 seconds).

In an embodiment, the machine learning model architecture is comprised of convolutional and long short-term memory (LSTM) layers with a softmax decision layer, selected for their suitability for time series classification problems. Once these decisions are made, the data is segmented, optionally automatically, into the various activities. One purpose of segmenting the data into various activities is to ensure that the appropriate algorithms are utilized for the given activity (i.e., walking, standing, stair navigation, turning, etc.).

For example, if someone is walking, then stops at a streetlight, that standing portion of the data is not analyzed by the walking algorithms, so their step time, cadence, velocity, etc. are not negatively affected. Another purpose will be to identify segments of data suitable for automatic assistive device detection (e.g., walking).

In another embodiment, the machine learning model is a fully connected neural network comprised of dense hidden layers with rectified linear unit (ReLU) activation functions and a softmax decision layer, which may provide a more robust architecture for evaluating large variability in measurements taken in the wild.

In other embodiments, the machine learning (ML) model architecture may comprise a combination of one or more of convolutional, LSTM and dense layers with a decision layer. In another embodiment, the ML model architecture may further comprise a decision-making engine which decides which should be employed in the ML model architecture for activity segmenting.

An advantage of employing one or more hidden layer types (i.e., convolutional, LSTM, dense) is that it increases the flexibility and/or adaptability of the model. For example, if the model receives new data that presents challenges to classification that it has not yet resolved (e.g., a gait phenotype that has abnormalities that manifest over time periods that are significantly longer than a previously monitored window length), the decision-making engine may employ a new requirement to the model architecture, requiring layers with recurrent units like LSTM or similar.

Methods for training a machine learning model to recognize abnormal gait patterns in the wild are disclosed herein.

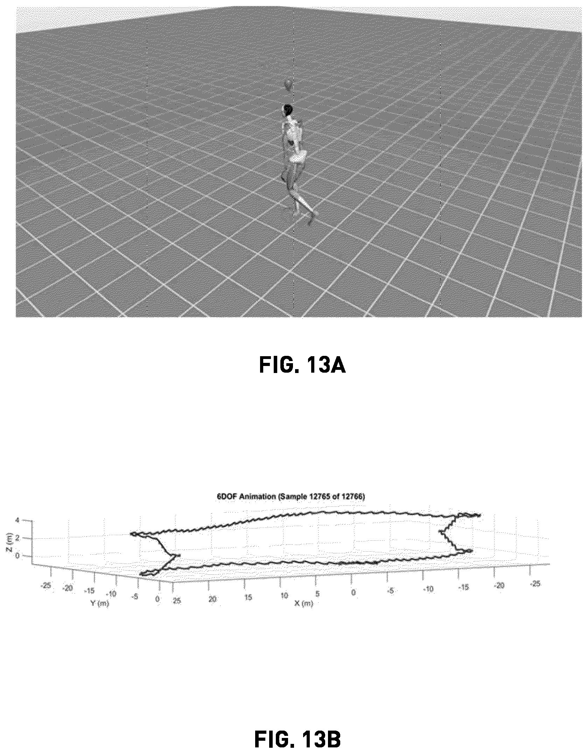

In order to segment data, ground truth labels are needed to train the algorithms 144, 150. Generating ground truth labels requires manually identifying labels using external 3rd party data sources (e.g., video, other motion capture device). An example is illustrated in FIG. 13A.

Given the significance of not only segmenting and removing irrelevant data (i.e. standing at a streetlight while monitoring walking), and segmenting data into different categories, algorithms 144, 150, must be trained to recognize different activities and/or data to be removed.

Identifying these data segments for a person's mobility data measured in the wild is inherently more difficult than in a lab, because the algorithm is not informed by what the person is doing. This challenge may be overcome by the embodiments disclosed herein, by collecting data in a lab under controlled conditions *and* pseudo random conditions, together with the 3rd party data source to generate the labels needed to train. To ensure robustness of the algorithms 144, 150, many different activities are simulated, and many different environments are used to train the algorithm.

Data from group 1 algorithms 248 may be sent to one or more algorithms for gait pattern classification 236. Group 2 algorithms 244 evaluate whether a user is walking with assistance, and what kind of assistive device they are using. Walking patterns between assisted (e.g., use of a cane, walker or other assistive device) and unassisted walking may be considerably different. Accordingly, in certain embodiments, the method of the present disclosure may automatically determine what kind of assistive device is being used (if any), and on which side of the body (when applicable) prior to detecting gait. Group 5 algorithms 246 may determine one or more gait phenotypes such as healthy, spastic, ataxic, and the like, from either raw, segmented, or processed gait pattern data 236.

In some embodiments, using group 2 algorithms 244, the data related to walking is analyzed, optionally automatically, to determine what kind of assistive device is being used (if any), and on which side of the body (when applicable). In specific embodiments, a machine learning algorithm is used to automatically recognize what kind of assistive device is being used (if any), and on which side of the body (when applicable). In certain embodiments, the method determines if the walking is unassisted, with a walker, with a cane, with an ankle foot orthosis. In embodiments where the assistive device can be unilateral (e.g., a cane and an ankle foot orthosis), the method further determines which side the assistive device is used on. In other embodiments, information related to any assistive devices is inputted or has been previously provided. For example, the information related to any assistive devices may be entered with other personal details when configuring the mobile device app 1772 or the information may be requested/confirmed each time the mobile device app is used.

In certain embodiments, gait parameter algorithms 234 may comprise a group 3 algorithm 240: gait detection algorithm. The group 3 algorithm 240 evaluates each foot as being in one of four phases: heel strike (HES), foot on floor (FOF), heel raise (HER), or toe-off (TOF). These phases are triggered based on a set of pressure threshold values redefined for each walking session, predefined accelerometer and gyroscope thresholds, and are reliant on the preceding events. In certain embodiments, the threshold values used to identify phase of gait is dependent on the assistive device used, if any. In some embodiments, gait detection data 240 is further processed by a group 4 algorithm 242: assessment. The group 4 algorithm determines key gait metrics and evaluates the gait detection data 240 against them. Some key gait metrics may comprise stride time, stride length, cadence, asymmetry, and the like. In certain embodiments, the methods of the present disclosure detect gait from the data related to walking. In specific embodiments, the gait detection algorithm is optimized, optionally automatically, based on assistive device used.

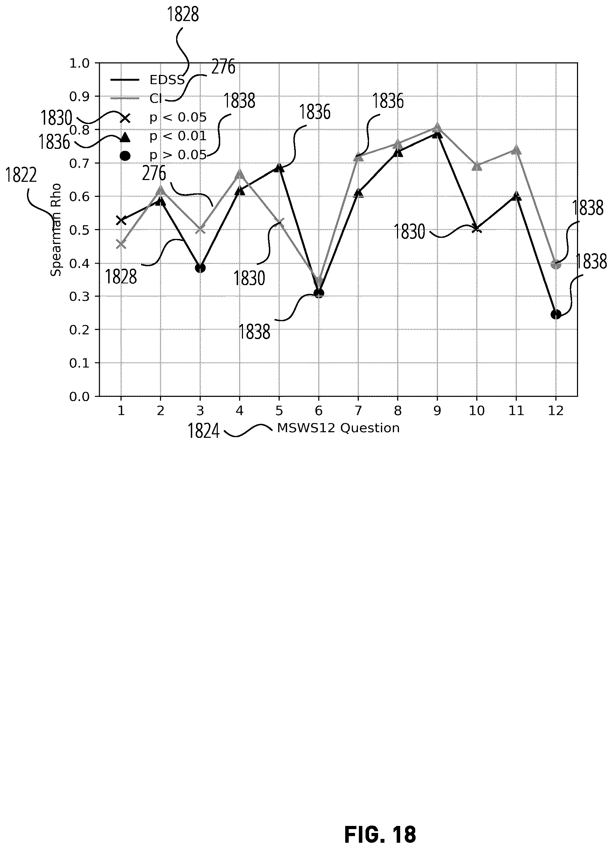

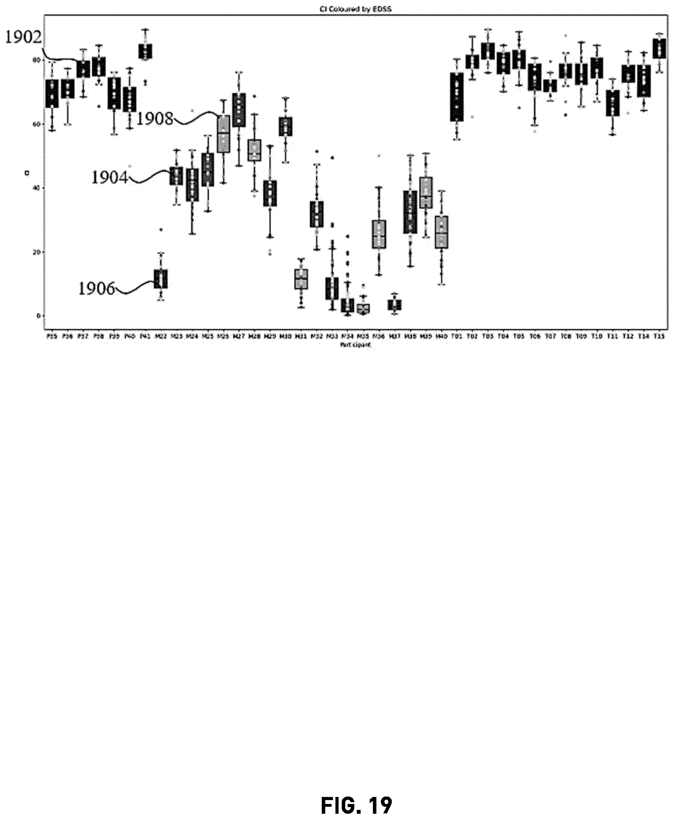

In certain embodiments, the present methods comprise a group 6 algorithm 250 for providing a framework which may calculate a composite movement quality score 276 based on various outputs from the algorithms of groups 1-4, such as outputs of activity classification, assisted walking determination, gait detection, gait assessment and gait phenotyping, and optionally information from previous walking sessions retrieved from the database. In certain embodiments, this score will be a single interpretable number between 0 and 100% and can be used to describe an individual's gait pattern in respect to the population (i.e., z-score). Clinicians may visualize this score based on different populations, such as their patient's mobility (e.g., EDSS score), age, phenotype, assistive device use, etc. This composite movement quality score 276 may be used to track an individual's change from baseline over a period of days, weeks, months, or years. Novel methods of providing a framework for developing a composite index for gait type and health will be described in FIG. 18 and FIG. 19.

In various embodiments, there are disclosed herein computer implemented methods for determining and evaluating an individual's gait signature/pattern, determining and evaluating changes in an individual's gait signature/-pattern, and/or determining and evaluating an individual's response to physical therapy or surgery following an injury, based on the individual's gait signature/pattern and/or changes in it. In some embodiments, evaluating the individual's signature gait pattern further comprises assigning the determined signature gait pattern a composite movement quality scores 276 and communicating it to the individual or their clinician via a mobile device or computing device. In some embodiments, assigning the determined signature gait pattern a composite movement quality scores 276 comprises entering the individual's gait data, measured by one or more sensors integrated into, embedded (removably or permanently), or communicatively coupled to, a pair of smart insoles, into one or more algorithms, which may comprise machine learning ml algorithms 144, trained on both healthy users' gaits, and persons with abnormal gaits, such as PwMS.

The methods may comprise one or more steps, including, but not limited to: (a) wirelessly receiving, by a computing device, data, optionally automatically, collected by a pair of smart insoles worn by the individual in wireless communication with the computing device, the smart insoles comprising one or more sensors selected from the group consisting of accelerometer, gyroscope, magnetometer, pressure sensors and ultra-wide band; (b) segmenting, by the computing device, optionally automatically, data related to gait into heel strike, foot on floor, heel raise and toe off data; (c) determining, by the computing device, optionally automatically, gait metrics from said heel strike, foot on floor, heel raise and toe off data; and (d) determining, by the computing device, optionally automatically, the gait signature of the individual from said gait metrics.

Additionally, sensors may comprise temperature sensors which may alert a user as to the sensors' warm up status.

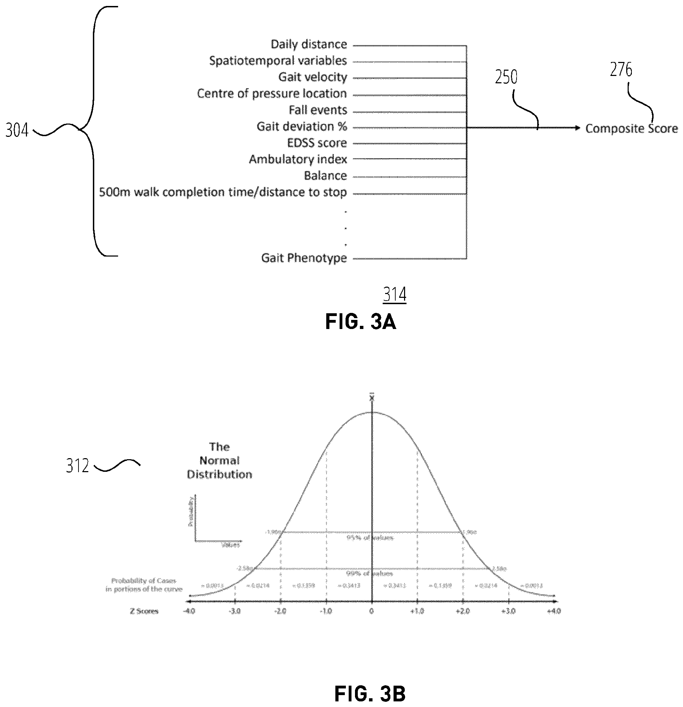

FIG. 3A illustrates one or more variables, which may be processed by a group 6 algorithm: composite score 250, to provide an output comprising one or more composite movement quality scores 276.

A composite score 276 may be calculated using one or more variables 304, both changing and static. The variables 304 may include, but are not limited to, a user's: daily distance, spatiotemporal variables, gait velocity, centre of pressure location, fall events, gait deviation %, EDSS score, ambulatory index, balance, 500m walk completion time/-distance to stop, gait phenotype, and the like. The variables 304 may be automatically or manually retrieved/entered by from raw data 126, from the algorithms 1-4, a patient's medical records, or inputs from the patient and/or clinician.

In certain embodiments, to calculate the composite movement quality score 276, a weighted average of gait metrics (evaluated by gait assessment algorithm group 4 242), phenotypes, and assistive device use, as well as a series of linear regressions and scores from previous sessions, are used to fit the individual within the population of interest. In certain embodiments, individuals will be automatically compared to all populations of interest and logged into the database.

It can be seen in FIG. 3B that, in some embodiments, a user's composite score 276 may be compared with a plurality of other users' composite scores, in order to evaluate it. Comparison may be performed by plotting the user's composite score against the normal distribution 312 of a plurality of users' composite scores and noting its deviation from the mean. Other methods, known in the art, of evaluating a difference between a user's score 276 and an aggregation of other users' scores may be employed instead.

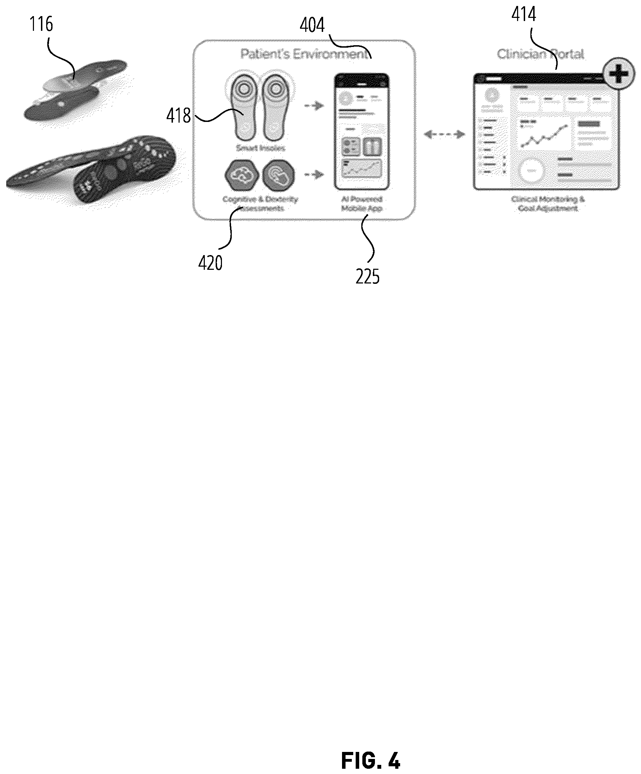

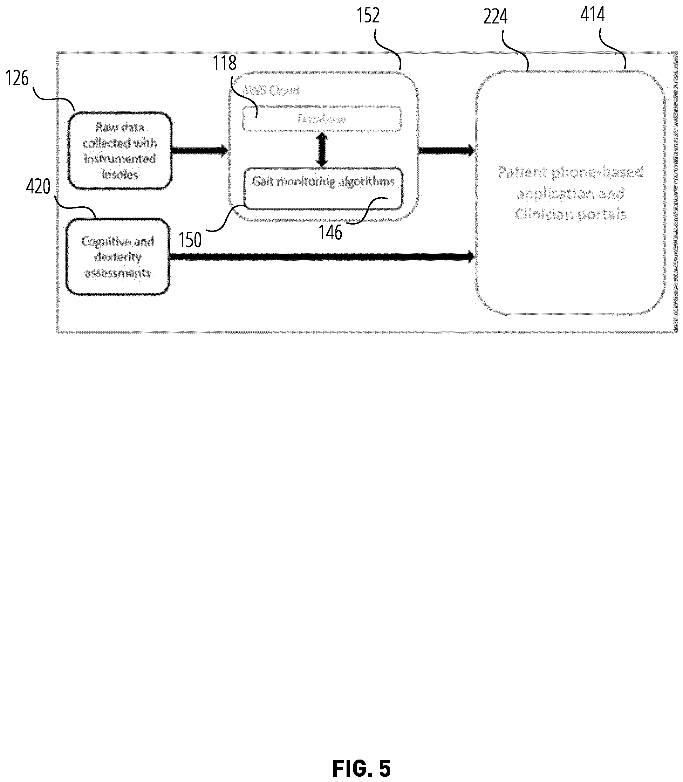

FIG. 4 and FIG. 5 illustrate, schematically, an exemplary flow diagram detailing sensor inputs from smart insoles 116, outputs, algorithms and clinician portal and patient app, in accordance with one embodiment.

In certain embodiments, a patient environment 404 may comprise a user interface 225, coupled to the cloud-based computing device 152 as described in FIG. 1 and FIG. 2. The cloud based computing device receives a patient's gait-related raw data 126 from sensors in the insoles 116, and/or cognitive and dexterity data from cognitive and dexterity assessments 420 (performed in the user interface 225 or received from an external source), and processes the data using the one or more algorithms 146, 150, as described in FIG. 1 and FIG. 2. The processed data is sent to a clinician portal 414, where a clinician may view real time and/or trending assessments of the patient's gait.

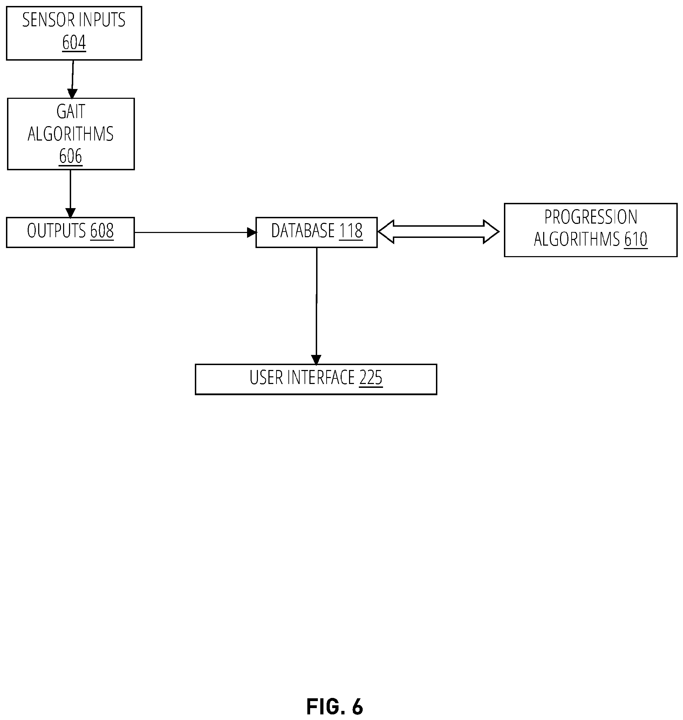

FIG. 6 illustrates systems and methods for monitoring an individual's gait signature/pattern,

Below are exemplary computer implemented methods according to one or more embodiments of the present disclosure.

Generally, sensor inputs 604 are received by a computing device 112 and processed by one or more gait algorithms 606, which may comprise any one of the algorithms described in FIG. 1 and FIG. 2, and sent to a database 118 as outputs 258. The outputs may be further processed by one or more progression algorithms 610, which may comprise any one of the algorithms described in FIG. 1 and FIG. 2, modified to further process the outputs 258 to determine any changes in the user's gait over time or with respect to other users' gaits. The progression data is converted to one or more composite movement quality scores 276, and sent to a user interface 225, such as a patient environment (accessible online or via a smart phone, for example) or a clinician portal (accessible online or via a smart phone, for example).

In accordance with an aspect of the present disclosure, there is provided a computer implemented method for determining an individual's gait signature/pattern; the method comprising: (a) wirelessly receiving, by a computing device, data, optionally automatically, collected by a pair of smart insoles worn by the individual in wireless communication with the computing device, the smart insoles comprising one or more sensors selected from the group consisting of accelerometer, gyroscope, magnetometer, pressure sensors and ultra-wide band; (b) segmenting, by the computing device, optionally automatically, data related to gait into heel strike, foot on floor, heel raise and toe off data; (c) determining, by the computing device, optionally automatically, gait metrics from said heel strike, foot on floor, heel raise and toe off data; and (d) determining, by the computing device, optionally automatically, the gait signature of the individual from said gait metrics. In certain embodiments, the gait phenotype of the individual is determined. In some embodiments, the method is a real-time method. Exemplary gait phenotypes include but are not limited to healthy, ataxic, spastic, hemiplegic using the gait measures, and the direct signals with phases.

In another aspect of the present disclosure, there is provided a computer implemented method of monitoring changes in gait pattern of an individual; the method comprising: (a) wirelessly receiving, by a computing device, data, optionally automatically, collected by a pair of smart insoles worn by the individual in wireless communication with the computing device, the smart insoles comprising one or more sensors selected from the group consisting of accelerometer, gyroscope, magnetometer, pressure sensors and ultra-wide band; (b) segmenting, by the computing device, optionally automatically, data related to gait into heel strike, foot on floor, heel raise and toe off data; (c) determining, by the computing device, optionally automatically, gait metrics from said heel strike, foot on floor, heel raise and toe off data, optionally the gait metrics are selected from stride time, stride length, cadence and asymmetry; (d) determining, by the computing device, optionally automatically, the gait signature of the individual from said gait metrics; and (e) comparing, by the computing device, optionally automatically, the gait signature of the individual to a baseline gait signature of the individual to identify changes in gait pattern. In certain embodiments of the above method, the method further comprises (f) sending, optionally automatically, a notification to the individual and/or to the individual's healthcare provider regarding any changes in the gait pattern, optionally the notification includes recommended interventions. The interventions may include a change in treatment/rehabilitation plan, change in use of assistive device(s). In certain embodiments, the method includes implementation of the recommended interventions.

In accordance with another aspect of the present disclosure, there is provided a computer implemented method of monitoring (i) progression of a neurological disorder which impacts gait/(ii) for increased risk of falls; the method comprising: (a) wirelessly receiving, by a computing device, data, optionally automatically, collected by a pair of smart insoles worn by the individual in wireless communication with the computing device, the smart insoles comprising one or more sensors selected from the group consisting of accelerometer, gyroscope, magnetometer, pressure sensors and ultra-wide band; (b) segmenting, by the computing device, optionally automatically, data related to gait into heel strike, foot on floor, heel raise and toe off data; (c) determining, by the computing device, optionally automatically, gait metrics from said heel strike, foot on floor, heel raise and toe off data, optionally the gait metrics are selected from stride time, stride length, cadence and asymmetry; (d) determining, by the computing device, optionally automatically, the gait signature of the individual from said gait metrics; and (e) comparing, by the computing device, optionally automatically, the gait signature of the individual to a baseline gait signature of the individual to identify changes in gait pattern, wherein an increase in frequency of abnormal stride patterns is indicative of progression of said neurological disorder/increased risk of falls. In certain embodiments, the above method comprises (f) sending, optionally automatically, a notification of the individual and/or the individual's healthcare provider if there is progression of the neurological disorder and/or increased risk of falls, optionally the notification includes recommended interventions. The interventions may include a change in treatment/rehabilitation plan, change in use of assistive device(s) and/or fall prevention strategy. In certain embodiments, the method includes implementation of the recommended interventions.

In accordance with another aspect of the present disclosure there is provided a computer implemented method of monitoring effectiveness of rehabilitation program/post injury recovery; the method comprising: (a) wirelessly receiving, by a computing device, data, optionally automatically, collected by a pair of smart insoles worn by an individual in a rehabilitation program, in wireless communication with the computing device, the smart insoles comprising one or more sensors selected from the group consisting of accelerometer, gyroscope, magnetometer, pressure sensors and ultra-wide band; (b) segmenting, by the computing device, optionally automatically, data related to gait into heel strike, foot on floor, heel raise and toe off data; (c) determining, by the computing device, optionally automatically, gait metrics from said heel strike, foot on floor, heel raise and toe off data, optionally the gait metrics are selected from stride time, stride length, cadence and asymmetry; (d) determining, by the computing device, optionally automatically, the gait signature of the individual from said gait metrics; and (e) comparing, by the computing device, optionally automatically, the gait signature of the individual to a baseline gait signature of the individual obtained prior to start of the rehabilitation program or immediately following the injury to identify changes in gait pattern, wherein a decrease in frequency of abnormal stride patterns is indicative of effectiveness of said rehabilitation program or injury recovery. In certain embodiments, the above method further comprises (f) sending, optionally automatically, a notification of the individual and/or the individual's healthcare provider regarding effectiveness of said rehabilitation program or status of the injury recovery, optionally the notification includes recommended changes to rehabilitation program. The interventions may include a change in treatment/rehabilitation plan. In certain embodiments, the method includes implementation of the recommended interventions.

In the case that the one or more gait algorithms 606 cannot solve for a gait cycle, the phase will be repeated even when not appropriate and a dysfunctional label is put in its place so those cycles can be removed in subsequent algorithms.

Preferably, the gait algorithms 606 of the present disclosure comprise technical improvements which mitigate the algorithm's 606 inability to solve for one or more gait labels. Presently, a decision-making engine of the algorithm 606 finds all toe off events within the data being processed, then works within each gait cycle (toe off to toe off) so that any errors are localized within a single gait cycle, rather than cycling through the same on repeat, until it can solve for a gait event again. This improvement results in a faster, more efficient, and more robust system.

In certain embodiments of the above methods, the phases of a gait cycle comprise heel strike, foot on floor, heel raise and toe off; and step (b) comprises comparing said data to predetermined thresholds for each phase of a gait cycle; and segmenting the data into data related to gait into heel strike, foot on floor, heel raise and toe off data based on the comparison.

In certain embodiments of the above methods, the gait metrics are temporal metrics, optionally calculated using the indices of the gait phases from both feet in the time domain.

In certain embodiments of the above methods of the disclosure, the gait metrics are spatial metrics, optionally calculated by using the indices of the gait phases from both feet to apply sensor fusion (i.e., Madgwick filter) to the accelerometer and gyroscope data, optional ultra-wideband, and deep learning approaches such as neural networks (long-short term memory, convolutional, etc.).

In certain embodiments of the above methods of the disclosure, the gait metrics are spatiotemporal (time and distance-based, velocity), optionally calculated by taking the integral of the accelerometer signals over time between gait phase indices and by multiplying temporal and spatial metrics.

The gait detection algorithm 606 assesses the pressure, gyroscope, and accelerometer values based on the maximum values observed within this specific batch of data. In certain embodiments, for consistency, the data presented to the gait detection algorithm will begin with the right foot in the FOF phase and the left foot in TOF (i.e., right foot single support). In certain embodiments, the assessment is as follows:

The foot is considered to be in the FOF phase when the values from the heel, toe, or midfoot pressure sensors are greater than or equal to their threshold, the values of the vertical accelerometer and pitch gyroscope are within their respective bounds ($\pm0.8$ G and $\pm10$ degrees/second, respectively), and the previous phase is HES or FOF.

The foot is considered to be in HER when the pressure value from the heel or midfoot are less than their threshold, the toe pressure value is greater than its threshold and the previous phase was FOF or HER.

The foot is considered to be in TOF if the values from all pressure sensors are below their respective threshold and the previous phase was HER or TOF.

For HES, the striking pattern is first evaluated to be either a heel, midfoot, or toe strike. This allows individuals to change how they walk throughout the session as there is no guarantee the individual will strike their foot the same way between gait cycles. For a heel strike, the foot is considered to be in HES when the heel pressure value is greater than or equal to its threshold and the toe is less than or equal to its threshold. For a midfoot strike, the midfoot pressure sensor is greater than or equal to its threshold, and the vertical accelerometer and pitch gyroscope are outside of their respective threshold bounds. For a toe strike, the toe pressure value is greater than or equal to its threshold, and the vertical accelerometer and pitch gyroscope are outside of their respective threshold bounds. For all striking types, HES must be preceded by the TOF or HES phase.

In another embodiment, improvements have been made to the above processes, such that a method for monitoring and evaluating gait, may comprise the following steps:

1. The gait detection algorithm 606 assesses the pressure, gyroscope, and accelerometer values based on thresholds relative to the maximum values observed within this specific batch of data. In certain embodiments, the data are presented to the gait detection algorithm with both feet in the FOF phase.

i. In certain embodiments, the assessment may be as follows:

1. TOF events are identified by first identifying mid swing indices, which may comprise locating a local peak gyroscope that is within 20% of the average peak value; filters may be in place to accommodate outliers in magnitude and time.
2. For each mid swing index, the pre-swing phase may be identified by dynamically altering a threshold value (10-50%) relative to the local peak gyroscope pitch.
3. The average minimum pressure location across all pressure sensors is used as the TOF event.

2. To identify all other phases, data are first segmented into gait cycles using the TOF events. Preferably, this ensures any failures of the detection algorithm are localized within one gait cycle.

HES events may be identified in two ways: pressure-based, and accelerometer-based.

In a pressure-based embodiment, if a pressure group (heel, midfoot, toes) surpassed the threshold set by the maximum observed pressure, an HES event is triggered.

However, pressure insoles do not always provide reliable data; pressure data can be present when the foot is in swing phase, also termed residual pressure.

In the case of residual pressure at mid swing that surpasses the threshold set for the pressure-based algorithm, a decision-making engine triggers the employment of the accelerometer-based embodiment to evaluate the data.

In the accelerometer-based embodiment, a local minimum value in the forward accelerometer is used to identify the breaking phenomenon found in cyclical gait.

Using average pressure data found in a pre-set time range, which may include for example, 0-100ms, 100 ms-1s, and greater than 1s, pressure thresholds may recalibrate and pressure-based algorithm described above is used.

To account for different types of foot planting, HES events may be identified as a heel-strike, mid foot-strike, or a toe-strike. The foot may no longer be identified in the HES event when the vertical accelerometer and pitch gyroscope are within the FOF thresholds.

The foot is considered to be in the FOF phase when the values from the heel, toe, or midfoot pressure sensors are greater than or equal to their threshold, the values of the vertical accelerometer and pitch gyroscope are within their respective bounds ($\pm0.8$ G and $\pm10$ degrees/second, respectively), and the previous phase is HES, or FOF.

The foot may be considered to be in HER when the pitch gyroscope is greater than or equal to 20% of the local peak prior to the TOF event.

In certain embodiments, the identified phases of gait are then used for gait assessment. In such embodiments, using the gait cycle phases, a series of temporal (i.e., time-based), spatial (i.e., distance-based), and spatiotemporal (i.e., time and distance-based) variables are calculated for gait assessment. The variables may include both within-foot and between-feet variables. Within-foot variables include stride time, stride length, stride height, swing velocity, stance time/percent, swing time/percent, single support time/percent, and double support time/percent. Between-feet variables include step time, step length, step width, asymmetry, total distance, local dynamic stability, and cadence.

Other sensors embedded in (either permanently or removably), or communicatively coupled to the smart insoles 116 may comprise one or more of: accelerometers, gyroscopes, magnetometers, pressure sensors and ultra-wide bands.

Additionally, the smart insoles 116 may comprise temperature sensors, which may alert a user of a warm up status of the insoles.

Temporal metrics depend on the heel strike and toe-off events, while the spatial metrics (and, by proxy, spatiotemporal metrics) are based on a combination of machine learning algorithms and sensor fusion algorithms to determine the distance travelled within and between feet.

# EP 4 623 819 A1

Table 1. Gait Metrics Calculated.

| Metric | Unit |
|---|---|
| Gait velocity | km/hr or equivalent |
| Swing velocity | m/s |
| Cadence | steps per minute |
| Stride length | centimetres |
| Step length | centimetres |
| Step height | centimetres |
| Step width | centimetres |
| Walk ratio | mm/step/min |
| Left step duration | seconds |
| Right step duration | seconds |
| Step duration | seconds |
| Stride duration | seconds |
| Single support | % of gait cycle |
| Double support | % of gait cycle |
| Stance phase | % of gait cycle |
| Swing phase | % of gait cycle |
| Single support time | seconds |
| Double support time | seconds |
| Stance time | seconds |
| Swing time | seconds |
| Local dynamic gait stability | $\lambda$ max |
| Walking asymmetry | % difference between feet |

Accordingly, in certain embodiments, gait assessment comprises a determination of one or more of the following: gait velocity, swing velocity, cadence, stride length, step length, step height, step width, walk ratio, left step duration, right step duration, step duration, stride duration, single support, double support, stance phase, swing phase, single support time, double support time, stance time, swing time, local dynamic gait stability, and walking asymmetry.

In certain embodiments, a combination of cut raw signals (pressure, accelerometer, gyroscope, etc.) following activity recognition, gait phases, and spatiotemporal metrics, a pattern classification algorithm identifies the greatest modes of variance within the signal and a machine learning algorithm clusters those features into groups that describe different gait phenotypes (e.g., healthy, ataxic, spastic, hemiplegic, etc.).

An individual may exhibit several phenotypes and express different ones as they fatigue, for example. Accordingly, in certain embodiments, the gait cycles are not grouped as one phenotype. That is, each gait cycle is treated as its own input to the model so an individual can drift into different gait phenotypes over the course of a walking session. In such embodiments, Therefore, the output is a percentage of several phenotypes expressed throughout the session. An example output for a given walking session is 80% hemiplegic, 10% ataxic, and 10% healthy.

In a further embodiment, foot drop events are detected using only raw data signals in a machine learning algorithm. A foot drop is defined as the event following the inability to sustain a dorsiflexed position during the toe-off portion of the gait cycle, resulting in the foot suddenly contacting the floor at heel strike. This is a common symptom experienced by PwMS. In certain embodiments, foot drops are counted and the value added to the database for longitudinal tracking.

In certain embodiments, the systems and methods for monitoring and evaluating a user's gait are conducted in real-time. Such systems and methods may be used for one or more of the following: monitoring gait and/or balance; determining an individual's gait signature; detecting changes in gait patterns; monitoring increased risk of falls; monitoring the progression of diseases and disorders that impact gait, including but not limited include to neurological disorders such as multiple sclerosis (MS) or Parkinson's Disease; assessing the effectiveness of rehabilitation programs; monitoring the post-injury recovery; identifying gait phenotypes and assessing the effect of interventions (including but not limited to physical, medical, psychological, etc.).

In certain embodiments, the system and method of the present disclosure provide healthcare providers and/or pharmaceutical companies and insurers, with insights on conditions which impact gait that would otherwise be undetectable by traditional / existing clinical tools.

The present disclosure provides methods which extract and process data from smart insoles for determining gait and/or balance quality. In certain embodiments, the methods extract and process data from smart insoles comprising one or more sensors selected from a group comprising one or more of: accelerometers, gyroscopes, magnetometers, pressure sensors, ultra-wide band etc and wireless communication capabilities. Other commercially available sensors and/or smart insoles may be employed.

In certain embodiments, the methods of the present disclosure track progression of gait over time using progression algorithms 610. This may be to track disease progression, to assess the effectiveness of rehabilitation programs or monitor post-injury recovery. In certain embodiments, a personalized baseline is calculated using several walking sessions. This is to establish an overall description of their typical walking pattern. A baseline may be calculated at different time points. In this way, interventions may be directly evaluated (i.e., pharmacological, exercise, assistive device, surgical, etc.).

From this baseline, trendlines are calculated to explain how their gait pattern changes (e.g., worsening/improving) over any given timeline (i.e., days, weeks, months, years, etc.). Metrics such as composite movement quality score 276, frequency of assistive device use, number of foot drop events per number of gait cycles, and phenotyping may be used to track progression. In certain embodiments, the composite movement quality score 276 alone or in combination with one or more of the following: frequency of assistive device use, number of foot drop events per number of gait cycles, and phenotyping is used to track progression.

In certain embodiments, the methods of the present disclosure provide feedback to the user and/or healthcare provider. In certain embodiments, the feedback may include alerts including but not limited to increased risk of falls or metrics such as ambulatory index or EDSS. Moreover, visual representations of trendlines may provide clinicians with an early indicator that an intervention is needed to slow the progressing of the disease or change a rehabilitation program is required. Accordingly, in certain embodiments, the methods further comprise changing a patient's treatment and/or rehabilitation regime in response to the feedback provided. In addition, alerts to users regarding increased fall risk may prevent fails by allowing fall prevention measures to be implemented, including strength and balance exercise programs, introduction of assistive walking devices and/or the reduction of safety hazards in the patient's living environment. Accordingly, in certain embodiments, the methods further comprise implementing fall prevention measures in response to the feedback provided.

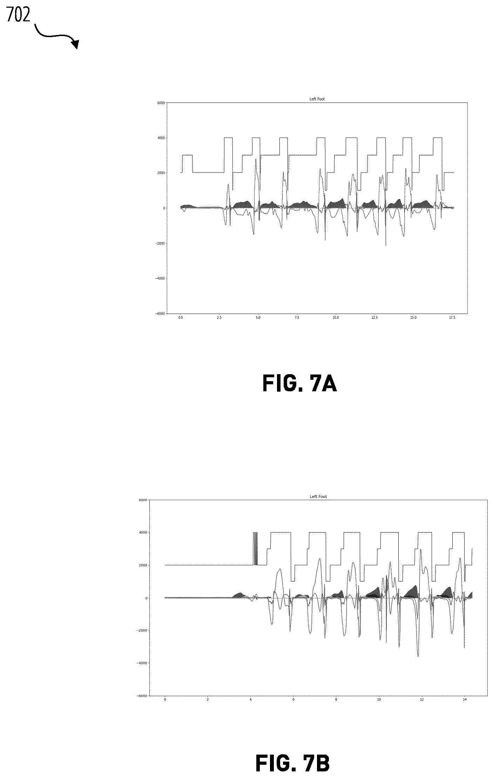

FIG. 7A, FIG. 8, FIG. 9A &B, FIG. 10A &B, all illustrate various data processing methods in accordance with one or more aspects of the above methods.

FIG. 7A provides a non-limiting illustrative example of the difference in gait data 702 and/or gait pattern of unassisted vs assisted walking measured with the system of an embodiment of the disclosure

FIG. 7B illustrates a dysfunctional gait pattern measured with the system of an embodiment of the present disclosure. The gait detection method can segment the data into distinct gait cycles, and each gait cycle into the different phases (heel strike, foot on floor, heel raise, and toe off) based on the plantar pressure raw data retrieved from the instrumented insoles.

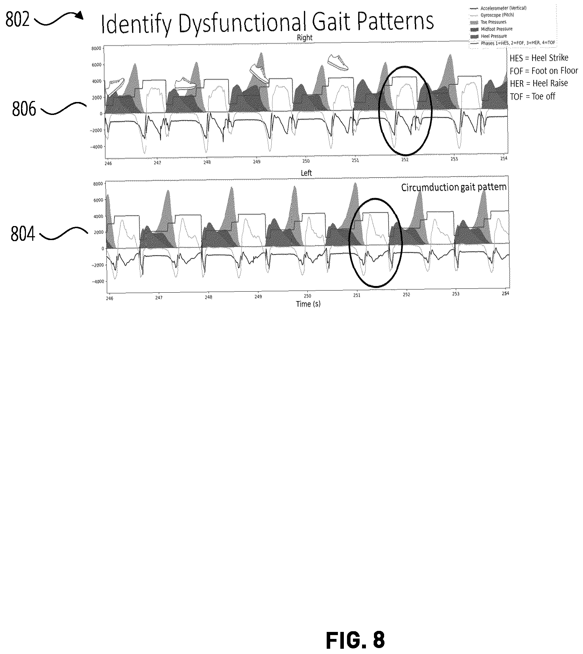

FIG. 8 illustrates a visual representation of the difference in the patterns of the gyroscope pitch between the left foot 804 (circumduction) and right foot 806 (see area circled).

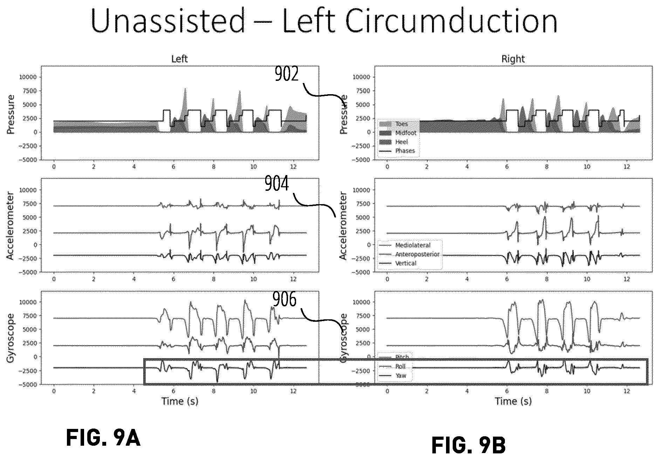

FIG. 9A and B illustrates unassisted (left circumduction) gait pattern of an individual whose left leg expresses circumduction (i.e., rotates about the hip to clear their foot as they do not have sufficient dorsi flexion strength) determined using a method in accordance with an embodiment. Sensor measurements may comprise pressure 902, accelerometer 904, and/or gyroscope 906 measurements, among others.

The circled area illustrates difference in patterns of the Yaw gyroscope between the left foot and the right foot. Gait algorithms 606 may detect these differences in order to assess the user's circumduction.

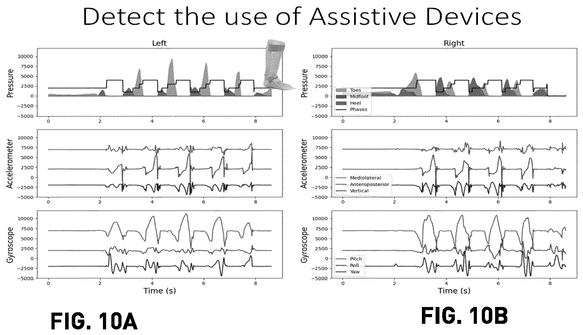

FIG. 10A illustrates ground truth whole-body motion capture, in accordance with an embodiment of the disclosure.

FIG. 10B illustrates assisted gait pattern determined using a method of an embodiment of the present disclosure.

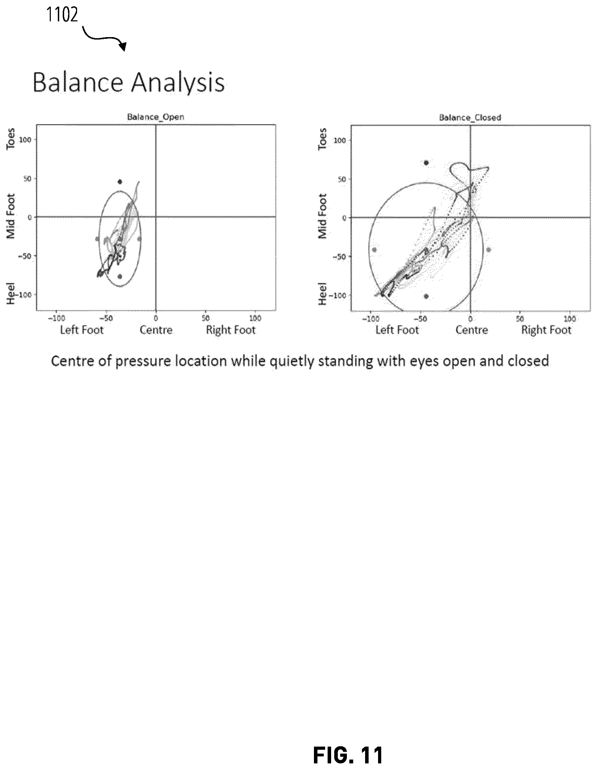

FIG. 11 illustrates graphically analysis of balance data 1102 using a method of an embodiment of the disclosure. Balance metrics can identify changes due to eyes being open and closed (i.e., reducing feedback to the central nervous system to maintain balance). A larger area is indicative of a reduced balance quality.

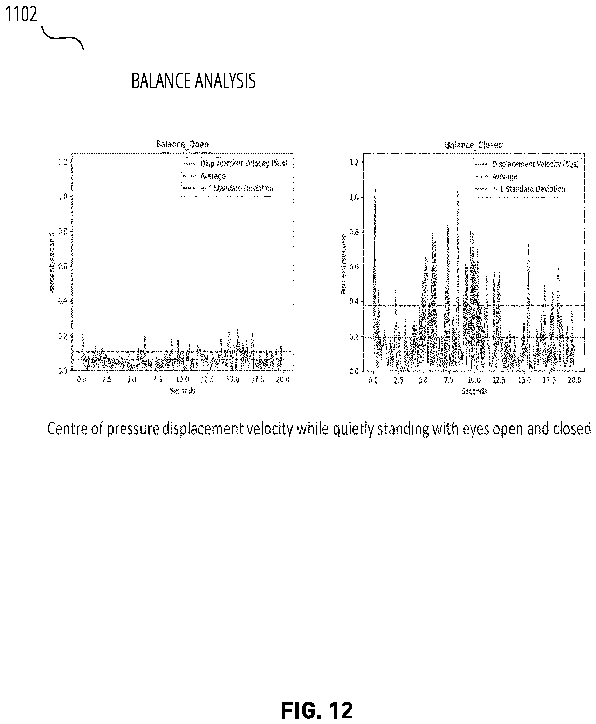

FIG. 12 illustrates analysis of balance data 1102 in accordance with the system of an embodiment of the present disclosure. By extracting and processing the same data as illustrated in FIG. 11, the velocity of the centre of pressure displacement was assessed. Higher values are correlated with an increase in fall risk.

FIG. 13A illustrates a ground truth method 1302 used (MVN Link, Xsens, Netherlands) with a user performing an activity outside of a motion capture laboratory.

Xsens is a commercially available ground truth method.

FIG. 13B illustrates a foot position of an instrumented insole 116. The participant performs a ~125 metre walk with a stair ascend and descend portion. Foot position was calculated using a Madgwick sensor fusion algorithm.

The fusion method used is widely used in IMU research. The Madgwick filter is a well-known filter for IMUs being used for gait, everyday activities, drones, and cars. The specific update method we use is also standard. It is termed the zero-velocity update method. The zero-velocity update reorients the IMU to gravity and is performed when the foot is on the floor, but can be used any time no movement is detected.

For the CI score, these solutions would not be able to do it on their own out of the box. Xsens would provide users with the information needed (i.e., joint angles, segment positions, rudimentary gait events), but significant independent development is required. Similar for the filtering method, it is only one piece of the puzzle, but an arguably required step.

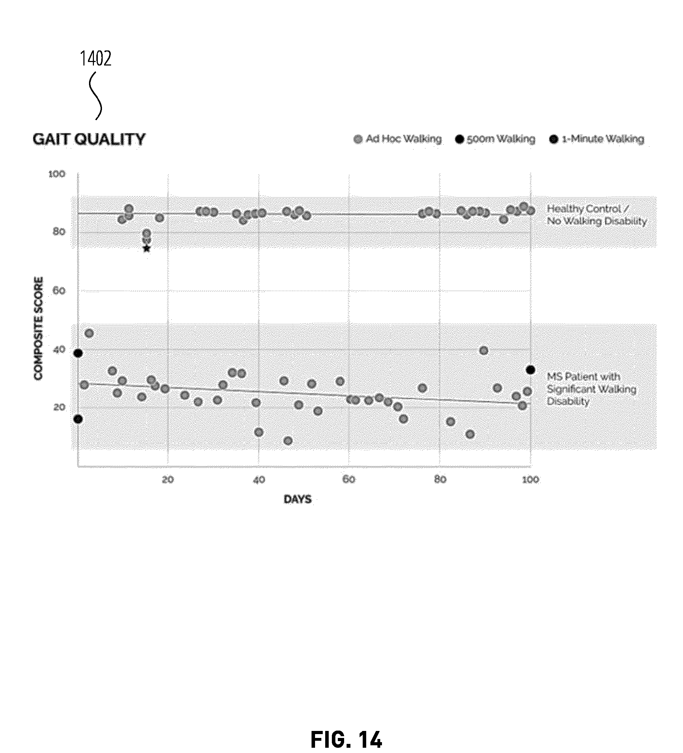

FIG. 14 illustrates provides an illustrative example of a composite score of gait quality calculated over time of an individual, or a graphical composite score progression 1402, in accordance with one embodiment.

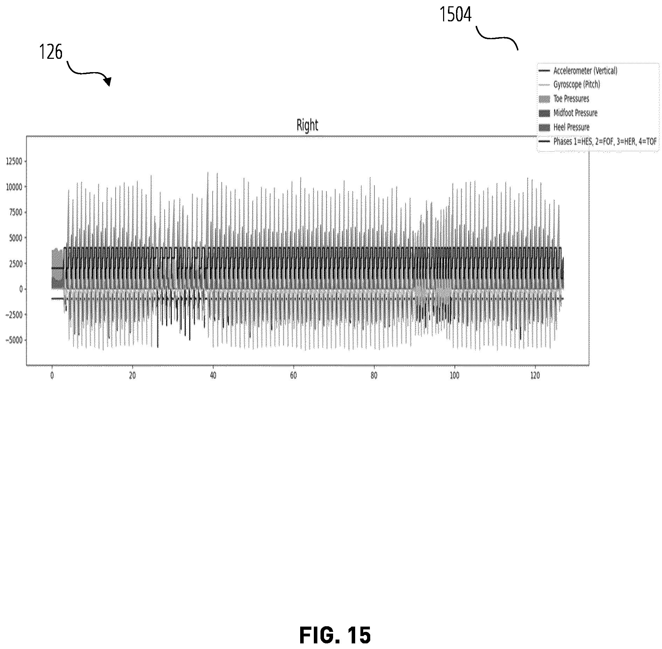

FIG. 15 depicts the raw sensor raw data 126 from the instrumented shoe insole and the gait event overlay of a participant performing an ~125 metre walk with a stair ascend and descend portion, in accordance with one embodiment. In the illustrated embodiment, the sensor data comprises data measured by vertical accelerometers 904, pitch gyroscopes 906, toe, midfoot, and heel pressure 902 sensors, and sensor-identified events, including: HES, FOF, HER, TOF events.

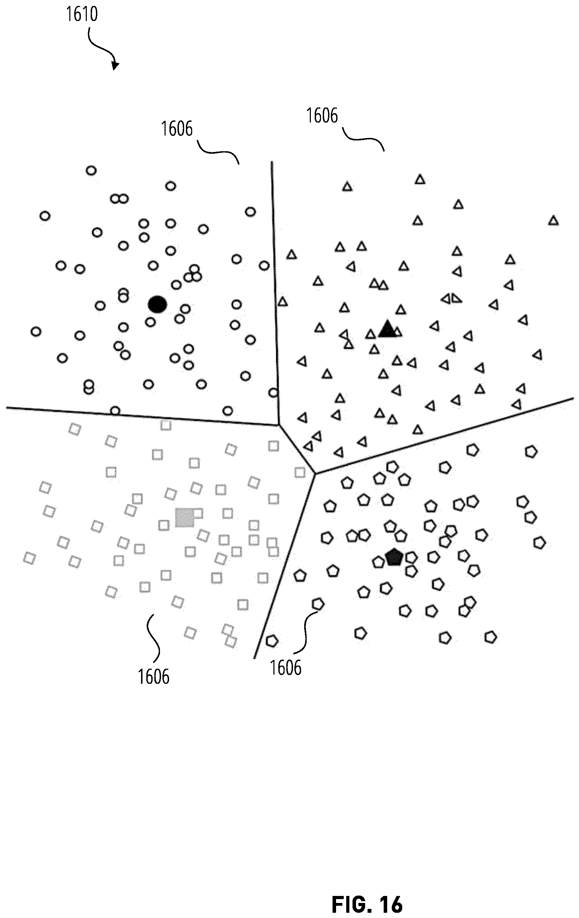

FIG. 16 illustrates depicts a hypothetical machine learning ML clustering 1610 result of principal component scores to identify gait phenotypes 1606 (e.g., healthy, ataxic, hemipelagic, etc.), in accordance with one embodiment.

The skilled person in the art will appreciate that determining phenotypes outside of a laboratory environment is a substantial technical challenge. Known methods typically uses whole-body movement patterns (i.e., lower and upper body motion) to determine gait phenotypes, which is typically restricted to laboratory technology. To analyze the appropriate data (i.e., straight walking, etc.), activity detection needs to be performed, which requires overcoming the technical challenges of training an ML algorithm as discussed. To train an ML algorithm to identify the gait phenotype, ground truth labels from experts who can visually observe the person's walking pattern are also required. Prior to phenotyping, a robust segmentation method is preferably developed to ensure phenotyping is accurate - i.e. walking pattern being segmented into gait events before phenotyping. Further, when moving from a laboratory environment to the wild, the method must have been perfected and tested, before introducing new variables to train the algorithms on. The present disclosure advantageously overcomes those technical challenges by only using, in accordance with different embodiments, foot motion to determine gait phenotypes.

Phenotyping in previous literature is also described as spatiotemporal (e.g., stride time, stride length, etc.) and whole-body kinematic changes (i.e., joint angles) compared to healthy controls. They measure the person for several seconds on specialized pressure maps, using proprietary accelerometer software, and expensive motion capture systems, that are restricted to laboratories.

In the lab, only short segments of data can be measured while a user is moving overground. The present method assesses data over many consecutive these segments to improve its accuracy, which in turn introduces new challenges in that the methods need to be sensitive enough to detect changes in gait over a long period of time.

These challenges may be overcome by generating labels in a lab, extensively testing them, and then employing the solution in the wild, iteratively evaluating phenotype progression over time.

Phenotyping gait by coupling smart insole data with novel algorithms employs a practical method for assessing MS patients' gaits.

In certain embodiments, the raw signals are first organized using the gait phases, and is analyzed using principal component analysis (PCA) to identify the modes of variability in that signal, deemed principal components (PCs). Scores are given to each gait cycle that relates to how well a given PC explains the raw data within that gait cycle. These PC scores are then input to a clustering-based machine learning algorithm (e.g., K-means, hierarchical cluster analysis). Using ground truth labels which have been previously created from neurologists analyzing videos of people with multiple sclerosis (PwMS), the clusters that are created by the machine learning algorithm are labelled as a specific gait phenotype.

Employing the disclosed gait phenotyping methods, gait insights 220 may be delivered using terminology that is meaningful to clinicians. For example, an assessment describing a patient's mild spasticity in left leg progressing to moderate / high spasticity over a time period. Insights 220 may be geared selectively towards patients, by converting the phenotypes 1606 into challenges that patients will face. For example, an assessment describing that a patient may struggle to walk for more than 5 minutes.

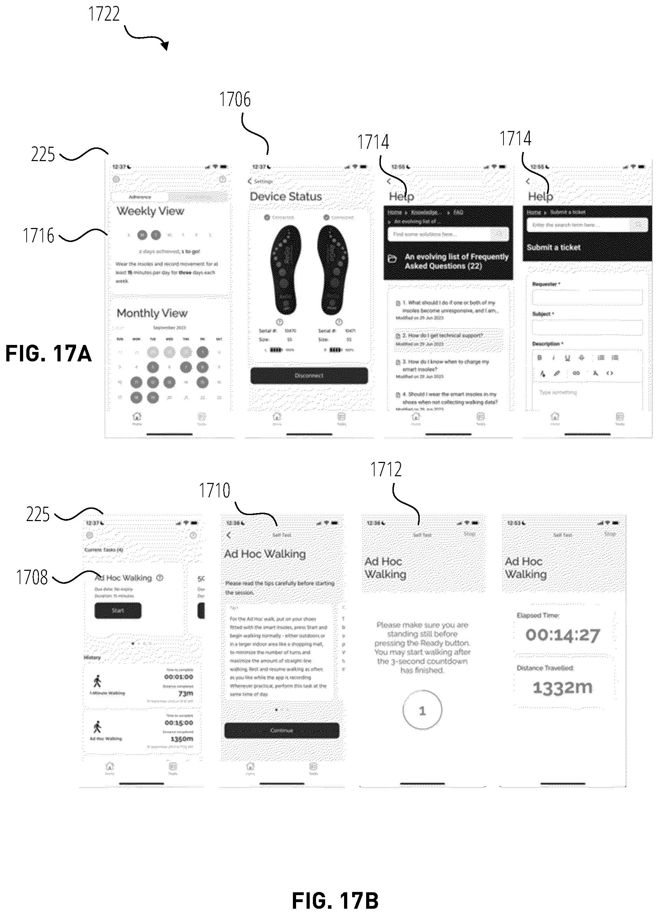

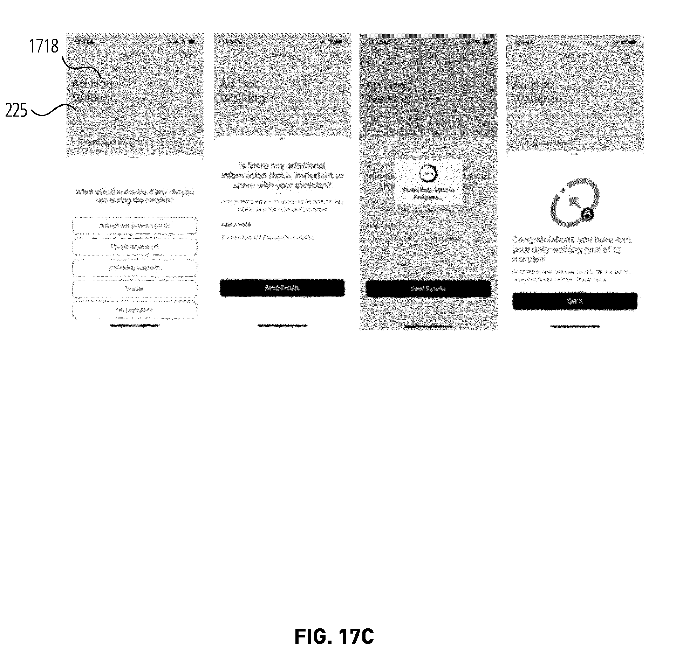

FIG. 17A and FIG. 17B and FIG. 17C, generally, illustrate one or more steps set forth by a user interface 225 of a mobile app 1722 of an embodiment of the present disclosure.

The present disclosure further comprises a system comprising a computing device configured to perform the method steps set forth above. In certain embodiments, the computing device and any databases are cloud-based.

Also provided is a non-transitory, computer readable memory and/or medium having recorded thereon statements and instructions for execution by a computer the method steps detailed above.

In the patient environment 404, as was illustrated in FIG. 4, the user interface 225 of the mobile app 1722 may display one or more interactive screen displays, such that a user may view scheduled exercises, progress, processed data, as well as device settings.

The one or more steps executed by the app 1722 may comprise set-up steps, including but not limited to pairing Bluetooth enables insoles with the app, setting up a user profile with user data (age, weight, height, etc.), and setting up a clinician profile (electronic medical records, goals which may be sent to the user, and the like). The one or more steps may comprise prompts to the user (walk, stand, balance, etc.) or one or more insights to the clinician.

FIG. 17A illustrates a user interface 225 for adherence tracking 1716 by the 1722.

The mobile app 1722 may tracks adherence to a walking goal, which may be set by a clinician or by the user.

The mobile app 1722 may include a device status (size, serial number, battery level) page with a built-in help page 1714, which may, upon a user selecting the help function, trigger a mechanism alerting a need for technical support. The help page may further display frequently asked questions (FAQ).

Clinicians may set, on the clinician portal 414, expectations for patient exercise, which may then be sent, over a network 136, to the patient environment 404, and viewed on the adherence tracking 1716 page by the patient. Patients may record their exercise data in the adherence environment, by pairing their smart insoles 116 with their mobile device and the mobile app, where it may be sent, automatically or manually, to the clinician portal 414, over a network.

Still further, the mobile app 1722 may first send the patient data to the cloud-based computing device 152, where the plurality of algorithms as illustrated in FIG. 1 and 2. May process the data and assign a composite score 276 to the data, before sending the score 276 to the clinician portal.

Preferably, but not necessarily, a step triggering a reminder to the patient to conduct an exercise, occurs automatically after a pre-determined (either by the clinician or by an ML algorithm 144), and the steps sending the data to the cloud, processing the raw data, and sending the score to the clinician portal, and occur automatically once the exercise is performed, thereby minimizing the clinician's time spent sending reminders.

FIG. 17B illustrates an aspect of a user interface 225 in which the mobile app 1722 displays interactive gait-test prompts 1708. In addition to ad-hoc walking, patient's may be asked to perform various gait-tests.

Clinicians may set, on the clinician portal 414, expectations for patient exercise, which may then be sent, over a network 136, to the patient environment 404, and viewed on the adherence tracking 1716 page by the patient. Patients may record their exercise data in the adherence environment, by pairing their smart insoles 116 with their mobile device and the mobile app, where it may be sent, automatically or manually, to the clinician portal 414, over a network.

Still further, the mobile app 1722 may first send the patient data to the cloud-based computing device 152, where the plurality of algorithms as illustrated in FIG. 1 and 2. May process the data and assign a composite score 276 to the data, before sending the score 276 to the clinician portal.

FIG. 17C illustrates an aspect of a user interface 225 in which the mobile app 1722 displays interactive supplemental gait-test information 1718. Patients may be prompted, after completing an exercise or a gait test, to answer some supplemental questions, which may include, but are not limited to, assistive walking devices used, additional notes to send to the clinician, identifying from a selectable dropdown list further symptoms the patient is experiencing, and the like.

The data obtained walking prompts, additional gait-test prompts, and supplemental questions may be sent to the cloud-based computing device 152 and processed by the one or more algorithms before sending the score 276 to the clinician portal.

Any of the processed results may be sent to the user patient environment 404 of the system as well.

The mobile app 1722 may further comprise a prompt for the clinician prior to releasing the processed patient data to the patient environment 404.

Requests may be triggered by the mobile app 1722 after periods of inactivity, or triggered by missing or incomplete data identified by one or more machine learning algorithms before completing an assessment or a time-based (progression) assessment of the patient's composite movement quality score 276.

FIG. 18 displays, graphically, correlation results comparing CI 276 and EDSS 1828 to MSWS12 questions 1824.

Extended Disability Status Scale (EDSS 1828) represents a global standard for MS neurologist patient assessments. Each vertical line represents a different trial participant.

MSWS-12 1824 is a clinically validated Patient Reported Outcome (PRO) measure of MS walking quality.

For each of the CI 276 and the EDSS 1828, results for Spearman Rho 1822 were plotted against each MSWS12 question 1824.

x marks 1830 represent a significance threshold of $p < 0.05$, triangle marks 1836 represent a significance threshold of $p < 0.01$, and circle marks 1838 represent an acceptance of the null hypothesis. Absolute values for rho are used for visualization; all CI rho values are negative.

The composite index score 276 as illustrated, is closely aligned with the MSWS-12 assessment. The primary outlier (out of 40 participants) in the illustrated graph may be attributed to the heightened sensitivity of the score 276 to balance impairments, the score 276 identifying balance impairments not captured by present models (such as MSWS-12).

FIG. 19 depicts, graphically, the CI scores calculated for each participant during an indoor 500 metre walk test.

In FIG. 19, CI scores 276 are sorted based on their Extended Disability Status Scale (EDSS 1828). Boxplots drawn in Blue represents healthy participants 1902, Green represents an EDSS score < 3.5 1904 (low disability level), Yellow represents 3.5 < EDSS > 4.5 1908 (medium disability level), and Red represents an EDSS > 4.5. EDSS 1906 (high disability level).

In the illustrated examples, the presented framework uses raw pressure, accelerometer, and gyroscope data streamed to a mobile device 264 to analyze the gait patterns of PwMS.

Through one or more ML algorithms 146 (including group 1 algorithms 248, group 2 algorithms 244, group 3 algorithms 240, group 4 algorithms 242, group 5 algorithms 246), raw data 126 is segmented into activity type, gait cycles, and the like, and analyzed to produce a plurality gait metrics.

These gait metrics are further processed by the group 6 algorithm 250 in order to conclusively assign a composite score 276, as illustrated in FIG. 19 to be a single overarching metric termed the composite movement quality score 276, or composite index 276 (CI), to provide useful and actionable insights to patients and clinicians. The CI 276 may be displayed on a mobile device 264 or a computing device 112, in a graphical user interfaces 225.

The CI 276 may be trained by a support vector machine (SVM) in order to classify healthy participants and PwMS; the participant's projected location on the hyperplane calculates a Z-score, translated to a value between 0-100%.

Preferably, the SVM measures and analyzes a plurality of features relating to a user's mobility. In the Example 3. Below, the chosen SVM uses 22 features and achieves an accuracy, F1-score and κ of 95.58%, 95.59%, and 0.909, respectively, suggesting that the trained SVM model is highly accurate and reliable for classifying the tested population.

Known methods have not, thus far, been capable of developing a framework for a composite index, interpretable by clinicians and patients, in part because they did not incorporate means of evaluating healthy patients as well as PwMS.

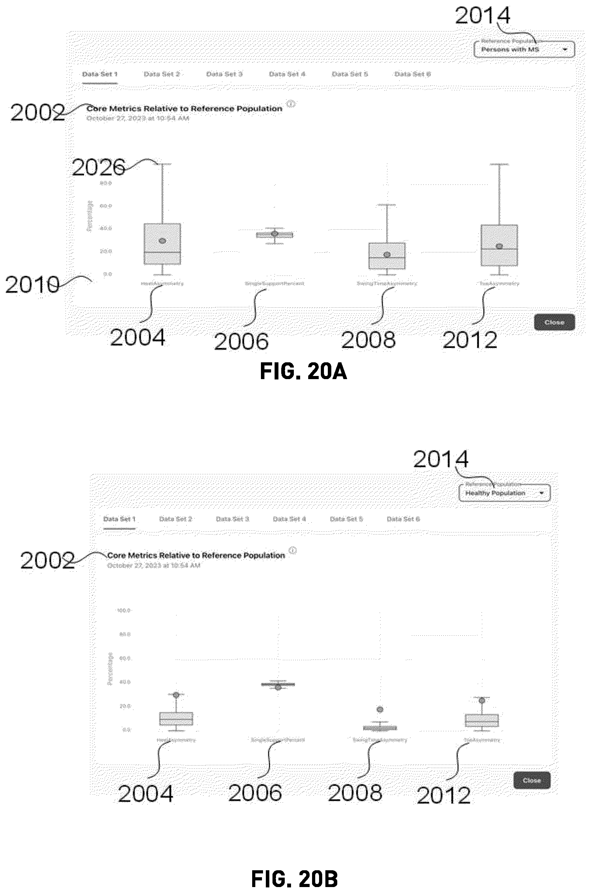

The features of the model in the illustrated embodiment are from all major gait domains as reported by several research endeavors: pace, rhythm, asymmetry, and variability (e.g., Kushioka et al., 2022; Lord et al., 2014; Monaghan et al., 2021; Shema-Shiratzky et al., 2019), and can be seen in greater detail in FIG. 20A and FIG. 20B.

It can be seen by way of the examples below, that the composite score 276 of the present disclosure exhibits a higher ML performance metrics than others previously presented (e.g., Trentzsch et al., 2021).

Future iterations of the composite index 276 may comprise tuning the one or more algorithms for processing the data received by group 6 algorithm: composite score 250, to be sensitive to identifying diseases or disease states.

Future iterations of the composite index 276 may comprise training the CI 276 with data from a multisite project, in order to capture weekly walking patterns of PwMS over several months.

As the CI evolves, more/different features may be included in the SVM model to improve accuracy of the assessments.

Measuring gait quality in PwMS with a CI 276 produces an objective outcome measure that is very strongly correlated to accepted disability metrics.

The CI score 276 can be used to determine trends in gait quality but also as a starting point to investigate which metrics are causing the CI to increase/decrease (e.g., increase in double support time, reduction in cadence), an opportunity not available to traditional walking tests that only measure time and distance.

Further, a wearable solution to assess gait quality removes several barriers for PwMS when interacting with the healthcare system, allowing for remote monitoring and an increased frequency in gait evaluations, allowing for a healthcare approach that can be proactive rather than reactive.

In accordance with an embodiment of the disclosure, gait algorithms 606 may be modified to be robust to residual pressure (i.e., significant pressure when the foot is off the ground). Modifications may comprise a decision matrix which first identifies toe-offs using a foot pitch (flexion-extension) gyroscope 906, then deciding whether to use an unmodified gait algorithm 606 if there is no residual pressure or identifying the breaking phenomenon in the forward accelerometer as the heel strike, then using the unmodified gait algorithms 606 for all other aspects. Modifying the gait algorithm to be able to identify toe-off may significantly improve the performance of the methods in real-life use.

In accordance with another embodiment of the disclosure, the composite movement quality score 276 or CI may be converted into a prediction of a patient's EDSS 1828, within specific ranges (low / moderate / high) as well as their MSWS-12 score.

To gain a better understanding of the disclosure described herein, the following examples are set forth. It will be understood that these examples are intended to describe illustrative embodiments of the disclosure and are not intended to limit the scope of the disclosure in any way.

**EXAMPLES**

**EXAMPLE 1: Comparison of results obtained using the system and method of the present** disclosure **compared to a markerless motion capture system.**

Ten healthy individuals with no current musculoskeletal injury or neurological disorder were recruited to perform 39 overground walks on a six-metre walkway with two embedded force plates (FP-4060, Bertec, USA) at their preferred walking speed. Participants wore a pair of instrumented insoles with pressure, accelerometer and gyroscope sensors (Neurogait 3.0, Salted, South Korea) while their whole-body kinematics were simultaneously collected using eight video cameras (Vue, Vicon, UK). The video camera data were processed using Theia 3D (C-motion, USA) to identify joint landmarks and Visual 3D (C-motion, USA) was used to automatically identify gait events (heel strike and toe off) based on kinematics and force plate data (all events were visually identified and adjusted as necessary). Instrumented shoe insole data were analyzed using the methods described in the present application. That is, gait events were segmented into heel strike, foot on floor, heel raise, and toe off events based on pressure, accelerometer, and gyroscope thresholds and logical events. Using the gait events from each technology, the same temporal variables were calculated and compared using a 3,1 intraclass correlation ($ICC_{(3,1)}$) in Python. A total of 1206 gait cycles were used for each comparison. An average $ICC_{(3,1)}$ value of 0.955 was calculated across all metrics representing an excellent reliability between motion capture systems. Results averaged between left and right side are presented in Table 2.

Table 2. Instrumented Shoe Insole Validation Compared to a Markerless Motion Capture System

| Metric | $ICC_{(3,1)}$ | CI95% |
|---|---|---|
| Stride Time | 0.999 | [0.995, 1.000] |
| Stance Time | 0.992 | [0.970, 1.000] |
| Swing Time | 0.984 | [0.935, 0.995] |
| Stance Percent | 0.911 | [0.690, 0.980] |
| Swing Percent | 0.911 | [0.690, 0.980] |
| Single Support Time | 0.982 | [0.935, 1.000] |
| Double Support Time | 0.935 | [0.760, 0.980] |
| Single Support Percent | 0.925 | [0.730, 0.980] |
| Double Support Percent | 0.918 | [0.705, 0.980] |
| Step Time | 0.999 | [1.000, 1.000] |
| Cadence | 0.989 | [0.960, 1.000] |

Note: Values are averaged between left and right sides. The markerless motion capture system used is Theia 3D (c-motion, USA). Percent represents the percentage of gait cycle from heel strike to heel strike of the same foot. $ICC_{(3,1)}$ represents an intraclass correlation coefficient of fixed raters where those raters are the only of interest. CI95% represents 95% confidence intervals of the ICCs. ICC thresholds: poor: < 0.50, moderate: 0.50-0.75, good: 0.75-0.90, and excellent: >0.90.

**EXAMPLE 2: Temporal and Spatial Metrics of Unassisted vs Assisted Gait**

A case study a participant with multiple sclerosis (PwMS) who was recruited for an initial investigation is described here. Specifically, a sub-portion of our protocol where individuals were asked to walk over a six-metre walkway with two embedded force plates (FP-4060, Bertec, USA) at their preferred walking speed is presented. The same methods as example 1 were used here. That is, video camera and instrumented insole data were simultaneously collected and their respective processing steps were performed to obtain gait events and thus calculate temporal variables. However, this example focuses on the results obtained from the instrumented insoles.

The participant of interest arrived at the laboratory with an ankle foot orthosis (AFO; unilateral left side) and a cane (unilateral right side). The individual was asked to perform the overground walk six times with two repetitions of unassisted, assisted with an AFO, and assisted with AFO + cane, respectively.

It is demonstrated in Table 3. below how methods of the present disclosure have the ability to detect how an assistive device can alters an individual's gait pattern. Notably for this case, the individual exhibited a decrease in variability (i.e., lower standard deviations) for the majority of temporal gait metrics.

Table 3. Objective Difference Between Conditions

Objective Differences Between Conditions

| | | | Temporal Metrics | | | | | | |
|---|---|---|---|---|---|---|---|---|---|
| | Asymmetry (%) | Cadence (step/min) | Double Support Time (s) | Single Support Time (s) | Stance Time (s) | Swing Time (s) | Stance Percent (%) | Swing Percent (%) | Step Time (s) |
| Unassisted | 7.12 | 94.87 (13.4) | 0.23 (0.03) | 0.52 (0.07) | 0.75 (0.08) | 0.50 (0.09) | 59.8 (4.76) | 40.3 (4.76) | 0.64 (0.09) |
| AFO | 4.52 | 90.57 (7.58) | 0.22 (0.04) | 0.58 (0.07) | 0.80 (0.04) | 0.52 (0.04) | 60.3 (2.41) | 39.7 (2.41) | 0.60 (0.06) |

(continued)

Objective Differences Between Conditions

| | Temporal Metrics | | | | | | | | |
|---|---|---|---|---|---|---|---|---|---|
| | Asymmetry (%) | Cadence (step/min) | Double Support Time (s) | Single Support Time (s) | Stance Time (s) | Swing Time (s) | Stance Percent (%) | Swing Percent (%) | Step Time (s) |
| AFO + Cane | 1.06 | 95.49 (7.44) | 0.16 (0.06) | 0.58 (0.06) | 0.74 (0.02) | 0.54 (0.01) | 57.9 (0.64) | 42.0 (0.64) | 0.60 (0.05) |

AFO: Ankle Foot Orthosis, 1 cane used unilaterally

## EXAMPLE 3. Composite Movement Quality Score Development

In this example, a study evaluating the effectiveness of developing a framework for a composite movement quality score 276 is disclosed.

Significant technical challenges are present in the development of such a framework because laboratory data is required to ensure the spatiotemporal (or any variable) used to train a machine learning (ML) algorithm is valid

Good-quality labels are needed when training ML algorithms. A large enough sample size is also needed. In the illustrated example, a clinician provided participants' disability status, and their perceived walking ability through a survey (MSWS12).

The best decisions need to be made in terms of feature selection, hyperparameters, and ML architecture.

As disclosed below, the example overcomes these challenges by: monitoring both healthy individuals and PwMS to determine 22 key metrics in evaluating and determining a composite index. The example illustrates a projection of participants onto the decision boundary, produce a Z score from and expressed as a 0-100%.

Participants: 22 healthy and 19 PwMS were recruited for this investigation. Healthy was defined as any individual who has not experienced a musculoskeletal injury within the last six months at the time of testing and does not suffer from a neurological disorder. PwMS were recruited from The Ottawa Hospital, who had undergone a neurological exam within the last 12 months, where they were given an EDSS score of less than 7.0; the average EDSS score was 3.76 ($\pm$1.71). Demographic information for all participants is displayed in Table 4.

Table 4. Participant Demographics

| Population | Age | EDSS |
|---|---|---|
| Healthy | 28.1 (6.66) | - |
| PwMS | 54.2 (16.5) | 3.76 (1.71) |

Movement Protocol and Instrumentation:

The original insole inside the participant's shoe was replaced with an instrumented shoe insole 116, ( in the present example, the insole was an off-the shelf insole from ReGo, Moticon, Germany; 50 Hz), which streamed raw pressure, accelerometer, and gyroscope data to a mobile application (developed by Celestra Health, Canada).

If the shoe insole could not be removed, the instrumented insole was placed on top of the existing shoe insole. Participants were asked to perform walking tasks in the motion capture laboratory (i.e., overground and treadmill walks), in the indoor hallways outside the laboratory (500- and 125-metre walks), and outdoors on a paved multiuse pathway (500-metre walk; healthy only).

In the laboratory, all participants performed at least six overground walks measuring six metres over two force plates (FP-4060, Bertec, USA; 1000 Hz).

If participants arrived with an assistive device, the six walks were split evenly to progress the participant from unassisted to fully assisted (e.g., a participant with a cane had three repetitions unassisted and three with a cane).

All healthy (optionally PwMS) performed a seven-minute walk on a treadmill at their preferred walking speed. Whole-body kinematics were collected using an eight-video-camera system (Vue, Vicon, UK; 50 Hz) and analyzed using Theia3D (Theia, USA).

In the hallways adjacent to the laboratory, participants were asked to walk up to 500 metres around two pylons placed 25 metres apart, making left- and right-hand turns around the pylons (i.e., making a figure 8). Participants were instructed to walk comfortably without stopping; chairs were set up along the hallway to provide rest if needed, and a research assistant walked with participants at higher disability levels.

Participants were encouraged to perform the 500-metre walk without assistance; however, they were not prevented from using an assistive device if requested. All healthy (optionally PwMS) also performed a 125-metre walk indoors, which included several turns (left and right), a stair ascend, and a stair descend section. Only healthy participants performed a 500-metre walk outside, which contained four right-hand turns and a slight uphill and downhill section on a paved multiuse pathway.

Data Analysis

*Markerless Motion Capture Data*

For the movements performed in the laboratory, force platform data and kinematics calculated through Theia 3D were imported into Visual3D (C-motion, USA). Gait events were initially identified using the automatic gait detection pipeline and imported into Vicon Nexus 2 (Vicon, UK), where they were visually verified using video data and manually adjusted as necessary. Gait event and spatial data were then exported to Matlab(MathWorks, USA) and used to calculate spatiotemporal variables.

*Instrumented Insole Data*

Instrumented insole data were streamed via Bluetooth to the Celestra Health mobile application on a mobile device 264. Raw pressure, accelerometer, and gyroscope data were exported to a .csv file, which was imported into Python 3.10.

A human activity recognition (HAR) layer with a fully connected neural network (NN) architecture identified standing, walking, turning, stair ascend, and stair descend activities. The raw pressure, accelerometer, and gyroscope data were passed into an NN with a softmax decision layer with a logical layer to filter activities with short window sizes. For the present example, only the walking activity label is discussed.

Following HAR, data identified as walking passed through a gait detection algorithm developed based on the work of Chatzaki et al. (2021). In short, the pressure sensors were grouped into heel, midfoot, and toe segments and the developed algorithm used threshold values (i.e., 7% maximum pressure) to trigger different gait events based on the previous state parameter: heel strike (HES), foot-on-floor (FOF), heel raise (HER) and toe off (TOF).

As acknowledged by Chatzaki et al. (2021), individuals may not strike the floor with their heel to trigger an HES event; therefore, the gait detection algorithm accommodates HES events triggered by a toe strike.

Building on the work done by Chatzaki et al. (2021), an embodiment of the algorithm of the present disclosure includes a midfoot pressure group, which allows for HES events to be triggered by midfoot-strike events.

Still further, in another embodiment, the algorithm may comprise a midfoot pressure group combined with stricter parameters were also set for FOF events: vertical acceleration has to be between 1.1 and 0.9 m/s$^2$, and pitch gyroscope had to be between 20 and -5 deg/s. These additional parameters may be used to aid in the reliability of the FOF event, as this event is critical to the performance of the sensor fusion algorithm discussed below.

In another embodiment, an algorithm of the present disclosure combines one or more of: pressure groups, strict FOF parameters, and IMU measurements. The resulting algorithm results in highly accurate and robust assessments and evaluations of gait in the wild.

Following gait detection, the accelerometer and the gyroscope data are fused using a Madgwick filter to calculate the position of the insoles from an arbitrary starting position using methods described by (Rebula et al., 2013).

Participants are instructed to stand for three seconds for all trials, which was used to determine and remove signal biases from all IMU channels. Following bias removal, the accelerometer was rotated to an earth reference frame, and strict FOF periods were identified. These FOF periods were calculated by first high-pass filtering (cut off 0.001 Hz) and then lowpass filtering (cut off 5 Hz) the Euclidean norm of the accelerometer; subindices of the FOF segment that fell below 1.5 times the median absolute deviation of that FOF segment was used as periods of zero velocity to update the orientation of the fusion algorithm. Initial convergence of the Madgwick filter was done over the middle 75% of the three-second stationary period using a gain of 0.057; this gain value was used for each strict FOF period, while a gain of 0.018 was used during all other phases.

As per Rebula et al. (2013), the orientation was used to rotate the accelerometer within the earth's reference frame and was integrated to calculate velocity using the trapezoidal method. At each period of strict FOF, velocity was set to 0 m/s and the data were detrended between each FOF event (Rebula et al., 2013). The corrected velocity was then integrated to calculate the insole's position over time.

For standardization, walking data were split into 10-second segments to ensure that central tendency measures from minutes-long data weren't being compared to those from seconds. For each 10-second segment, 15 spatiotemporal gait metrics (e.g., step time, stride time, swing time, stance time, single and double support time, etc.), six pressure metrics (i.e., the average and average peak area under the curve during stance phase for each pressure group), six IMU metrics (i.e., average peak, and range, root mean squared error, and meanSD for the accelerometer and gyroscope), and their respective variability metrics when appropriate (i.e., asymmetry, and standard deviation), resulting in 128 gait metrics. When appropriate, each metric's average and standard deviation between feet and across the 10-second segment were calculated, resulting in 90 metrics.

Although segments are 10 seconds in the above example, it should be readily understood that the data may be segmented into shorter, segments, i.e. 1-10 second segments, and longer, i.e. 10-120 second segments, and still further, the segments may be shorter than 1 second and longer than 120 seconds.

*Composite Index (CI): Training*

A support vector machine (SVM) was trained to classify the gait metrics of healthy participants and PwMS. The training data consisted of 1743 rows (899 healthy; 843 PwMS) of 90 gait metrics representing 10-second walking segments retrieved from all walking trials. Feature selection was performed to reduce the feature space by removing redundant or irrelevant features in three steps: 1) T-tests identified 84 metrics as significantly different ($p < 0.05$) between groups; 2) Pearson correlations were run, and those metrics that had an absolute r value $\geq 0.99$ were removed, resulting in 69 metrics; and 3) an iterative partial least squares (PLS) regression was used to iteratively identify which subset of features best separated the class labels (i.e., incrementally adding one feature up to 69 features). An SVM was trained using each subset of features identified through PLS. For each feature set, features were scaled using sklearn's standard scaler (Pedregosa et al., 2011) and the SVM's hyperparameters were optimized using a grid search and cross-validated using a leave-one-out approach, where entire participants' data were iteratively removed; assessed hyperparameters are listed in Table 5.

Table 5. Hyperparameters

| Parameter | Value |
|---|---|
| C | **0.1**, 1, 10 |
| Gamma | **0.001,** 0.01, 0.1, 1 |
| Kernal | **Linear,** radial basis function, polynomial |
| Note: grid search was performed using sklearn's GridSearchCV method. Bolded values are the hyperparameters used for the final model. ||

For each PLS iteration, the best hyperparameters from the grid search operation were selected based on accuracy and F1, resulting in 69 trained SVM models to classify PwMS and healthy participants.

CI values were calculated for all participants across all 69 SVM models. To calculate the CI, the training data was projected onto the trained SVM's hyperplane to calculate its mean and standard deviation. Using the mean and standard deviation of the projected training data, the Z-score of a participant's projected data is calculated. To receive a score of 0-100%, the cumulative distribution function was applied to the Z-score. To select the final SVM model, the accuracy, F1-score, and correlation values (Pearson or Spearman used when appropriate) comparing the CI scores to the PwMS's EDSS and MSWS12 scores and all participant's cumulative Z-score across all metrics (i.e., Z-scores were calculated for each metric and averaged then compared to CI) were evaluated.

Results: Accuracy and Correlation

Spearman Rho ($\rho$) values were interpreted as very strong ($\rho > 0.69$), strong ($0.40 < \rho \leq 0.69$), moderate ($0.29 < \rho \leq 0.40$), weak ($0.19 < \rho \leq 0.29$), and negligible ($0 \leq \rho \leq 0.19$). The significant cut-off to reject the null hypothesis was set to $p < 0.05$; if $p < 0.01$, the results were interpreted as highly significant. The SVM's accuracy, F1, and Cohen's Kappa ($\kappa$) score were calculated using the sklearn library.

*Composite Index (276)*

An SVM with 22 features (Table 6) was chosen to classify Healthy Controls vs. PwMS. using a linear kernal with a regularization parameter (C) of 0.1 and a gamma or 0.001; the model is 95.58% accurate with an F1-score of 95.59% and a $\kappa$ of 0.909.

Table 6. Gait Metrics Chosen for SVM

| Gait Metric | Gait Metric Domain |
|---|---|
| Swing Phase Percent Asymmetry | Asymmetry |
| Toe Pressure Asymmetry | |
| Heel Pressure Asymmetry | |
| Distance travelled in 10 seconds | Pace |
| Cadence (steps/minute) | |
| MeanSD Accelerometer Euclidean norm | Movement variability |
| MeanSD Gyroscope Euclidean norm | |
| RMSE Accelerometer Euclidean norm | |
| RMSE Gyroscope Euclidean norm | |
| SD of Peak Heel Pressure | Pressure variability |
| MeanSD Toe Pressure | |
| SD of Right foot's Percent Toe pressure | |
| MeanSD Heel Pressure | |
| MeanSD Midfoot Pressure | |
| RMSE Midfoot pressure across steps | |
| Percent time Single Support | Rhythm |
| Double Support Time (seconds) | |
| Stance Time (seconds) | |
| Average Peak Midfoot Pressure | |
| Step Time (seconds) | |
| Average Peak Accelerometer Euclidean Norm | |
| Average Peak Gyroscope Euclidean Norm | |

Note: Gait metrics in the present example were averaged across sides of the body unless otherwise stated. RMSE, SD, MeanSD, and average peak, are calculated using gait cycles within each 10 second walking bout. RMSE = root mean squared error; SD = standard deviation, MeanSD = average standard deviation across all gait cycles for each normalized time point.

The CI 276 values had a very strong significant negative correlation with the EDSS score: $\rho$ = -0.805 ($p$ < 0.001). In relation to the MSWS12 scores, the CI values had ten significant negative correlations (seven were highly significant) that ranged from strong to very strong (i.e., $\rho$ > 0.40); on average across all questions, $\rho$ = -0.602 ($\pm$0.154; range: -0.343 - -0.807).

Similarly, in relation to the MSWS12 scores, EDSS had nine significant positive correlations (seven were highly significant); on average across all questions, $\rho$ = 0.550 ($\pm$0.166; range: -0.246 - -0.789).

When MSWS-12 results are expressed as a single value (i.e., the sum of all answers), EDSS was significantly and strongly positively correlated ($\rho$ = 0.688, $p$ = 0.001), and CI was significantly and very strongly negatively correlated ($\rho$ = -0.721, $p$ < 0.001).

The present example included young-healthy individuals in the dataset, as well as PwMS at high disability levels, in order to develop a comprehensive CI score. Different disease states and an "ideal" walking pattern were necessary to understand the distribution of walking ability.

The resulting CI 276 had a very strong negative correlation compared to the widely accepted EDSS score ($\rho$ = -0.805) and had superior correlation values than EDSS across all MSWS12 questions (CI: $\rho$ = -0.602 $\pm$0.154; EDSS: $\rho$ = -0.550 $\pm$0.166), the CI followed similar trends to the EDSS across all MSWS12 questions with an average absolute difference between correlation coefficients being 0.091 ($\pm$0.060), and when MSWS12 is represented as a single value, the EDSS and CI scores had strong to very strong correlations (EDSS: $\rho$ = 0.688; CI: $\rho$ = -0.721). The correlations found in the present study are similar in significance albeit lower for the correlation coefficient to previous research correlating EDSS

and MSWS12 ($\rho = 0.78$ $p = 0.0001$; Motl and Snook, 2008), likely driven by the considerable differences in population size (19 PwMS vs 132).

The present disclosure illustrates, significantly, that MSWS12 and EDSS may be excellent predictors of one another, while the MSWS12 provides much-needed context to perceived mobility dysfunctions.

Further, the CI's very strong correlations to EDSS and MSWS12 display its utility for objectively quantifying a PwMS's gait quality while not confining patients to burdensome clinical visits.

FIG. 20A and FIG. 20B illustrate an aspect of a user interface for displaying a composite movement quality score 276 platform, trained on a combination of metrics, to identify core metrics pertaining to gait data from reference populations 2014, in accordance with an embodiment.

FIG. 20A illustrates core four core metrics associated with the reference population 2014 being persons with MS, while FIG. 20B illustrates four core metrics associated with a reference population 2014 being a healthy control population.

This information may be displayed in a clinician portal 414 of a system according to an aspect of the disclosure. Clinicians may select, in the user interface 225 of the clinician portal 414, a given composite index walking score 276 to view underlying metrics and how they compare with both healthy population and MS populations. The interactive platform transforms the raw data to a tangible score, without losing the underlying metrics, allowing clinicians to make informed assessments of the patient's CI score 276 with ease.

Still further, metrics used for training a machine learning model to identify core metrics pertaining to gait data, may comprise entering: 15 spatiotemporal gait metrics (e.g., step time, stride time, swing time, stance time, single and double support time, etc.), six pressure metrics (i.e., the average and average peak area under the curve during stance phase for each pressure group), six IMU metrics (i.e., average peak, and range, root mean squared error, and meanSD for the accelerometer and gyroscope), and their respective variability metrics when appropriate (i.e., asymmetry, and standard deviation), resulting in 128 gait metrics.

When appropriate, each metric's average and standard deviation between feet and across the 10-second segment were calculated, resulting in 90 metrics.

A machine learning model may, according to some embodiments, identify 22 core metrics to separate MS patients from a healthy control population. This example shows identified 4 core metrics 2002, heel asymmetry 2004, single support percent 2006, swing time asymmetry 2008, toe asymmetry 2012.

In the illustrated embodiment, it can be seen that the MS patient reference population 2014 gait is highly consistent with MS population but significantly different from the healthy participants.

Further, by analyzing the box plot's whiskers 2026 for the MS population, it can be seen that the variability in PwMS is much greater than in healthy people, resulting in many challenges when categorizing data without a significant and robust database comprising a variety of test environments and movement activity to train the ml algorithms 144.

The present disclosure comprises novel systems and methods for measuring and monitoring gait. The present disclosure incorporates a plurality of data layers to calculate gait metrics in the wild (i.e., activity recognition, gait detection, assistive device detection). Furthermore, the present disclosure teaches systems and methods for detecting assistive device usage, phenotyping in the wild, and assessing prolonged walking and how that changes phenotypes/spatiotemporal metrics.

Further to the novel systems and methods for measuring and monitoring gait is a novel system and method for evaluating gait in the form of a practical, actionable, SVM-trained CI score for gait quality specific to neurological disorders, as well as longitudinal tracking of CI scores compared to baseline values. Insights and analyses may also be determined and displayed.

In still further embodiments of the disclosure, there is taught unique user interfaces with patient and clinician environments and portals, where CI scores, their meanings, and their methods of calculation may be displayed.

In still further embodiments of the disclosure, there is taught unique user interfaces for patients which may automatically trigger reminders for exercises, measure exercise data, and send the measured data to cloud computing devices for processing and transformation into a score and assessment.

In accordance with one or more embodiments of the disclosure, there are described systems and methods for the development of a data-driven scoring system for MS that provides more insights and more clarity to clinicians than existing scales (e.g., EDSS).

It will be appreciated that, while not explicitly considered in the examples described above, in some embodiments the CI score may be tailored or adapted to be specific to a patient's demographics. Parameters that may be considered include, without limitation: age, sex, location/geography, ethnicity or the like. In some embodiments, the CI score may be adapted to be disease specific as well.

Neurological conditions can be negatively impacted by weather (e.g., hot/cold temperatures, humidity, ultraviolet ray intensity, etc.). Further, terrain (e.g., elevation, ground type, slope etc.) has been found to alter an individual's gait pattern. Therefore, it is important to consider environmental conditions when evaluating gait quality in the wild. In some embodiments, the system of the present disclosure, in accordance with different embodiments, may use positional data such as GPS data obtained from the user's smartphone to retrieve relevant weather and terrain information.

In some embodiments, the computer device 112 may be communicatively coupled to one or more databases operable to provide, based at least in part on the user's location, weather and/or environmental information (i.e., terrain, weather, ambient temperature, humidity, etc.).

In certain embodiments, the weather and terrain information may be provided and displayed to the user and clinician on a user device to provide additional context when presenting the CI. In certain embodiments, the weather and terrain information may be displayed on the user device in the form of a dialogue box. In certain embodiments, the weather and terrain information may be used as a scaling factor applied to the CI to reduce the influence of weather and terrain on the user's gait quality.

In some embodiments, the system of the present disclosure may be configured to provide feedback to users or patients in the form of CI trend data only (increasing, decreasing, constant), without including the specific CI scores. This allows patients to see, for example, if their walking quality is improving, deteriorating, or staying the same, without bringing to the patient's attention the fact that their walking quality may be significantly worse than a healthy control.

IMPROVED INSOLE FRAMEWORK

In accordance with different embodiments, an improved Insole Framework (IF) is described below. The improved IF provides an improved technical means for accurately assessing a user's gait, as well as diagnose the assessment in terms of the user's disorder, progression of their disorder, injury, and progression of injury/ recovery from injury. As noted above, this improved framework automatically recognizes ambulatory activities (e.g., walking, turning, climbing stairs, etc.) using machine learning-based human activity recognition (HAR), then processes the data using standardized approaches to compute spatiotemporal (ST) metrics, using the same hardware infrastructure and instrumented shoe insoles (each comprised of pressure sensors and an inertial measurement unit (IMU)) as described above (and for example in FIGS. 1 and 2). However, the previously discussed embodiments showed validity of the gait detection algorithm results only for healthy individuals (see for example Table 2). The new improved embodiments discussed below further establish its validity for different groups, namely healthy individuals, but also people with multiple sclerosis (PwMS), and people with Parkinson's disease (PwPD). The skilled person in the art will appreciate that other groups or people with a designated disease that can affect gait may also be used to train the HAR models as well.

The new embodiments may further rely on one or more general or population/phenotype specific models for HAR, which provides improved IF's ability to identify ambulatory activities. In addition, the embodiment of the improved IF discussed below comprises additional improvements such as additional neural layers and an adjusted number of nodes per layer, although some other variations not discussed may be contemplated by the skilled person in art. Finally, the new improved IF is able to produce a CI that is trained on a plurality of reliable gait metrics and is highly correlated with clinically meaningful disability metrics. The improved model has also been evaluated for generalizability to unseen data in unstructured environments instead of indoors outside the laboratory. In this regard, the improved framework provides satisfactory results compared to the current standard of care (EDSS scores). That is, the improved framework achieves the same level of insight as the EDSS while being able to further describe all aspects of the PwMS's gait pattern, in contrast to only assessing that a user can only walk a designated distance. The new model has also been evaluated longitudinally in a healthy population and therefore provides a much better understanding of how it should behave long-term, providing a baseline to further develop progression-based algorithms.

The improved IF enables the assessment of prolonged walking in clinical and free-living settings, which may be better indicators of true walking performance compared to laboratory assessments. Compared to the gold-standard MoCap system, the improved IF was able to calculate all core and pace spatiotemporal gait metrics with good to excellent reliability ($\geq 0.824$) regardless of neurological status. This is in contrast with other known methods or systems that for example rely on multiple sensors placed all over the body of the patient to perform targeted assessments. These systems are difficult to use, and often need skilled personnel to calibrate the system to a biomechanical model so data can be collected. These systems can be slow to use, requiring at least 15 minutes or more for a skilled person to rig up the patient, do the movements, and tear down. Additional time is required to process all the data and get a CI. In contrast, the solution discussed below provides everything needed, and only requires that the patient puts the instrumented soles in their shoes (requiring less than a minute) and start walking in free-living conditions. The system automatically provides the assessment immediately after the walk is over, and thus can be used as an at-home solution, which is a notable technological improvement. The following sections A, B, C and D describe in detail the new IF and its improvements mentioned above.

SECTION A: VALIDATION

Section A is based on a study that describes the improved insole framework and provide data showing that the improvements allow to better calculate traditional spatiotemporal gait metrics in health and dysfunctional gait. The purpose of this work was to validate an Insole Framework (IF) that uses pressure and IMU data obtained from instrumented shoe insoles to detect ambulatory activities to segment walking trials, perform gait detection, and calculate spatiotemporal gait metrics. The results obtained from our proposed framework are compared to data from a laboratory-based motion capture (MoCap) system using data collected from three populations: healthy individuals (Healthy), PwMS, and PwPD.

**A2.0 Methods - A2.1 Participants** - Twenty-two Healthy, 19 PwMS, and 10 PwPD were recruited for this investigation. Healthy was defined as any individual who has not experienced a musculoskeletal injury within the last 6 months at the time of testing and does not suffer from a neurological disorder. PwMS and PwPD were recruited from The Ottawa Hospital and had undergone a neurological exam within the last 12 months where they were given an Extended Disability Status Score (EDSS) of less than 7.0 (PwMS; range: 0-10, with higher scores indicating higher levels of disability; Kurtzke, 1983) or a Hoehn and Yahr Scale (HY) score less than 3.0 (PwPD; range: 0-5, with higher scores indicating more severe disease progression; Hoehn & Yahr, 1967). While it was not used in the inclusion criteria, participants filled out the 12-item Multiple Sclerosis Walking Scale (MSWS-12) as a measure of perceived walking function (range: 0-100%, with higher values indicating increased walking impairment; Hobart et al., 2003); although designed for MS, PwPD were also asked to fill out the MSWS-12. The average EDSS for the PwMS was 3.8 $\pm$1.6 (range: 0-6), the average MSWS-12 score was 60.9% $\pm$22.2 (range: 20-90), and nine PwMS expressed using one or more assistive devices in their daily lives. The average HY score for the PwPD was 1.9 $\pm$0.32 (range: 1-2), the average MSWS-12 score was 40.7% $\pm$21.1 (range: 22-83), and no PwPD expressed daily usage of an assistive device. Participant demographics, disability metrics, and assistive device usage are presented in Table 1. Both the University of Ottawa (H-11-21-7565) and The Ottawa Hospital's (20220239-01H) research ethics boards approved this research.

**Table A1.** Participant demographics

| | Sex M\|F | Age (years) | Height (cm) | Mass (kg) | EDSS Score | MSWS-12 Score | MS Type | H-Y Score | Assisti ve device |
|---|---|---|---|---|---|---|---|---|---|
| Health y | 11\|1 1 | 28.1 (6.66) | 174.8 (9.47) | 75.7 (14.8) | - | - | - | - | N: 22 |
| PwMS | 6\|13 | 54.2 (16.5) | 158.0 (7.28) | 78.7 (26.4) | 3.8 (1.6) | 60.9 (22.4) | RR: 10; PP: 4; SP: 1 | - | AFO: 1; WS: 3; W: 4; C: 5; N: 10 |
| PwPD | 6\|4 | 63.9 (7.19) | 159.1 (9.96) | 75.0 (15.1) | - | 40.7 (21.1) | - | 1.9 (0.3 2) | N: 10 |

Values in **Table A1** are presented as mean (standard deviation) or total count when appropriate. M = Male; F = Female, PwMS = People with Multiple Sclerosis; PwPD = People with Parkinson's Disease; MS = Multiple Sclerosis; EDSS = Expanded Disability Status Score; MSWS-12 = 12-Item Multiple Sclerosis Walking Scale; MS Types: RR = relapse remitting, PP = primary progressive, SP = secondary progressive; Assistive devices: AFO = ankle foot orthosis, WS = two walking sticks, W = walker, C = cane, N = no assistive device. Note, some PwMS expressed using multiple assistive devices (e.g., cane for short walks/with a companion and a walker for longer walks/using shopping carts), each mentioning of an assistive device was counted (i.e., for the previous example, cane and walker would each be counted as one instance). Four PwMS expressed using multiple assistive devices.

**A2.2 Movement Protocol and Instrumentation** - Participants arrived at the University of Ottawa's Movement Biomechanics and Analytics Laboratory for a single day. All participants were then asked to remove their indoor footwear so the appropriately sized pair of instrumented shoe insoles (ReGo, Moticon, Germany; 50 Hz) could be placed inside their shoes. The sizes used in this investigation ranged from S2 to S7 (EU shoe size 34-45, US shoe size Women 4-13, Men 3.5-11.5). For each walking trial, both instrumented shoe insoles streamed raw data from 16 pressure sensors and a triaxial accelerometer and gyroscope to a mobile application (Celestra Health, Canada) installed on a laboratory-owned smartphone (iPhone 13, Apple, USA). Participants performed walking tasks in the laboratory (i.e., overground and treadmill walks), in the indoor hallways outside the laboratory (500- and 125-metre walks), and outdoors on a paved multiuse pathway (500-metre walk). Participants began and ended all walking trials by standing for approximately three seconds.

In the laboratory, all participants performed at least six overground walks measuring six metres over two force plates (FP-4060, Bertec, USA; 1000 Hz) placed in parallel. If participants arrived with an assistive device(s), they were asked if the six walks could be split evenly to progress them from unassisted to fully assisted walking (e.g., a participant with a cane would perform three unassisted walks and three with a cane). All healthy participants, optionally PwMS (N = 2) and PwPD (N = 0), performed a seven-minute walk on a treadmill at their preferred walking speed, determined as per Dingwell & Marin (2006) (i.e., iteratively speeding and slowing the treadmill to find the average preferred speed). Whole-body kinematics were collected using an eight-video-camera system (Vue, Vicon, UK; 50 Hz) and analyzed using Theia3D (Theia Markerless Inc., Canada), which has been validated for capturing gait metrics against marker-based motion capture systems.

In the hallways outside the laboratory, participants were asked to walk up to 500 metres around two pylons placed 25 metres apart; participants were asked to make alternating left- and right-hand turns around the pylons (i.e., making a figure 8). Participants were instructed to walk at a comfortable speed without stopping; a research assistant walked alongside participants at higher disability levels, and chairs were set up along the hallway at 5-metre intervals, allowing participants to stop and rest if needed. Participants were encouraged to perform the 500-metre walk without assistance; however, they were not prevented from using assistive devices. All healthy participants, optionally PwMS (N = 10) and PwPD (N = 9), also performed a 125-metre walk inside, which included several 90-180° turns in either direction, a stair ascent, and a stair descent section. Only healthy participants performed a 500-metre walk outside, which contained four main ~ 90° turns and a slight uphill and downhill section on a paved multiuse pathway.

Apart from the main protocol, a subset of healthy participants (N = 15) were invited to perform a stair ascent/descent protocol, where they walked up and down four flights of stairs twice under two conditions: assisted (i.e., actively using the railing to aid balance and propulsion) and preferred (i.e., no instructions given). This additional stair protocol was used to increase the training data for the activity recognition algorithm discussed below.

For all tasks outside the laboratory, participants were filmed by a researcher on a laboratory-owned smartphone to facilitate ground truth labelling for the human activity recognition (HAR) algorithm. Videos focused from below the neckline to the feet.

**A2.3 Framework Development** - The framework developed to analyze gait patterns using instrumented shoe insole data first identified the ambulatory activities performed during the walking trial, detected gait phases, fused the IMU data to obtain foot position, standardized the analysis into 10-second segments, and calculated gait metrics.

**A2.3.1 Human Activity Recognition (HAR)** - Activity labels were manually identified by synchronizing the instrumented insole data with the video recordings. Turning events were informed by the definition provided by Kluge et al. (2021) in combination with observing the shoulder and pelvis rotation and foot orientation from the recorded videos, as well as observing the IMU signals for signs of pattern changes in foot acceleration and angular velocity. Stair ascent and descent started at the swing phase of the first foot to contact the stair tread and ended at the heel strike of the last foot to hit the floor/landing. Standing was identified as a period when the feet were in double support, and no other step was immediately initiated. These activity labels were used as the ground truth for developing the HAR neural network model.

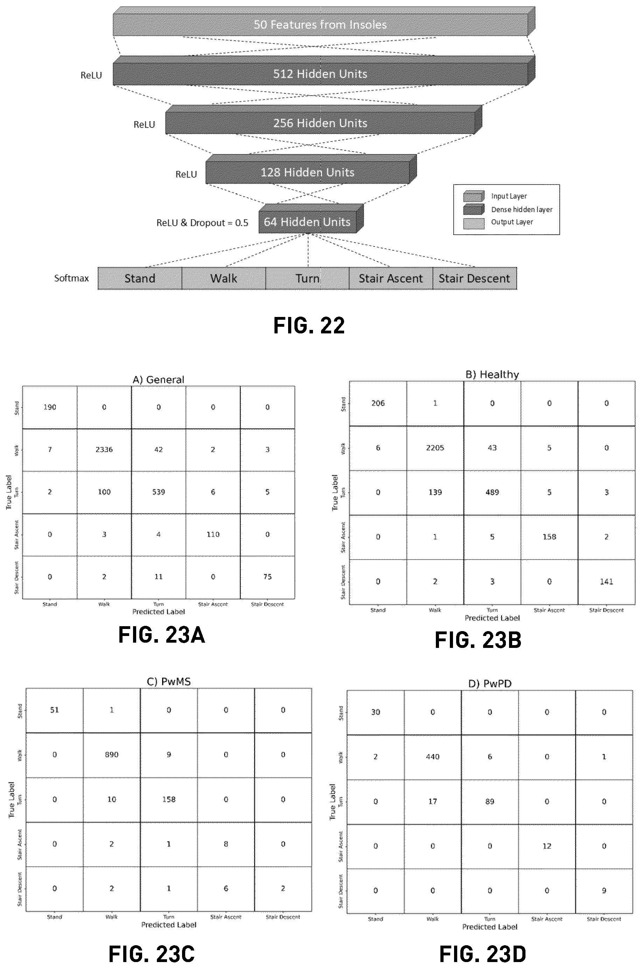

In Python, the sequential model from the Keras library was used to develop artificial neural networks (ANNs) with four fully-connected dense layers with a decreasing number of hidden units for layer-wise feature compression (Wang et al., 2023). This ANN was used to identify five ambulatory activities: walking, standing, turning, stair ascent, and stair descent. The model's first layer reshaped the input data for time-series analysis, followed by four dense layers with rectified linear unit activation functions for non-linear transformation. A time-distributed layer was used for dropout and regularization, followed by a flattening layer for vectorization. The final softmax layer performed the multi-class classification (FIG. 22). Input data (i.e., raw pressure and IMU data) from the instrumented insoles were first scaled by subtracting the mean and dividing by the standard deviation and then reshaped into a vector. Activity classifications were made using a sliding window approach. Window sizes for each HAR model were selected heuristically from a predefined set of values (52, 100, 152, 200, or 252 frames, corresponding to 1.04 - 5.04 s of data) based on prior research, shown in Table 2. The step size was always ¼ of the window size to balance overlap and computational efficiency. Windowed predictions were then reformatted as frames, and logic was applied to enforce real-world constraints (i.e., use knowledge of previous and next activity predictions to reevaluate short activities). To enforce standardization in the spatiotemporal analysis, periods identified as walking were indexed into 10-second segments.

Four HAR models were trained and evaluated: a General model (i.e., all participants included); a healthy-specific model; an MS-specific model; and a PD-specific model. A hold-out testing approach was used to train and evaluate the models, where the randomly selected participants included in each train, validation, and test subsets are shown in Table A2. Data from each participant were always kept in the same subset to avoid exposure during training. To accommodate for class imbalance (i.e., varying number of samples for each ambulatory activity), a class weight dictionary was computed using the training set and implemented during training using the "balanced" setting in the SciKit-Learn library. This setting implemented a heuristic method utilizing logistic regression to deal with rare events. The models were trained using a batch size of 24, maximum epochs of 500, and data were randomly shuffled before epochs to mitigate the learning of order effects. Loss was computed using the categorical cross-entropy loss function, where the gradients of the cost function were computed with respect to the parameters using backpropagation, where stochastic gradient descent updated the parameters to minimize loss. This was performed until the early stopping criterion was reached: training loss ceased to improve for five consecutive epochs. The model weights from the epoch with the lowest validation loss were saved for evaluation on the test set. Overall performance on the test set was evaluated using loss, accuracy, weighted averaged F1-score, and a confusion matrix; activity-specific performances were evaluated using precision, recall, and F1-score.

Table A2. The data divisions and sliding window sizes for training and evaluating the artificial neural networks using hold-out testing

| Model | Total Frames | Train Set | Validation Set | Test Set | Sliding Window Size (Frames) |
|---|---|---|---|---|---|
| Gener al | 1,823,62 6 | 78.59% (16 Healthy, 15 PwMS, 8 PwPD) | 11.98% (3 Healthy, 2 PwMS, 1 PwPD) | 9.43% (3 Healthy, 2 PwMS, 1 PwPD) | 200 |
| Health y | 727,654 | 76.74% (16 Healthy) | 11.74% (3 Healthy) | 11.52% (3 Healthy) | 100 |
| PwMS | 755,959 | 78.17% (15 PwMS) | 12.29% (2 PwMS) | 9.54% (2 PwMS) | 252 |
| PwPD | 340,013 | 79.96% (8 PwPD) | 8.74% (1 PwPD) | 11.30% (1 PwPD) | 252 |
| PwMS = people with multiple sclerosis; PwPD = people with Parkinson's disease | | | | | |

**A2.3.2 Gait Detection** - The gait detection algorithm used data from the pressure and IMU sensors to separate each gait cycle into four phases: heel strike (HES), foot on floor (FOF), heel rise (HER), and toe off (TOF), based on the definitions from Chatzaki and colleagues (2021). Pressure data were pre-processed by grouping the 16 pressure sensors into three sections: heel (sensors 1-4), midfoot (sensors 5-7), and toes (sensors 9-16). IMU data were filtered using a 4th-order dual-pass Butterworth filter with a 6 Hz cut-off.

Gait detection began by independently identifying the middle of the swing phase (midSwing) using pressure and IMU data. midSwing was considered to be the location of the local valley in the pitch gyroscope signal and the middle of a sustained period with pressure below 7% of the maximum observed pressure for that walking session. Logic was used to determine the final location of the midSwing using the information obtained from the pressure and IMU indices (i.e., relative location, relative values). Pre-swing was then discovered using methods described by Trojaniello and colleagues (2014), defined as the region where the pitch gyroscope is $\geq$ 50% of the local peak while rising toward that peak. The location where all pressure sensor groups are minimized was considered the start of the TOF phase (Salis et al., 2021).

After TOF, HES was independently determined by the pressure and IMU data. The IMU solution identified the valley in the forward accelerometer that occurs during the breaking phenomenon (Trojaniello et al., 2014). The pressure solution identified the first frame where any pressure group surpassed 7% of the maximum observed value in the trial (Chatzaki et al., 2021), which allowed any type of foot strike event (i.e., heel, midfoot, or toe strike). Logic was used to determine the final location of the HES event using the information obtained from the pressure and IMU indices. The HES event continued to be expressed until the vertical accelerometer and pitch gyroscope were within the empirically tested range of 0.9-1.1 g and - 5-20 dps, respectively, which defined the FOF phase. The FOF phase continued while the vertical accelerometer and pitch gyroscope were within their respective range, the proper events proceeded (i.e., HES or FOF), and/or the midfoot pressure group was above the 7% threshold (Chatzaki et al., 2021).

Finally, HER was found. Since this framework was designed to accommodate dysfunctional gait, the algorithm allows the person to transition between FOF and HER events when, for example, people lift their heel slightly during the stance phase but do not take a step forward (common in people using walkers who perform HES with their toes). The pre-swing indices found in the initial TOF calculations that have not been overwritten as another gait phase were automatically filled to be HER. Any remaining frames in the gait cycle are then evaluated for FOF; if they did not conform to the logic described above, they were labelled HER events.

**A2.3.3 Sensor Fusion** - To remove the bias in the gyroscope signal, the median value for all three axes was calculated using all of the standing activities identified through HAR, and subtracted from the signals. Then, a Madgwick filter with a zero-velocity update was applied to the raw IMU data (gyroscope bias-corrected) to obtain the linear acceleration of each foot in global space (Madgwick et al., 2011). The global linear acceleration was then integrated to obtain velocity and was drift-corrected using the stationary period within the stance phase (Kitagawa and Ogihara, 2016). The corrected velocity was then integrated to obtain the foot's position. The gain value used for this analysis was determined by minimizing the root mean squared error between the position calculated through the IF and the MoCap systems.

**A2.4 Motion Capture (MoCap) Data** - For all laboratory-based movements, video camera data were processed in Theia 3D (Theia Markerless Inc., Canada) to obtain whole-body kinematics. Gait events were calculated by importing the kinematic and kinetic data into Visual 3D (V5, HAS-Motion, Canada) and using their automatic gait detection pipeline. These events were imported into Vicon Nexus 2.12 (Vicon, UK), where the events were visually verified and adjusted as necessary. Spatial, temporal, spatiotemporal and asymmetry variables were calculated using ProCalc (Vicon, UK) and custom algorithms in Matlab 2018b (MathWorks, USA).

**A2.5 Gait Metrics Calculated by the Insole Framework (IF)** - Gait analyses in this investigation focused on relatively straight walking between turns, standing, or stair ascent/descent activities. When walking trials were longer than 30 seconds (i.e., 125- and 500-metre indoors and 500-metre outdoors), these walks were separated into 10-second segments. In cases where a period of walking was interrupted by another ambulatory activity and the walking duration was greater than 10 seconds but less than 20 seconds (or any other base ten value), 10-second segments were taken as the middle portion of the walk. For example, if a participant turned after 16 seconds of straight walking, the first and last three seconds would be discarded, and the middle 10 seconds would be analyzed as described below. Using the identified gait events, spatiotemporal metrics were calculated and grouped into four categories: **core, pace, percentage, and asymmetry.**

Core spatiotemporal metrics were metrics from which all other categories' metrics were calculated. Within the same foot, stride time was the time between HES events; stance time was the time between HES and TOF events; swing time was the time between TOF and HES events; and stride length was the arclength distance between HES events. Between feet, step time was the time between HES events of opposing feet; single support time was the time when only one foot was in the stance phase; and double support time was the time when both feet were in the stance phase (initial and terminal double support were combined). Pace metrics describe how fast the participant was moving. Cadence was calculated by multiplying the step time by 60 seconds to obtain steps per minute (steps/min). Stride velocity was the stride length divided by the stride time to obtain metres per second (m/s). Percentage metrics were temporal-based core metrics normalized to a percent of stride time as shown in equation A1:

$$Percentage = \left( \frac{Metric\ (seconds)}{Stride\ Time\ (seconds)} \right) \times 100 \qquad \text{Eq. A1}$$

Asymmetry metrics were core metrics expressed as a percent difference between sides of the body. Asymmetry was calculated by rounding the metrics to four decimal places and then following equation A2.

$$Asymmetry = \left( \frac{|Righ_{metric} - Left_{metric}|}{(Right_{metric} - Le_{metric}) \times 0.5} \right) \times 100 \qquad \text{Eq. A2}$$

**A2.5 Statistical Analysis** - When appropriate, MoCap and insole data were aligned using the HES events that occurred at the force plate. The events were manually identified for each foot, and the average offset between feet was used to update the timestamp of the MoCap system. If an HES event did not align with the rising edge of the vertical force values (e.g., an assistive device contacted the force plate first; HES happened on a force plate that they were already standing on), two HES events per foot located anywhere in the trial were manually identified and used to find the average temporal offset to update the MoCap time stamp. Because the MoCap system and the insoles were a random sample of their respective system (i.e., MoCap can have many calibrations and camera placements; insoles come in different sizes and variations in manufacturing), and because future use will only use the measurements of a single rater (Koo and Li, 2016), two-way random effects with *consistency* and *single-rater* intraclass correlations ($ICC_{2,1}$) were calculated to assess the reliability of the IF for calculating spatial, temporal, and spatiotemporal metrics (Koo & Li, 2016) for all overground walks in the laboratory. Bland-Altman Limits of Agreement (LoA) were also calculated for each gait metric to assess the agreement between the two systems. $ICC_{2,1}$ and LoA were calculated separately for the healthy, PwMS, and PwPD populations to assess any differences between populations. One-way analysis of variance tests (ANOVAs) were run to identify significant differences between populations to evaluate whether both systems could identify the same significance trends between populations. When parametric assumptions were violated (i.e., through significant Levene's tests and/or Shapiro-Wilk), Kruskal-Wallis (KW) tests were performed. To avoid type one errors, a Bonferroni correction was performed for the group-wise comparisons (i.e., 0.05/19 tests = significance at $p < 0.0026$) and post-hoc tests (i.e., 0.05/3 tests = significance at $p < 0.0167$).

**A3.0 Results** - For all ICC results, the reliability of the IF was interpreted as excellent (> 0.90), good (0.75-0.90), moderate (0.50-0.75), or poor (< 0.50) as per Koo and Li (2016).

**A3.1 Human Activity Recognition (HAR)** - The overall classification performances of each ANN are presented in Table A3, and the activity-specific performances are presented in Table A4. The confusion matrices for predictions on the test sets for all ANNs are presented in FIG. 23A-FIG.23D. These figures show the confusion matrices for the predictions made by the following artificial neural networks: FIG. 23A) General (all participants); FIG. 23B) Healthy; FIG. 23C) People with Multiple Sclerosis (PwMS); and FIG. 23D) People with Parkinson's Disease (PwPD).

**A3.2 Reliability and Agreement** - Nineteen spatiotemporal gait metrics were compared between the MoCap system and the IF. The average $ICC_{2,1}$ value for core, pace, percentage, and asymmetry metrics were 0.938 ±0.065, 0.981 ±0.007, 0.664 ±0.045, and 0.553 ±0.226, respectively, for healthy participants; 0.957 ±0.051, 0.981 ±0.021, 0.854 ±0.011, and 0.876 ±0.082, respectively, for PwMS; and 0.965 ±0.0045, 0.991 ±0.002, 0.848 ±0.031, 0.834 ±0.092, respectively for PwPD. Degrees of freedom for the healthy population are 21, 21; 17, 17 for the PwMS; and 9, 9 for the PwPD. $ICC_{2,1}$ values for each metric are presented in Table A5. LoA results were similar between populations, and overall results were considered acceptable, with temporal metrics having a bias generally within the limitations of both systems' sampling frequencies (i.e., 0.02 seconds; 50 Hz) and stride length having a bias of less than 2%. All LoA results are presented in Table A6.

**Table A3.** Classification performance for each gait activity recognition model

| Model | Loss | Accuracy | Weighted-average F1-score |
|---|---|---|---|
| General | 0.197 | 94.56% | 94.47% |
| Healthy | 0.211 | 93.70% | 93.52% |
| PwMS | 0.133 | 97.20% | 96.98% |
| PwPD | 0.123 | 95.71% | 95.63% |
| PwMS = people with multiple sclerosis; PwPD = people with Parkinson's disease | | | |

**Table A4.** Activity-specific classification performances for each artificial neural network

| Model | Performance Metric | Stand | Walk | Turn | Stair Ascent | Stair Descent |
|---|---|---|---|---|---|---|
| General | Precision | 0.955 | 0.957 | 0.904 | 0.932 | 0.904 |
| | Recall | 1.000 | 0.977 | 0.827 | 0.940 | 0.852 |
| | F1-Score | 0.977 | 0.967 | 0.864 | 0.936 | 0.877 |
| Healthy | Precision | 0.972 | 0.939 | 0.906 | 0.940 | 0.966 |
| | Recall | 0.995 | 0.976 | 0.769 | 0.952 | 0.966 |
| | F1-Score | 0.983 | 0.957 | 0.832 | 0.946 | 0.966 |
| PwMS | Precision | 1.000 | 0.983 | 0.935 | 0.571 | 1.000 |
| | Recall | 0.981 | 0.990 | 0.940 | 0.727 | 0.182 |
| | F1-Score | 0.990 | 0.987 | 0.938 | 0.640 | 0.308 |
| PwPD | Precision | 0.938 | 0.963 | 0.937 | 1.000 | 0.900 |
| | Recall | 1.000 | 0.980 | 0.840 | 1.000 | 1.000 |
| | F1-Score | 0.968 | 0.971 | 0.886 | 1.000 | 0.947 |
| PwMS = people with multiple sclerosis; PwPD = people with Parkinson's disease | | | | | | |

**Table A5.** ICC$_{2,1}$ results comparing the motion capture system and Insole Framework

| Categ ory | Metric | Populati on | ICC$_{2,1}$ [95% CI] | F-value | Interpretation |
|---|---|---|---|---|---|
| Core | Stride Time (s) | Healthy | 0.998 [1.00, 1.00] | 1111 | Excellent |
| | | PwMS | 1.000 [1.00, 1.00] | 8615 | Excellent |
| | | PwPD | 0.998 [0.99, 1.00] | 1229 | Excellent |
| | Stance Time (s) | Healthy | 0.974 [0.94, 0.99] | 74.8 5 | Excellent |
| | | PwMS | 0.985 [0.96, 1.00] | 298. 6 | Excellent |
| | | PwPD | 0.983 [0.94, 1.00] | 121. 3 | Excellent |
| | Swing Time (s) | Healthy | 0.884 [0.75, 0.95] | 20.5 2 | Good |
| | | PwMS | 0.894 [0.75, 0.96] | 22.0 8 | Good |
| | | PwPD | 0.954 [0.83 ,0.99] | 62.4 2 | Excellent |
| | Single Support Time (s) | Healthy | 0.929 [0.84, 0.97] | 27.2 6 | Excellent |
| | | PwMS | 0.882 [0.72, 0.96] | 18.5 3 | Good |
| | | PwPD | 0.960 [0.85 ,0.99] | 58.5 0 | Excellent |
| | Double Support Time (s) | Healthy | 0.824 [0.62, 0.93] | 10.3 8 | Good |
| | | PwMS | 0.948 [0.87, 0.98] | 37.5 1 | Excellent |
| | | PwPD | 0.870 [0.57 ,0.97] | 14.3 7 | Good |
| | Stride Length (m) | Healthy | 0.965 [0.95, 0.99] | 89.4 9 | Excellent |
| | | PwMS | 0.993 [0.98, 1.00] | 307. 3 | Excellent |
| | | PwPD | 0.991 [0.97, 1.00] | 348. 3 | Excellent |
| | Step Time (s) | Healthy | 0.997 [0.99, 1.00] | 586. 6 | Excellent |
| | | PwMS | 1.000 [1.00, 1.00] | 6204 | Excellent |
| | | PwPD | 0.997 [0.99, 1.00] | 757. 5 | Excellent |
| Pace | Cadence (steps/min) | Healthy | 1.000 [0.99, 1.00] | 494. 1 | Excellent |
| | | PwMS | 0.966 [0.91, 0.99] | 58.2 6 | Excellent |
| | | PwPD | 0.990 [0.96, 1.00] | 193. 7 | Excellent |
| | Stride Velocity (m/s) | Healthy | 0.976 [0.94, 0.99] | 95.7 3 | Excellent |
| | | PwMS | 0.996 [0.99, 1.00] | 506. 8 | Excellent |
| | | PwPD | 0.993 [0.97, 1.00] | 321. 2 | Excellent |
| Perce ntages | Stance Percent (%) | Healthy | 0.669 [0.39, 0.85] | 5.37 0 | Moderate |
| | | PwMS | 0.852 [0.67, 0.94] | 20.9 5 | Good |
| | | PwPD | 0.872 [0.58, 0.97] | 15.0 4 | Good |
| | Swing Percent (%) | Healthy | 0.669 [0.39, 0.85] | 5.37 0 | Moderate |
| | | PwMS | 0.852 [0.67, 0.94] | 20.9 5 | Good |
| | | PwPD | 0.872 [0.58 ,0.97] | 15.0 4 | Good |
| | Single Support Percent (%) | Healthy | 0.766 [0.52, 0.90] | 7.75 4 | Good |
| | | PwMS | 0.868 [0.70, 0.95] | 23.7 2 | Good |
| | | PwPD | 0.839 [0.49, 0.96] | 12.3 8 | Good |
| | Double Support Percent (%) | Healthy | 0.720 [0.44, 0.87] | 6.19 3 | Moderate |
| | | PwMS | 0.841 [0.63, 0.94] | 11.6 6 | Good |
| | | PwPD | 0.807 [0.40, 0.95] | 9.38 7 | Good |

(continued)

| Categ ory | Metric | Populati on | ICC$_{2,1}$ [95% CI] | F-value | Interpretation |
|---|---|---|---|---|---|
| Asym metry | Stride Time Asymmetry (%) | Healthy | 0.838 [0.65, 0.93] | 11.3 4 | Good |
| | | PwMS | 0.960 [0.90, 0.98] | 48.4 2 | Excellent |
| | | PwPD | 0.700 [0.17, 0.92] | 5.67 4 | Moderate |
| | Stance Time Asymmetry (%) | Healthy | 0.702 [0.41, 0.86] | 5.71 0 | Moderate |
| | | PwMS | 0.866 [0.98, 0.95] | 13.8 8 | Good |
| | | PwPD | 0.870 [0.56, 0.97] | 14.4 4 | Good |
| | Swing Time Asymmetry (%) | Healthy | 0.506 [0.12, 0.76] | 3.04 8 | Moderate |
| | | PwMS | 0.864 [0.67, 0.95] | 13.7 5 | Good |
| | | PwPD | 0.973 [0.89, 0.99] | 72.2 0 | Excellent |
| | Stride Length Asymmetry (%) | Healthy | 0.616 [0.27, 0.82] | 4.20 5 | Moderate |
| | | PwMS | 0.731 [0.41, 0.89] | 6.43 5 | Moderate |
| | | PwPD | 0.841 [0.48, 0.96] | 11.5 7 | Good |
| | Single Support Time Asymmetry (%) | Healthy | 0.177 [-0.25, 0.55] | 1.43 2 | Poor |
| | | PwMS | 0.893 [0.74, 0.96] | 17.7 0 | Good |
| | | PwPD | 0.847 [0.50, 0.96] | 12.1 0 | Good |
| | Double Support Time Asymmetry (%) | Healthy | 0.479 [0.08, 0.75] | 2.84 2 | Poor |
| | | PwMS | 0.945 [0.86, 0.98] | 35.4 6 | Excellent |
| | | PwPD | 0.773 [0.32, 0.94] | 7.80 7 | Good |

In the table above: PwMS = people with multiple sclerosis, PwPD = people with Parkinson's disease, s = seconds, m = metres, min = minutes, m/s = metres/second, and % = percentage. Percentage variables are the percent of stride time, and asymmetry variables are the percent difference between sides **(Eq A2).**

**Table A6.** Bland Altman Limits of Agreement Results comparing the motion capture system and Insole Framework

| Category | Metric | Populati on | Mean | Bias (SD) | Limits of Agreement [Upper, Lower] |
|---|---|---|---|---|---|
| Core | Stride Time (s) | Healthy | 1.151 | -0.016 (0.019) | [0.021, -0.053] |
| | | PwMS | 1.375 | -0.019 (0.027) | [0.034, -0.072] |
| | | PwPD | 1.257 | -0.015 (0.028) | [0.040, -0.069] |

(continued)

| Category | Metric | Population | Mean | Bias (SD) | Limits of Agreement [Upper, Lower] |
|---|---|---|---|---|---|
| | Stance Time (s) | Healthy | 0.725 | 0.004 (0.021) | [0.045, -0.038] |
| | | PwMS | 0.918 | -0.017 (0.045) | [0.072, -0.106] |
| | | PwPD | 0.800 | -0.010 (0.026) | [0.040, -0.061] |
| | Swing Time (s) | Healthy | 0.427 | -0.020 (0.026) | [0.031, -0.071] |
| | | PwMS | 0.457 | -0.002 (0.045) | [0.087, -0.090] |
| | | PwPD | 0.458 | -0.004 (0.034) | [0.063, -0.071] |
| | Single Support Time (s) | Healthy | 0.439 | -0.005 (0.020) | [0.033, -0.044] |
| | | PwMS | 0.474 | 0.019 (0.044) | [0.105, -0.068] |
| | | PwPD | 0.478 | 0.009 (0.025) | [0.059, -0.041] |
| | Double Support Time (s) | Healthy | 0.285 | 0.009 (0.032) | [0.073, -0.055] |
| | | PwMS | 0.444 | -0.036 (0.071) | [0.2204, - 0.176] |
| | | PwPD | 0.322 | -0.019 (0.041) | [0.061, -0.099] |
| | Stride Length (m) | Healthy | 1.294 | 0.021 (0.073) | [0.165, -0.123] |
| | | PwMS | 0.961 | 0.018 (0.081) | [0.177, -0.142] |
| | | PwPD | 1.140 | 0.019 (0.061) | [0.140, -0.101] |
| | Step Time (s) | Healthy | 0.585 | 0.003 (0.038) | [0.078, -0.072] |
| | | PwMS | 0.693 | 0.001 (0.045) | [0.090, -0.088] |
| | | PwPD | 0.635 | 0.003 (0.034) | [0.069, -0.063] |
| Pace | Cadence (steps/- min) | Healthy | 103.5 | 0.010 (6.860) | [13.46, -13.44] |
| | | PwMS | 90.91 | -0.714 (11.97) | [22.74, -24.16] |
| | | PwPD | 95.33 | 0.486 (6.333) | [12.90, -11.93] |
| | Stride Velocity (m/s) | Healthy | 1.132 | 0.035 (0.067) | [0.166, -0.096] |
| | | PwMS | 0.738 | 0.026 (0.066) | [0.155, -0.103] |
| | | PwPD | 0.915 | 0.029 (0.053) | [0.132, -0.075] |
| Percentages | Stance Percent (%) | Healthy | 62.94 | 1.243 (1.943) | [5.051, -2.564] |
| | | PwMS | 66.35 | -0.177 (2.998) | [5.760, -5.993] |
| | | PwPD | 63.61 | -0.098 (2.273) | [4.357, -4.553] |
| | Swing Percent (%) | Healthy | 37.06 | -1.243 (1.943) | [2.564, -5.051] |
| | | PwMS | 33.65 | 0.177 (2.998) | [5.993, -5.760] |
| | | PwPD | 36.39 | 0.098 (2.273) | [4.553, -4.357] |
| | Single Support Percent (%) | Healthy | 38.18 | 0.051 (1.780) | [3.540, -3.437] |
| | | PwMS | 35.01 | 1.628 (2.997) | [7.502, -4.247] |
| | | PwPD | 38.00 | 1.147 (2.116) | [5.295, -3.000] |
| | Double Support Percent (%) | Healthy | 24.76 | 1.192 (2.840) | [6.759, -4.375] |
| | | PwMS | 31.34 | -1.744 (4.665) | [7.399, -10.89] |
| | | PwPD | 25.61 | -1.245 (3.255) | [5.134, -7.624] |

(continued)

| Category | Metric | Population | Mean | Bias (SD) | Limits of Agreement [Upper, Lower] |
|---|---|---|---|---|---|
| Asymmetry | Stride Time Asymmetry (%) | Healthy | 1.521 | 0.067 (0.903) | [1.836, -1.703] |
| | | PwMS | 2.350 | 0.059 (0.857) | [1.739, -1.621] |
| | | PwPD | 1.749 | 0.211 (0.970) | [2.113, -1.691] |
| | Stance Time Asymmetry (%) | Healthy | 2.594 | -0.132 (1.723) | [3.245, -3.510] |
| | | PwMS | 7.739 | 2.056 (3.737) | [9.381, -5.270] |
| | | PwPD | 3.582 | 0.320 (2.042) | [4.322, -3.681] |
| | Swing Time Asymmetry (%) | Healthy | 4.029 | 0.330 (3.599) | [7.384, -6.723] |
| | | PwMS | 14.76 | 3.307 (6.918) | [16.87, -10.25] |
| | | PwPD | 8.146 | 0.175 (3.636) | [7.302, -6.952] |
| | Stride Length Asymmetry (%) | Healthy | 13.49 | 0.318 (4.549) | [9.235, -8.599] |
| | | PwMS | 9.924 | 1.419 (6.190) | [13.55, -10.71] |
| | | PwPD | 14.08 | 1.676 (4.196) | [9.899, -6.548] |
| | Single Support Asymmetry (%) | Healthy | 3.031 | 0.293 (2.525) | [5.282, -4.695] |
| | | PwMS | 13.16 | 3.398 (5.929) | [15.02, -8.223] |
| | | PwPD | 5.727 | 1.991 (3.120) | [8.107, -4.125] |
| | Double Support Asymmetry (%) | Healthy | 4.991 | -0.445 (3.723) | [6.852, -7.743] |
| | | PwMS | 5.877 | -0.256 (2.457) | [4.560, -5.072] |
| | | PwPD | 4.481 | -1.333 (3.174) | [4.888, -7.554] |

**A3.3 Significance Testing** - Nineteen spatiotemporal metrics were independently assessed for significant differences between populations ($p < 0.0026$) using the IF and the MoCap system. All but one metric (i.e., stride length asymmetry) had the same significance interpretation between systems. For those spatiotemporal metrics that had significant differences between populations (N = 10), the systems disagreed on three metrics during post-hoc testing where the MoCap system found no difference between PwMS and PwPD during stance, swing, and double support percent, but the IF did ($p < 0.0167$). Significance testing results are presented in **Table A7.**

**Table A7.** Statistical Comparisons Between Populations.

| Category | Metric | Technology | p-value | Critical value | Eta | Healthy vs PwMS | Healthy vs PwPD | PwMS vs PwPD | Test |
|---|---|---|---|---|---|---|---|---|---|
| Core | Stride Time (s) | Insole | 0.003 | 11.46 | 0.197 2 | | | | KW |
| | | MoCap | 0.003 | 11.61 | 0.200 1 | | | | KW |
| | Stance Time (s) | Insole | 0.002 | 12.02 | 0.208 7 | 0.002 | 0.179 | 1.000 | KW |
| | | MoCap | **0.001** | 13.04 | 0.230 0 | **0.002** | 0.058 | 1.000 | KW |
| | Swing Time (s) | Insole | 0.042 | 3.397 | 0.126 3 | | | | ANO VA |
| | | MoCap | 0.175 | 1.809 | 0.071 5 | | | | ANO VA |
| | Single Support Time (s) | Insole | 0.012 | 4.864 | 0.171 5 | | | | ANO VA |
| | | MoCap | 0.062 | 2.953 | 0.111 6 | | | | ANO VA |
| | Double Support Time (s) | Insole | **0.002** | 12.75 | 0.223 9 | **0.001** | 0.994 | 0.164 | KW |
| | | MoCap | **0.001** | 15.00 | 0.270 9 | **0.000** | 0.107 | 0.904 | KW |
| | Stride Length (m) | Insole | **0.001** | 8.286 | 0.260 7 | **0.001** | 0.227 | 0.230 | ANO VA |
| | | MoCap | **0.000** | 9.393 | 0.285 6 | **0.000** | 0.175 | 0.205 | ANO VA |
| | Step Time (s) | Insole | 0.006 | 10.35 | 0.173 9 | | | | KW |
| | | MoCap | 0.003 | 11.42 | 0.196 2 | | | | KW |
| Pace | Cadence (steps/m in) | Insole | 0.011 | 8.935 | 0.144 5 | | | | KW |
| | | MoCap | 0.004 | 6.252 | 0.210 1 | | | | ANO VA |
| | Stride Velocity (m/s) | Insole | **0.000** | 17.53 | 0.323 6 | **0.000** | **0.008** | 1.000 | KW |
| | | MoCap | **0.000** | 17.50 | 0.322 8 | **0.000** | **0.014** | 1.000 | KW |
| Percentages | Stance Percent (%) | Insole | **0.000** | 10.57 | 0.310 3 | **0.000** | 1.000 | **0.004** | ANO VA |
| | | MoCap | **0.000** | 15.61 | 0.283 5 | **0.000** | 0.181 | 0.532 | KW |
| | Swing Percent (%) | Insole | **0.000** | 10.57 | 0.310 3 | **0.000** | 1.000 | **0.004** | ANO VA |
| | | MoCap | **0.000** | 15.61 | 0.283 5 | **0.000** | 0.181 | 0.532 | KW |
| | Single Support Percent (%) | Insole | 0.007 | 9.907 | 0.164 7 | | | | KW |
| | | MoCap | 0.003 | 11.84 | 0.205 0 | | | | KW |
| | Double Support Percent (%) | Insole | **0.000** | 9.009 | 0.277 1 | **0.001** | 0.976 | **0.005** | ANO VA |
| | | MoCap | **0.001** | 13.88 | 0.247 4 | **0.001** | 0.316 | 0.458 | KW |
| Asymmetry | Stride Time Asymme try (%) | Insole | 0.035 | 6.681 | 0.097 5 | | | | KW |
| | | MoCap | 0.073 | 5.230 | 0.067 3 | | | | KW |
| | Stance Time Asymme try (%) | Insole | **0.000** | 19.76 | 0.370 1 | **0.000** | 0.261 | 0.162 | KW |
| | | MoCap | **0.002** | 12.12 | 0.210 7 | **0.002** | 0.784 | 0.259 | KW |

(continued)

| Categ ory | Metric | Techno logy | p-value | Critical value | Eta | Health y vs PwMS | Healthy vs PwPD | PwM S vs PwPD | Test |
|---|---|---|---|---|---|---|---|---|---|
| | Swing Time Asymme try (%) | Insole | **0.000** | 27.20 | 0.525 1 | **0.000** | **0.006** | 0.830 | KW |
| | | MoCap | **0.000** | 29.06 | 0.563 8 | **0.000** | **0.002** | 1.000 | KW |
| | Stride Length Asymme try (%) | Insole | 0.004 | 6.087 | 0.206 | | | | ANO VA |
| | | MoCap | **0.001** | 7.795 | 0.249 1 | **0.001** | 0.957 | 0.022 | ANO VA |
| | Single Support Time Asymme try (%) | Insole | **0.000** | 26.67 | 0.514 | **0.000** | 0.066 | 0.159 | KW |
| | | MoCap | **0.000** | 19.78 | 0.370 | **0.000** | 0.286 | 0.146 | KW |
| | Double Support Time Asymme try (%) | Insole | 0.363 | 2.029 | 0.000 6 | | | | KW |
| | | MoCap | 0.739 | 0.606 | - 0.029 0 | | | | KW |

Note: A Bonferroni correction was performed for the group-wise comparisons (i.e., 0.05/19 tests = significance at $p < 0.0026$) and post-hoc tests (i.e., 0.05/3 tests = significance at $p < 0.0167$). Bolded values denote statistical significance. PwMS = people with multiple sclerosis, PwPD = people with Parkinson's disease, KW = Kruskal-Wallice, ANOVA = one-way analysis of variance, s = seconds, m = metres, min = minutes, m/s = metres/second, and % = percentage. Percentage variables are the percent of stride time, and asymmetry variables are the percent difference between sides **(Eq. A2).**

**A4.0 - DISCUSSION** - Evaluating walking quality is a key method for understanding disease progression in people who have neurological disorders (Vienne-Jumeau et al., 2020). However, the current standard of care focuses on gross walking ability rather than spatiotemporal gait metrics, mainly due to the barriers to accessing the technology needed and the frequency with which these metrics need to be collected to be valuable for longitudinal evaluations and proactive decision-making. Therefore, we propose a method that uses instrumented shoe insoles to collect the data needed to unobtrusively monitor the gait quality of individuals outside of a laboratory environment. The current paper validates an IF that uses a multi-layered approach to automatically identify ambulatory activities (i.e., walking, standing, turning, stair ascent, and stair descent), perform gait detection, and calculate reliable spatiotemporal gait metrics on standardized 10-second segments of walking.

Our HAR models showed strong performance (i.e., the General model had an accuracy of 94.56% and weighted-averaged F1-score of 94.47%), which is comparable to previous literature using instrumented shoe insoles to detect similar types and numbers of activities. However, the HAR models developed with data solely from individuals with neurological dysfunction (i.e., MS and PD) led to improved classification performances (accuracies ranged from 95.71 - 97.20%) compared to the General model. This was likely because the variability in gait patterns within a population was equal to or lesser than the General model, improving ANN's ability to correctly relate the raw insole data to gait activities. These findings should motivate the use of population-specific modelling for HAR. Consistent with previous literature (Moufawad el Achkar et al., 2016), our algorithms generally had a lower precision for stair ascent and descent compared to other activities, especially in the PwMS-specific model, which often incorrectly predicted stair ascent when the true label was stair descent. It is possible that similarities in the kinematics and kinetics of the first and last stair tread of stair ascent versus descent may have contributed to the poorer performance in these activities. While classification performances for the stair activities were stronger in the other models, the larger variance in gait parameters amongst PwMS compared to healthy controls (Comber et al., 2017) may have posed a greater challenge to recognizing their stair activities; more training examples are warranted. The results of the present work suggest that classification of ambulatory activities for PwMS (and potentially other disorders affecting gait) may be further strengthened by developing HAR models specific to gait phenotypes (e.g., ataxic, spastic, hemiplegic, etc.).

Compared to the gold-standard MoCap system, the IF was able to calculate all core and pace spatiotemporal gait metrics with good to excellent reliability ($\geq 0.824$) regardless of neurological status. All spatiotemporal metrics had moderate to excellent reliability for PwMS ($\geq 0.731$) and PwPD ($\geq 0.700$), while healthy participants had moderate to good reliability for percent metrics (0.669-0.766) and poor to good reliability for asymmetry metrics (0.177-0.838). On average, across all metrics, PwMS had the highest $ICC_{2,1}$ values (0.912), followed by PwPD (0.902); healthy participants had the lowest average $ICC_{2,1}$ values (0.773). LoA results indicate that bias values for core temporal metrics across all populations are within the limitations of the sampling frequencies (i.e., 0.02 seconds) except for double support time in PwMS (0.036 seconds). On average, PwMS had the highest bias values, followed by PwPD, and healthy participants had the lowest biases.

Significance testing using one-way ANOVAs and Kruskal-Wallice tests indicated that similar statistical findings would be discovered if analyzing the same population using either the IF or the MoCap system. Of the 19 spatiotemporal metrics assessed, the systems disagreed on one population-level test (i.e., stride length asymmetry) and three post-hoc comparisons (i.e., PwMS vs. PwPD for stance, swing, and double support percent). Further, across all tests, $\eta^2$ values - which represent the proportion of variance explained by group differences - were relatively consistent between technologies; the average absolute $\eta^2$ difference across all tests was 0.046. This consistency suggests that, regardless of the system used, a similar amount of variance was explained for the captured group-based differences.

Overall, the presented temporal results are comparable to previous research that reported ICC (Braun et al., 2015; Kluge et al., 2017; Schwesig et al., 2011; Washabaugh et al., 2017) and LoA values (Ganguly et al., 2023; Jagos et al., 2017; Kluge et al., 2017; Salis et al., 2023) when comparing instrumented insoles to a gold-standard system. Stride length results in the current study are similar to what has been previously reported with foot-mounted IMUs (Kitagawa and Ogihara, 2016; Kluge et al., 2017; Washabaugh et al., 2017) and instrumented shoe insoles (Ganguly et al., 2023; Salis et al., 2023), reaffirming that using a fusion algorithm (i.e., Madgwick et al. 2011, in the current study) with a zero-velocity update is appropriate to use in healthy and dysfunctional gait. Percentage and asymmetry gait metrics had the lowest reliability in the healthy population, which is similar to findings from a review by Kobsar et al. (2020), who found that throughout the literature, asymmetry and variability metrics have poor reliability in healthy populations. For PwMS and PwPD, moderate to excellent reliability was found for asymmetry metrics; the stronger reliability is likely due to the increased variability in gait quality amongst the PwMS and PwPD populations, favouring ICC calculations compared to the less variable healthy population.

## SECTION B - RELIABILITY

This section is based on a study that examines the reliability of 63 spatiotemporal gait metrics across five consecutive days of six-minute treadmill walking, and weekly bouts of six-minute treadmill walking over six months in healthy controls obtained through the improved IF described above in Section A. Because people with multiple sclerosis (MS) and people with Parkinson's disease (PD) have highly variable gait, the goal of this work was to use the IF to assess spatiotemporal gait metric reliability in a healthy population to identify which metrics are reliable and can be potentially used to monitor disease progression. Between-day reliability was assessed under two conditions: 1) across five consecutive days of treadmill walking (Short-term), and 2) during weekly treadmill walking over the course of 6 months (Long-term); ground-truth data were collected from fully instrumented treadmill (FIT) force plates to confirm metric reliability.

**B2.0 Methods - B2.1 Participants** - Five healthy participants (3M, 2F), mean (SD) age of 25.9 (3.4) years, height of 173.3 (10.3) cm, and weight of 74.0 (14.8) kg were recruited to perform a treadmill walk for five consecutive days, and weekly treadmill walks for six months. All aspects of this study were approved by the University of Ottawa research ethics board (H-11-21-7565).

**B2.2 Movement Protocol and Instrumentation - B2.2.1 Short-term Protocol** - Participants arrived at the University of Ottawa's Movement Biomechanics and Analytics Laboratory for five consecutive days at approximately the same time each day. On day one, participants provided informed consent and were fitted with a pair of instrumented shoe insoles (50 Hz; ReGo, Moticon, Germany); the same pair were worn each visit, and depending on size, multiple people used the same insole. The insole sizes ranged from S3 to S7 (EU shoe size 36-45; US shoe size Women 5.5-13, Men 4.5-11.5). Following sizing, participants were asked to perform a four-minute familiarization session on a dual-belt force plate FIT (Bertec, USA). During this familiarization session, participants were asked to identify their preferred walking speed by following the methods outlined by Dingwell & Marin (2006) (i.e., iteratively speeding and slowing the treadmill to find the average preferred speed). Once their preferred walking speed was identified, participants were given a rest (2-5 minutes) and asked to perform a two-minute warmup, immediately followed by a six-minute walking trial at their preferred speed; the warmup and walking trial were performed at each subsequent visit. The average preferred walking speed across all participants was 1.3 $\pm$0.1 m/s.

**B2.2.2 Long-term Protocol** - Following the Short-term protocol, the same participants attended the University of Ottawa's Movement Biomechanics and Analytics Laboratory approximately once per week for six months. The average time between visits was 7.69 days. Participants wore the same pair of insoles and walked at the same speed as determined in the Short-term protocol (section B2.2.1). During each walking session, participants performed a two-minute warmup, immediately followed by a six-minute walking trial at their preferred walking speed.

**B2.3 Data Analysis** - Gait event detection in the IF is explained in detail above in Example A. Using the identified gait events, spatiotemporal metrics were calculated and grouped into six categories: core, variability, pace, percentage, asymmetry, and IMU (Table B1).

To obtain ground truth data, foot on and off events from the FIT data were identified using a threshold set at 10% of the maximum vertical ground reaction force recorded in each trial. Once the first and last foot off events were identified, the trial was split into 10-second segments to align with the analysis of the IF. Data from the FIT and the IF were aligned using the first toe-off event identified by both systems. The first and last 20 seconds of walking were discarded to avoid non-steady-state walking. Using the identified gait events, spatiotemporal metrics were calculated (Table B1).

Table B1. Summary of spatiotemporal metric calculations

| Category | Metric | Description/Calculation |
|---|---|---|
| Core | Stride Time (s) | Time between heel strike events (same foot) |
| | Stance Time (s) | Time between heel strike and toe off events (same foot) |
| | Swing Time (s) | Time between toe off and heel strike events (same foot) |
| | Single Support Time (s) | Time that only one foot is on the ground (same foot) |
| | Double Support Time (s) | Time where two feet are on the ground |
| | Stride Length (m) | Arclength distance between heel strike events (same foot) |
| | Step Time (s) | Time between heel strike events of opposing feet |
| Variability | SD (all core, pace, and percentage metrics; 13 total) | Standard deviation of each metric calculated in the "core," 'pace,' and 'percentage' categories |
| | CV (all core, pace, and percentage metrics; 13 total) | Coefficient of variation of each metric calculated in the 'core,' "pace," and 'percentage' categories |
| Pace | Cadence (steps/min) | Step time $\times$ 60 s |
| | Stride Velocity (m/s) | Stride length $\div$ stride time |
| | Distance in 10 s (m) | Arclength distance between the first and last heel strike in a 10-second segment |
| Percentage | Stance Percent (%) | $\left(\dfrac{Metric\ time}{Stride\ Time}\right) \times 100$ |
| | Swing Percent (%) | |
| | Single Support Percent (%) | |
| | Double Support Percent (%) | |
| Asymmetry | Stride Time Asymmetry (%) | $\left(\dfrac{|Metric_{right} - Metric_{left}|}{(Metric_{right} - Metric_{left}) \times 0.5}\right) \times 100$ |
| | Stance Time Asymmetry (%) | |
| | Swing Time Asymmetry (%) | |
| | Stride Length Asymmetry (%) | |
| | Stride Velocity Asymmetry (%) | |
| | Single Support Time Asymmetry (%) | |
| | Double Support Time Asymmetry (%) | |
| | Stance Percent Asymmetry (%) | |
| | Swing Percent Asymmetry (%) | |
| | Single Support Percent Asymmetry (%) | |
| | Double Supprt Percent Asymmetry (%) | |

(continued)

| Categor y | Metric | Description/Calculation |
|---|---|---|
| **IMU** | $Acc_{norm,meanSD}$ | $$\sum_{i=1}^{N}\left(\sum_{j=1}^{101} SD\left(\sqrt{acc_x^2 + acc_y^2 + acc_z^2}\right)_j \Big/ 101\right)_i \Big/ N$$ |
| | $Acc_{norm,peak}$ | $$\sum_{i=1}^{N}\left(\max\left(\sqrt{acc_x^2 + acc_y^2 + acc_z^2}\right)\right)_i \Big/ N$$ |
| | $Acc_{norm,peakSD}$ | $$SD\left(\max\left(\sqrt{acc_x^2 + acc_y^2 + acc_z^2}\right)\right)_i$$ |
| | $Acc_{norm,range}$ | $$\sum_{i=1}^{N}\left(\left\|\max\left(\sqrt{acc_x^2 + acc_y^2 + acc_z^2}\right) - \min\left(\sqrt{acc_x^2 + acc_y^2 + acc_z^2}\right)\right\|\right)_i \Big/ N$$ |
| | $Acc_{norm,rangeSD}$ | $$SD\left(\left\|\max\left(\sqrt{acc_x^2 + acc_y^2 + acc_z^2}\right) - \min\left(\sqrt{acc_x^2 + acc_y^2 + acc_z^2}\right)\right\|\right)_i$$ |
| | $Acc_{norm,rmse}$ | $$\sum_{i=1}^{N} RMSE\left(\sum_{j=1}^{101}\left(\sum_{i=1}^{N}\left(\sqrt{acc_x^2 + acc_y^2 + acc_z^2}\right)_i \Big/ N\right)_j \Big/ 101\right)_i \Big/ N$$ |
| | $Gyro_{norm,meanSD}$ | $$\sum_{i=1}^{N}\left(\sum_{j=1}^{101} SD\left(\sqrt{gyr_x^2 + gyr_y^2 gyr}\right)_j \Big/ 101\right)_i \Big/ N$$ |
| | $Gyro_{norm,peak}$ | $$\sum_{i=1}^{N}\left(\max\left(\sqrt{gyr_x^2 + gyr_y^2 + gyr_z^2}\right)\right)_i \Big/ N$$ |
| | $Gyro_{norm,peakSD}$ | $$SD\left(\max\left(\sqrt{gyr_x^2 + gyr_y^2 + gyr_z^2}\right)\right)_i$$ |
| | $Gyro_{norm,range}$ | $$\sum_{i=1}^{N}\left(\left\|\max\left(\sqrt{gyr_x^2 + gyr_y^2 + gyr_z^2}\right) - \min\left(\sqrt{gyr_x^2 + gyr_y^2 + gyr_z^2}\right)\right\|\right)_i \Big/ N$$ |
| | $Gyro_{norm,rangeSD}$ | $$SD\left(\left\|\max\left(\sqrt{gyr_x^2 + gyr_y^2 + acc_z^2}\right) - \min\left(\sqrt{gyr_x^2 + gyr_y^2 + gyr_z^2}\right)\right\|\right)_i$$ |
| | $Gyro_{norm,rmse}$ | $$\sum_{i=1}^{N} RMSE\left(\sum_{j=1}^{101}\left(\sum_{i=1}^{N}\left(\sqrt{gyr_x^2 + gyr_y^2 + gyr_z^2}\right)_i \Big/ N\right)\right)$$ |

wherein i = gait cycle number within 10-second segment; N = total number of gait cycles within 10-second segment; j = percentage across 1 gait cycle, acc = accelerometer; gyro/gyr = gyroscope; SD = standard deviation; CV = coefficient of variation; norm = Euclidean norm.

**B2.4 - Statistical Analysis** - For both systems, spatiotemporal metrics were calculated for each 10-second segment and were averaged when appropriate to produce one value per metric per segment. All segments were then averaged to produce one value per metric per trial. All five days of the Short-term protocol were analyzed. For the Long-term protocol, the metrics from the Short-term protocol were averaged to represent the first week of the Long-term protocol. For both protocols and systems, an $ICC_{3,1}$ with absolute agreement was used, treating the metrics as the ratings, trial numbers as the raters, and participants as the targets. $ICC_{3,1}$ with absolute agreement was chosen since this is a test-retest approach; therefore, the raters cannot be considered random and should have absolute agreement between walking sessions (Koo & Li, 2016). Further, a mixed-effects model was used to evaluate the relationship between the metrics and the number of elapsed days since the participant's first walking session, with the participant identifiers included as a random effect. To assess if adding a quadratic term improved the fit of the mixed-effects model, a Likelihood Ratio Test (LRT) was performed by comparing the log-likelihoods of the linear and quadratic models and evaluating the result using a chi-squared distribution. If the LRT was significant or the residual variance in the model was reduced by >5%, a quadratic mixed-effects model was used rather than a linear mixed-effects model. From the mixed models, a series of statistical measures were obtained: relative slope (i.e., rate of change over elapsed days expressed as a percentage of the mean), RC to mean ratio (i.e., the 95% range of expected values within a participant expressed as a percentage of the mean), and residual variability (i.e., within-subject variability expressed as a percentage of the mean). A metric was deemed reliable if, for *both* systems, during *both* the Short- and Long-term protocol $ICC_{3,1}$ was greater than 0.69. Else, during *both* the Short- and Long-term protocol, the metric had to have a relative slope of less than 10%, AND an RC to mean ratio of less than 30%, AND a residual variability of less than 20%. These thresholds were chosen to balance the relative stability and precision of the metrics when collected over short and long periods of time.

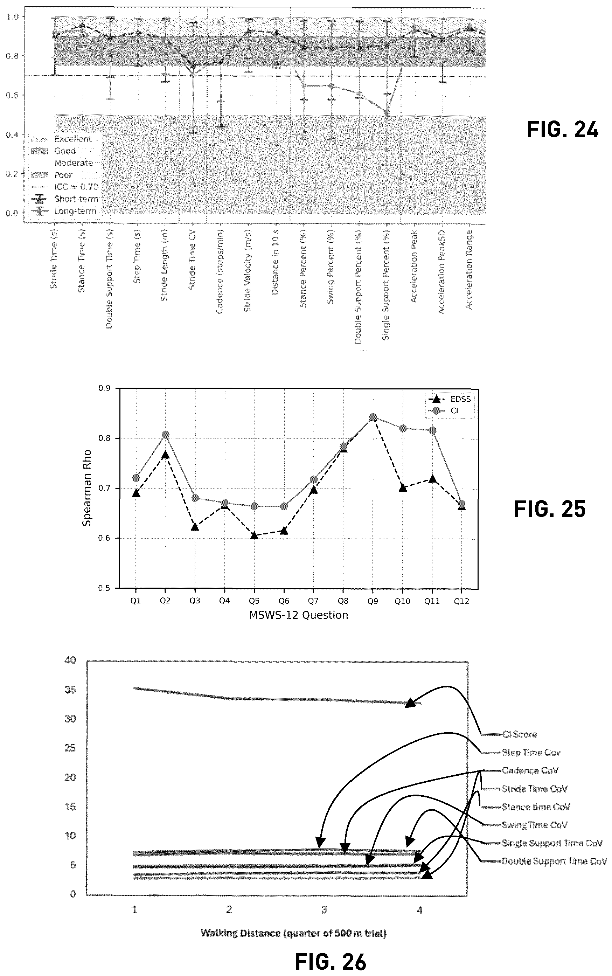

**B3.0 RESULTS** - For all ICC results, the reliability of the metrics was interpreted as excellent (> 0.90), good (0.75-0.90), moderate (0.50-0.75), or poor (< 0.50) as per Koo and Li (2016). Out of the 63 calculated gait metrics, 27 were identified as reliable in a healthy population during Short-term (i.e., five consecutive days) and Long-term (i.e., over six months) evaluations of gait quality using the IF. ICC values for the reliable metrics are depicted in FIG. 24, and the relative slope, RC ratio to the mean, and residual variability scores for the metrics identified are presented in Table B2.

FIG. 24 illustrates the Intraclass Correlation 3,1 Absolute Agreement Results. Error bars represent the upper and lower bounds for the 95% confidence intervals. Excellent (> 0.90), good (0.75-0.90), moderate (0.50-0.75), poor (< 0.50). Metrics that had an ICC > 0.69 were automatically deemed reliable (i.e., above horizontal dash dot line); those with lower ICC values were evaluated for temporal stability and precision. Dotted vertical lines signify the groupings of metrics: core, variability, pace, percentage, and IMU.

**Table B2.** Between-day reliability metrics for Short- and Long-term evaluations of gait quality

| Group | Metric | Relative slope | | RC ratio to the mean | | Residual variability | |
|---|---|---|---|---|---|---|---|
| | | *Short-term* | *Long-term* | *Short-term* | *Long-term* | *Short-term* | *Long-term* |
| *Core* | Stride time | -4.97 | 0.91 | 3.15 | 3.26 | 1.13 | 1.18 |
| | Stance time | -6.06 | 0.67 | 2.66 | 3.74 | 0.96 | 1.35 |
| | Swing time | -17.15 | -0.24 | 10.30 | 11.44 | 3.72 | 4.13 |
| | Single support time | 1.80 | 4.79 | 6.45 | 9.23 | 2.33 | 3.33 |
| | Double support time | 0.49 | 1.10 | 3.12 | 3.39 | 1.13 | 1.22 |
| | Stride length | -0.38 | 1.36 | 16.93 | 24.83 | 6.11 | 8.96 |
| | Step time | -0.19 | -0.45 | 5.02 | 5.56 | 1.81 | 2.01 |
| *Variab ility* | Stance time SD | 1.31 | 3.83 | 5.47 | 8.61 | 1.97 | 3.10 |
| | Stride time CV | 1.73 | 3.43 | 5.80 | 8.15 | 2.09 | 2.94 |
| | Stride time SD | -0.36 | -0.26 | 1.77 | 1.91 | 0.64 | 0.69 |
| *Pace* | Cadenc e | 0.64 | 0.45 | 3.08 | 3.32 | 1.11 | 1.20 |
| | Stride velocit y | 0.53 | 0.24 | 2.96 | 4.41 | 1.07 | 1.59 |
| | Distanc e in 10 s | -1.76 | -1.15 | 9.12 | 10.75 | 3.29 | 3.88 |

(continued)

| Group | Metric | Relative slope | | RC ratio to the mean | | Residual variability | |
|---|---|---|---|---|---|---|---|
| | | Short-term | Long-term | Short-term | Long-term | Short-term | Long-term |
| **Percen tage** | Stance % | - | - | - | - | - | - |
| | Swing % | 17.22 | 1.84 | 7.14 | 7.02 | 2.58 | 2.53 |
| | Single support % | -11.00 | 2.61 | 25.90 | 26.06 | 9.34 | 9.40 |
| | Double support % | 20.24 | 1.20 | 8.52 | 8.79 | 3.07 | 3.17 |
| **Asymm etry** | N/A | -10.87 | 1.85 | 25.91 | 27.01 | 9.35 | 9.74 |
| **IMU** | $Acc_{norm,peak}$ | -8.18 | 4.94 | 10.84 | 15.22 | 3.91 | 5.49 |
| | $Acc_{norm,peakSD}$ | 0.75 | 15.37 | 5.83 | 6.79 | 2.10 | 2.45 |
| | $Acc_{norm,range}$ | -15.46 | 4.93 | 14.64 | 27.20 | 5.28 | 9.81 |
| | $Acc_{norm,rangeSD}$ | 1.32 | 16.52 | 6.66 | 7.20 | 2.40 | 2.60 |
| | $Gyro_{norm,meanSD}$ | -13.81 | 2.25 | 14.83 | 25.97 | 5.35 | 9.37 |
| | $Gyro_{norm,peak}$ | -6.36 | 5.31 | 9.68 | 14.30 | 3.49 | 5.16 |
| | $Gyro_{norm,peakSD}$ | -4.97 | 0.91 | 3.15 | 3.26 | 1.13 | 1.18 |
| | $Gyro_{norm,range}$ | -6.06 | 0.67 | 2.66 | 3.74 | 0.96 | 1.35 |
| | $Gyro_{norm,rangeSD}$ | -17.15 | -0.24 | 10.30 | 11.44 | 3.72 | 4.13 |
| | $Gyro_{norm,rmse}$ | 1.80 | 4.79 | 6.45 | 9.23 | 2.33 | 3.33 |

RC = repeatability coefficient, Acc = accelerometer, norm = Euclidean norm, SD = standard deviation, Gyro = gyroscope, rmse = root mean squared error.

**B4.0 DISCUSSION** - In this study, the between-day reliability of 63 spatiotemporal gait quality metrics was evaluated using the validated IF described above in Section A and the FIT during Short-term (five days) and Long-term (six months) walking protocols. Although our overarching goal is to develop a tool for monitoring free-living gait quality in people with neurological conditions, these populations are susceptible to fluctuations in their walking quality, making it difficult to evaluate longitudinal gait metric reliability. As such, reliability was evaluated in healthy people during controlled treadmill walking to identify which gait metrics can be used as a benchmark to further assess aberrant movement quality and to longitudinally monitor changes to gait quality that may indicate disease progression (improvement, maintenance, worsening).

ICCs are the most commonly reported reliability statistic in the literature; however, additional reliability metrics from a hierarchical model were used to evaluate reliability because ICCs are negatively affected by low levels of between-subject variability (e.g., healthy controls, controlled treadmill walking) (Kobsar et al., 2020; Koo & Li, 2016). Overall, 23 metrics were identified as reliable (see Table 2). Of these metrics, stride time, stance time, step time, acceleration peak, and acceleration range had excellent reliability for both Short- (average ICC = 0.932) and Long-term (average ICC = 0.931) protocols. Stride velocity and distance travelled in 10 s had excellent Short-term reliability (average ICC = 0.926) and good Long-term reliability (average ICC = 0.889). Acceleration rangeSD was the only metric to have good short-term (ICC = 0.886) and excellent long-term (ICC = 0.905) reliability. Moreover, stride length, cadence, double support time, gyroscope peak, and gyroscope range had good Short-(average ICC = 0.821) and Long-term reliability (average ICC = 0.810). All percentage metrics (i.e., stance percent, swing percent, double support percent, single support percent) and gyro metrics (i.e., gyro meanSD, gyro peakSD, gyro rangeSD) had good Short-term reliability (average ICC = 0.819), but moderate Long-term reliability (average ICC = 0.634). Gyro RMSE, was the only metrics to have moderate reliability for both Short- (ICC = 0.741) and Long-term reliability (average ICC = 0.579).Notably, all asymmetry metrics and the majority of variability metrics were not identified as reliable for both Short- and Long-term protocols.

When evaluating new technology, issues related to reliability may stem from equipment/software error, human error, and/or movement variability. The validity of the IF relative to gold-standard optical motion capture equipment has been confirmed in Section A above; therefore, it is likely that equipment and/or software errors had no meaningful impact on reliability. Moreover, the technology used was designed to minimize human error since the insoles are placed in the shoes; thus, the location of the IMUs and pressure sensors are relatively fixed between walking sessions. While it is possible to have manufacturing inconsistencies between insoles, these differences are likely attenuated during factory calibration, and any residual differences are mitigated through post-processing as shown in Section A above. As such, it is believed that the reliability of gait quality metrics primarily captured the expected movement variability in a healthy population.

SECTION C: COMPOSITE INDEX (CI) DEVELOPMENT

This section describes a method that quantifies walking quality in PwMS by classifying gait metrics using an SVM and producing a CI based on the projection location relative to a decision boundary. The presented CI is trained on reliable gait metrics and is highly correlated with clinically meaningful disability metrics. By continuously monitoring the gait quality of PwMS in this way, it may be possible to identify disease progression to inform decisions related to interventions and objectively evaluate the efficacy of prescribed interventions.

The purpose of this work is to demonstrate the feasibility and efficacy of a CI score for quantifying disease status in clinical populations from walking data obtained from our Insole Framework (IF). People with multiple sclerosis (PwMS) were selected as the population of interest, as it is well known that their gait quality is significantly affected by disease severity, and many clinical tools (e.g., expanded disability status scale (EDSS), 12-item MS Walking Scale (MSWS), Timed 25-foot Walk (T25FW)) currently used in practice are designed to evaluate patient walking quality. However, these assessments are typically only performed on an annual/semi-annual basis and require a level of change to be meaningful (e.g., T25FW 20%; Hobart et al., 2013), leading to a reactive version of healthcare where the PwMS may have already experienced a significant and irreversible decline between visits. Therefore, we have identified PwMS as a population that could significantly benefit from continuous monitoring of their gait quality.

**C2.0 METHODS - C2.1** - **PARTICIPANTS** Twenty-seven healthy participants and 45 PwMS were recruited for this investigation. Healthy was defined as any individual who has not experienced a musculoskeletal injury within the last 6 months at the time of testing and does not suffer from a neurological disorder. Healthy participants were recruited as two populations: those who performed a single day of targeted walking trials (Healthy _L; N = 22) and those who performed a six-month walking protocol on a treadmill (Healthy_T; N = 5). PwMS were recruited as two populations: those who performed purposeful walks in free-living conditions (PwMS_FL; N = 26) and those who performed a single day of targeted walking trials (PwMS_L; N = 19). PwMS were recruited from The Ottawa Hospital (Ottawa, Canada; N = 37) and Brigham and Women's Hospital (Boston, USA; N = 8). All PwMS_L had undergone a neurological exam within the last 12 months where they were given an Extended Disability Status Score (EDSS; Kurtzke, 1983) of less than 7.0; PwMS_FL were given an EDSS score at each clinical visit. The average EDSS for all PwMS was $3.57 \pm 1.80$ (range: 0 - 6.5), the average 12-item Multiple Sclerosis Walking Scale (MSWS-12; Hobart et al., 2003) score was $52.78\% \pm 24.25$ (range: 20 - 90), and 18 PwMS expressed using one or more assistive devices in their daily lives. Participant demographics, including EDSS and MSWS-12 scores are presented in Table 1 below.

Table C1. Participant demographics

|  | Sex M\|F | Age (years) | Height (cm) | Mass (kg) | EDSS Score | MSWS-12 Score |
|---|---|---|---|---|---|---|
| Healthy | 14\|13 | 27.7 (6.2) | 174.4 (9.5) | 75.4 (14.5) | - | - |
| PwMS | 11\|34 | 49.1 (14.2) | 162.3 (9.2) | 77.9 (24.6) | 3.6 (1.8) | 52.8 (24.3) |

Note: Values are presented as mean (standard deviation) or total count when appropriate. M = Male; F = Female, PwMS = People with Multiple Sclerosis; EDSS = Expanded Disability Status Score; MSWS-12 = 12-Item Multiple Sclerosis Walking Scale.

**C2.2 Movement Protocol and Instrumentation** - The movement protocol was separated into three streams: a single-day lab-based protocol (Healthy _L, PwMS_L); a four-month treadmill protocol (Healthy _T); and a six-month free-living purposeful walking protocol (PwMS_FL).

**C2.2.1 Single-day Lab-based Protocol** - Twenty-two Healthy and 19 PwMS attended the University of Ottawa Movement Biomechanics and Analytics Laboratory on a single day. Details of the single-day lab-based protocol are described above in Section A; the current investigation uses metrics calculated by the Insole Framework (IF) calculated during the 500-metre indoor walk (i.e., 20 laps of 25 metres indoors), 500-metre outdoor walk (i.e., walking outside on a multi-use pathway with four main turns), and the 125-metre indoor walk with stair navigation (i.e., walking indoors with a stair ascend and descend portion). Only Healthy participants performed the 500-metre outdoor walk, and the 125-metre indoor walk was optional for the PwMS_L (N = 10). For all activities, participants wore the appropriately sized pair of instrumented shoe insoles (ReGo, Moticon, Germany) that streamed raw pressure data from 16 sensors and tri-axial inertial measurement unit (IMU) data to a smartphone application (Celestra Health Systems, Canada) downloaded on a laboratory-owned smartphone (iPhone 13, Apple, USA). The sizes used in this investigation ranged from S2 to S7 (EU shoe size 34-45, US shoe size Women 4-13, Men 3.5-11.5).

**C2.2.2 Long-term Lab-based Protocol** - The full details have been presented above in Section B.

**C2.2.3 Free-living Purposeful Walking Protocol** - Twenty-six PwMS were recruited from the general MS population in the surrounding areas of Ottawa, Ontario, Canada, and Boston, Massachusetts, USA. Depending on their location, participants were asked to attend The Ottawa Hospital or the Brigham and Woman's Hospital for a single day, where they were sized for a pair of instrumented shoe insoles (50 Hz; ReGo, Moticon, Germany), asked to download a smartphone application (Celestra Health Systems, Canada) on their iPhone (Apple, USA) and were given a full neurological assessment by their physician. The sizes used in this investigation ranged from S2 to S7 (EU shoe size 34-45, US shoe size Women 4-13, Men 3.5-11.5). If a participant did not have access to an iPhone, one was loaned to them. During the visit, participants were given a neurological exam to produce an up-to-date EDSS score, filled out an MSWS-12 questionnaire, performed the T25FW protocol, performed a one-minute walk, completed a Symbol Digit Modalities Test and a Nine-Hole Peg Test (9HPT). The T25FWT was recorded using standard methods (i.e., time-based results only), while the one-minute walk was recorded with the IF and was video recorded from the neck downwards by a research coordinator. Clinical visits were done at the study onset, three months, and six months.

During their first clinical visit, participants were trained to use the Celestra Health Systems smartphone application to record 15-minute walking samples in a free-living environment. Participants were instructed to record a 15-minute walking sample thrice weekly for six months. During each recording, participants were asked to perform purposeful walking outside or in large indoor facilities (e.g., a shopping mall). Purposeful walking was defined as a dedicated walk for exercise, socializing, or commuting. Participants were told they could stop at any point during the walk (e.g., talking to a neighbour, waiting at a crosswalk) since a human activity recognition layer described above in Section A identified non-walking activities and discarded them from the analysis. The current work uses a small subset of data (five walks surrounding a clinical visit) from the six-month data collection.

**C2.3 Composite Index (CI) Development - C2.3.1 Data Preparation** - For each walking session, data preparation procedures followed the methods outlined above in Example A. In Python (version 3.11), the walking data from the single-day laboratory protocol were organized in where each row was a 10-second segment, and each column was a spatiotemporal gait metric with two additional columns for the participant identifier and class label (i.e., Healthy = 0, PwMS = 1). The final size of the data before feature selection was 1835 10-second segments by 188 metrics.

C2.3.2 Feature Selection - **A feature selection procedure was performed to reduce the number of features before training the CI model:** between-side averaging, reliability assessment, significance testing, and partial least square (PLS) regressions. When appropriate, metrics were averaged between sides of the body (e.g., (right stride time + left stride time) / 2 = stride time). The between-side averaging step reduced the feature set to 68 features. As described in Section A above, metrics were evaluated for their absolute reliability, precision, and temporal stability over six months of weekly six-minute walking sessions on a FIT in a healthy population. This feature selection step reduced the feature set to 23 metrics. The significance testing step consisted of assessing the metrics for significant differences between the healthy population and PwMS using two-tailed independent T-tests, Welch's tests, or Mann-Whitney U, depending on the homogeny and distribution of the data. All tests had a significance cut-off of $p < 0.05$; all but gyroscope meanSD (i.e., the average of pointwise standard deviations across all gait cycles for the gyroscope in Euclidean space) were identified as significantly different between populations resulting in 22 metrics for training (Table C2).

Table C2. Input Features for Partial Least Squares Regression

| Core | Variability | Pace | Percentage | IMU |
|---|---|---|---|---|
| Stride Time (s) | Stride Time CV | Cadence (steps/min) | Stance Percent (%) | Acceleration Peak |
| Stance Time (s) | | Stride Velocity (m/s) | Swing Percent (%) | Acceleration PeakSD |
| Double Support Time (s) | | Distance in 10 s | Double Support Percent (%) | Acceleration Range |
| Step Time (s) | | | Single Support Percent (%) | Acceleration RangeSD |
| Stride Length (m) | | | | Gyroscope Peak |
| | | | | Gyroscope PeakSD |
| | | | | Gyroscope Range |
| | | | | Gyroscope RangeSD |

(continued)

| Core | Variability | Pace | Percentage | IMU |
|------|-------------|------|------------|-----|
|  |  |  |  | Gyroscope RMSE |

Note: All metrics (except Distance in 10 s) are the average of all gait cycles within a 10-second segment. Peak = average of the highest value observed for each gait cycle. PeakSD = standard deviation of the peaks. RangeSD = standard deviation of the range (max - min) across gait cycles. MeanSD = the average of pointwise standard deviations across all gait cycles. RMSE = root mean squared error.

A PLS regression model was fit to the 27 features (Table C2) to obtain latent variables for the number of components specified. Data were first scaled using the using sklearn's standard scaler (equation 1) and rounded to four decimal places.

$$scaledMetric = \left(value - metric_{average}\right)/metric_{standardDeviation} \qquad \text{Eq C1.}$$

The number of components in the PLS regression models was iteratively increased to train support vector machines (SVMs) with increasing feature spaces from one to 27 features. Using the latent variable from the PLS feature selection step as input, SVMs were trained to classify 10-second walking segments as someone who is Healthy (0) or is a PwMS (1). The same training approach was used for all 27 SVMs trained. Hyperparameters were identified using a grid search approach using the parameters reported in Table C3. The SVMs were cross-validated using a leave-one-group-out approach where each participant was a group. Accuracy, weighted F1 scores, and receiver operating characteristic (ROC) area under the curve (AUC) were used to evaluate model performance.

| Table C3. Hyperparameter Grid Search | |
|--------------------------------------|---|
| Hyperparameter | Searched Values |
| C | 0.1, 1, **10** |
| Gamma | **0.001,** 0.01, 0.1, 1 |
| Kernal | linear, **rbf,** poly |
| Note: **Bolded** values are the hyperparameters for the selected model. RBF = radial basis function, poly = polynomial. | |

C2.3.4 Composite Index (CI)

Twenty-two SVMs were trained to classify gait metrics obtained from 10-second segments as either belonging to someone who is Healthy or a PwMS. To calculate the CI, the input data were classified and projected onto the trained decision boundary to identify its coordinates. By projecting the training data onto the decision boundary and retaining their relative coordinates, Z-scores were calculated for new data and transformed into a percentage between 0 and 100% using the cumulative distribution function in the SciPy library.

**Statistics** - The CI scores from each 10-second segment were averaged to obtain one CI score per walking trial. For the PwMS_FL data, the resulting CI from their five walks were averaged to produce one CI per person. For every PwMS, the resulting CI score from every SVM model trained was correlated to their disability metrics using Spearman correlations: EDSS, MSWS-12 percentage, individual MSWS-12 questions, T25FW, SDMT, and 9HPT scores. Correlations were performed within each population to better understand the model's ability to generalize to unseen data (i.e., PwMS_FL). As a benchmark, the same procedure was repeated to calculate Spearman correlations of the EDSS scores to the disability metrics. A Spearman correlation of 0.10-0.29, 0.30-0.49 and 0.5+ were interpreted as small, medium and large, respectively.

To assess the reliability of the CI, intraclass correlations ($ICC_{3,1}$) with absolute agreement were employed on the Healthy_T data over six months of walking on a treadmill once weekly, assigning the CI as the rating, trial number as the rater, and participants as the target. $ICC_{3,1}$ with absolute agreement was chosen since this is a test-retest approach; therefore, the raters cannot be considered random and should absolutely agree between walking sessions (Koo & Li, 2016). The relative temporal stability and precision of the CI were evaluated using a linear mixed-effects model, with elapsed days since the first walking session as a fixed effect and participant identifier as a random effect. From the linear mixed-effects model, the relative slope (i.e., rate of change over elapsed days expressed as a percentage of the mean), repeatability coefficient (RC) to mean ratio (i.e., the 95% range of expected values within a participant expressed as a percentage of the mean), and residual variability (i.e., within-subject variability expressed as a percentage of the mean) were evaluated.

**C3.0 RESULTS** - **C3.1 MODEL SELECTION** - Twenty-two SVMs were trained to classify walking data as Healthy (0) or PwMS (1). The selection procedure for the final model focused on the generalizability of the PwMS_FL disability metrics and fit to the PwMS_L disability metrics. That is, the selected model had to have Spearman correlation values equal to or greater than the EDSS for the majority of the PwMS_FL disability metrics. Four models remained after this first step. The correlation of the GCI and EDSS to the MSWS-12 score in the PwMS_L was then evaluated. That is the selected model was the only one that produced a GCI score with a higher Spearman correlation than EDSS versus the MSWS-12 score. The model with 14 PLS latent variables was selected. Correlations to each disability metric and model performance metrics are presented in Tables C4 and C5. Correlations to each MSWS-12 question for the combined dataset are depicted in FIG. 25.

Table C4. Selected Model's Classification and Reliability Performance

| Accur acy | F1 | AU C | Relative Slope | RC to Mean Ratio | Residual Variability | ICC$_{3,1}$ [95% CI] | F |
|---|---|---|---|---|---|---|---|
| 90.18 % | 92. 84 % | 0.9 75 | 5.99% | 25.50% | 9.20% | 0.890 [0.74 0.99] | 185. 84 |
| Note: F1 represents the harmonic mean of precision and recall. AUC represents the area under the receiver operating characteristic (ROC) curve. The degrees of freedom for the ICC are 4 and 84. | | | | | | | |

Table C5. Spearman Correlation Results

| | PwMS_L | | PwMS_FL | | | | | |
|---|---|---|---|---|---|---|---|---|
| | MSWS -12 | EDSS | MSWS -12 | EDS S | SDM T | 9HPT_N D | 9HPT_ D | T25FW |
| CI | 0.696 | 0.766 | 0.85 | 0.859 | 0.718 | 0.586 | 0.616 | 0.834 |
| EDSS | 0.688 | 1 | 0.835 | 1 | 0.566 | 0.561 | 0.638 | 0.797 |
| MSWS-12 | 1 | 0.688 | 1 | 0.835 | 0.591 | 0.640 | 0.689 | 0.756 |
| T25FW | - | - | 0.756 | 0.797 | 0.642 | 0.750 | 0.600 | 1 |

FIG. 25 shows the MSWS-12 Question Correlations. Spearman correlation results for all people with multiple sclerosis (PwMS); all correlations are significant at $p < 0.0001$. Correlations are expressed as positive for visualization purposes. MSWS-12 represents the 12-point Multiple Sclerosis Walking Scale. Qs represent each question on the MSWS-12. Questions ask about how much in the previous two weeks a person's multiple sclerosis has limited walking ability (1), limited running ability (2), limited stair navigation (3), increased the difficulty of standing tasks (4), limited balance when standing/walking (5), limited walking distance (6), increased walking effort (7), necessity for indoor (8) and outdoor (9) walking support, slowed walking (10), affected walking smoothness (11), and walking concentration (12).

**C4.0 DISCUSSION** - The current work demonstrates a method that distills many important gait metrics obtained from our IF from free-living and laboratory settings into a single CI score that can be used to encapsulate overall walking quality in PwMS. The method developed uses the relative decision boundary projection locations of an SVM trained using PLS latent variables from gait metrics that are longitudinally reliable and significantly differ between Healthy and PwMS. The chosen SVM is informed by gait metrics belonging to many of the domains described in gait literature: pace, rhythm, variability, asymmetry, and complexity, and are very similar to the ones found by Trentzsch et al. (2021) for discerning Healthy from PwMS. Because the overarching goal driving this work is long-term monitoring, many variability and asymmetry metrics were excluded from the modelling due to poorly reported longitudinal reliability seen in a healthy population as discussed in Section B above. Previous research in this field used PCA as a feature dimensionality reduction technique (e.g., Arcolin et al., 2019; Morris et al., 2017; Olney et al., 1998); while PCA is a valid approach to capture the variance within a set of variables (Hair et al., 2009), the current work used PLS, which is similar to PCA but aims to maximize the covariance between groups (Twisk, 2019). Using the PLS latent variables, the selected model was able to achieve an accuracy, F1, and AUC scores of 90.18%, 92.84%, and 0.975, which are comparable to similar studies in the literature (Li et al., 2023; Plummer et al., 2022; Wang et al., 2020; Zhai et al., 2025).

The results from the current demonstrate excellent classification performance; however, the overarching goal is to develop an objective tool to aid clinicians in improving assessments of movement quality in patients. As such, it is important to understand how CI scores derived from the current model relate to common evaluation tools/metrics utilized in the current standard of care. There are many patient- and clinician-reported outcomes (e.g., EDSS Kurtzke, 1983), MSWS-12 Hobart et al., 2003), T25FW Motl et al., 2017)) that are used to evaluate disease severity, and have been investigated to identify how much of a relative change is clinically meaningful (Motl et al., 2017). Of these, the EDSS is the most accepted method of evaluating and classifying patients based on their disability status; therefore, EDSS scores were used as a 'ground truth' benchmark for evaluating the clinical meaningfulness of the model, and correlations with other disability metrics were evaluated to ensure the analysis was comprehensive. Spearman's correlations between CI scores and disability metrics were considered strong and closely resembled or were generally superior to the coefficients obtained by EDSS. Importantly, the correlation results for the CI versus EDSS and MSWS-12 were similar between the PwMS_L and PwMS_FL populations. Since the PwMS_FL were unseen during model training and the participants were unconstrained and unsupervised in their walks, which is what would be expected when deployed in real-world settings, thus these results are ecologically valid.

Other CI methods used to classify clinical populations from healthy controls (e.g., Li et al., 2023; Mar et al., 2020; Plummer et al., 2022; Wang et al., 2020; Zhai et al., 2025) have shown favourable results; however, only one study leveraged wearable movement data to calculate CI scores for evaluating disease progression using a machine learning algorithm, similar to what was presented here (Zhai et al., 2025). Using generalized estimating equations (GEE) and penalized GEE, Zhai et al. (2025) can classify movers as PwPD or Healthy, even at the de novo PD stage, with exceptional performance; their CI method also produces comparable two-year long trends to a clinically accepted measure in PD (i.e., MSD-UPDRS Part III). However, the method outlined by Zhai et al. (2025) currently relies on obtaining data from a six-sensor IMU system during multiple targeted movements (i.e., two-minutes of walking, timed up and go (TUG), and postural sway). While this protocol is acceptable for a clinical environment where highly qualified people can be trained to reliably collect and prepare the data, it would be incredibly challenging to adapt it to a patient-led remote monitoring solution, as is the goal of the current work. ; however, only one study leveraged wearable movement data to calculate CI scores for evaluating disease progression (Wang et al., 2020). The SVM model used in the study by (Wang et al., 2020) achieved an accuracy of 93.3% when classifying three groups of patients with neurological diseases and one healthy control group; however, the metric used to quantify gait abnormality was not evaluated relative to commonly used clinical measures, and the authors concluded that Euclidean distance to the corresponding hyperplane (i.e., decision boundary) may be a better indicator of disease status. Moreover, the study by Wang et al. (2020) evaluated gait during one controlled 10-metre walk, limiting the generalizability to free-living conditions and the reliability of longitudinal assessments using continuous monitoring.

The method presented in the current study was developed using longitudinally reliable metrics discussed in Section B, to instill confidence that the outputs can be used by clinicians to monitor reliable longitudinal trends in gait quality. Moreover, the system was developed for remote monitoring, which not only reduces patient burden related to travelling to and from clinical appointments but also provides a means to detect worsening gait quality in patients between visits. This between-visit monitoring has been identified as an important future direction in providing earlier interventions aimed at slowing disease progression and preventing harmful attacks known to significantly affect disability and quality of life in PwMS (Martin et al., 2006). The current method also provides a tool to objectively evaluate the efficacy of new interventions and to modify treatments accordingly. Moreover, the current method does not require costly hiring of additional staff to collect, analyze, and disseminate gait quality data, which also reduces the potential for human error and will help accelerate researchers' needs to collect the large amounts of data necessary for improving model generalizability and overall understanding of gait quality in relation to disease and disability status (Martin et al., 2006). While the presented CI score can provide clinicians with a single 'high-level' metric to evaluate gait quality trends, individual gait metrics should also still be available to inspect, providing clinicians with the option to explore changes in gait patterns in more detail, and providing rehabilitation specialists with targeted impairments to address.

SECTION D - GAIT VARIABILITY

The following section discusses how the improved IF can quantify gait variability and quality during prolonged walking outside the laboratory amongst PwMS. The findings showed that a deterioration in gait quality could be captured regardless of disability level, and can capture increases in ST gait variability metrics amongst individuals in the severe EDSS group. The results of this work support the use of the IF to improve the accessibility of sophisticated gait analyses for clinical and free-living use. Although gait variability metrics show promise in reflecting gait quality in PwMS, their usefulness as clinical biomarkers is limited because few valid, reliable, and accessible measurement tools exist to obtain these metrics outside the laboratory. The novel framework discussed above in Section A has been developed for clinical and commercial use outside of the laboratory. As discussed above, the improved Insole Framework (IF) automatically recognizes ambulatory activities (e.g., walking, turning, climbing stairs, etc.) using machine learning-based human activity recognition (HAR), then processes the data using standardized approaches to compute spatiotemporal (ST) metrics. The IF enables the assessment of prolonged walking in clinical and free-living settings, which may be better indicators of true walking performance compared to laboratory assessments. Since the IF has shown strong HAR performance and has been validated against gold-standard biomechanical measurement tools (as discussed in Section A), has been used to identify long-term reliable gait metrics (as discussed in Section B), and has been successfully implemented in free-living conditions (as discussed in Section C) it is believed that the IF is suitable for assessing gait quality outside of the laboratory.

The primary objective of this study was to employ the IF to quantify changes in gait variability and quality in PwMS throughout a prolonged walking trial. The secondary objective was to compare gait variability and quality between mild ($\leq$ 3), moderate (3.5 - 4), and severe (> 4) EDSS groups, and their interaction with walking distance. To this end, PwMS of various EDSS scores were recruited to perform a 500-metre indoor walk, a length was selected to induce fatigue (Burschka et al., 2012), while being relevant to tests performed in clinical practice (Kurtzke, 1983). The IF was used to measure pressure and kinematics, which were used to compute the CoV of various ST gait metrics. Additionally, a composite index (CI) score was computed to assess gait quality in Section C. It was hypothesized that the IF would capture an increase in gait variability and a decrease in gait quality throughout the trial. Furthermore, it was hypothesized that changes in gait variability would be larger in the severe EDSS group compared to the mild and moderate EDSS groups. These findings can improve patient care for PwMS by improving the accessibility of implementing complex gait analyses for clinical and at-home use.

**D2.0 METHODS - D2.1 Participant Recruitment** - Nineteen PwMS were recruited for this study from The Ottawa Hospital, who had undergone a neurological exam within the last 12 months where they were given an EDSS of less than 7.0 (mean EDSS score = 3.8, median EDSS score = 4). The average (standard deviation) age, height, and mass of the PwMS were 54.2 (16.5) years, 158 (7.28) cm, and 78.7 (26.4) kg. The average EDSS for the PwMS was 3.8 $\pm$1.6 (range: 0-6), the average 12-item MS Walking Score (MSWS-12) was 60.9% $\pm$22.2 (range: 20-90), and nine PwMS expressed using one or more assistive devices in their daily lives.

**D2.2 Movement Protocol and Instrumentation** - The experimental protocol was discussed above in Section A.

**D2.3 Data Analysis** - The IF's gait event detection and the HAR algorithms were described above. The identified gait events were used to calculate ST metrics within standardized 10-second segments. Seven ST metrics were used for the current analysis: step time, cadence, stride, stance, swing, single support, and double support times. For each 10-second segment, the mean and standard deviation of the ST metrics were computed and used to calculate CoV by dividing the standard deviation by the mean.

A composite index (CI) score was also assigned to each 10-second segment throughout the trial, described in detail in Section C. This CI score reflects a participant's gait quality with reference to walking patterns exhibited by healthy controls.

**D2.4 Statistical Analysis** - For statistical analyses, participants were assigned to one of three groups based on their EDSS scores: mild (EDSS $\leq$ 3; n = 6), moderate (EDSS = 3.5 - 4; n = 5), and severe (EDSS > 4; n = 8). To analyze the change in gait variability throughout the 500-metre walking trials, the ST metrics were averaged for each of the four quarters of the total walking distance (i.e., 125 m). Normality and sphericity of the data were assessed using the Shapiro-Wilk and Mauchly's tests, respectively. Based on these tests, linear mixed models were selected and implemented using RStudio v2024.12.0 software (RStudio, Massachusetts, USA). Disability status was a between-subject factor (three levels: mild, moderate, and severe), and distance was a within-subject factor (four levels: quarters 1, 2, 3, 4). Significance was set to $\alpha$ = 0.05.

**D3.0 RESULTS** - **D3.1 Summary Metrics** - The average time taken to complete the walking trial was 627.9 seconds (standard deviation = 254.9 s). The mean and standard deviation of the dependent variables for each quarter of the 500-metre walking trial are presented in Table D1 and FIG. 26. The mean and standard deviation of the dependent variables for each EDSS level are presented in Table 2. In FIG. 26, the mean of the dependent variables across each quarter of the 500-metre walking trial. CI = composite index; CoV = coefficient of variation.

**Table D1.** The mean (standard deviation) of the dependent variables across the walking trial, separated into quarters (125 m segments) of the 500-metre walking trial. CI = composite index; CoV = coefficient of variation.

| Dependent variable | Quarter 1 | Quarter 2 | Quarter 3 | Quarter 4 | Overall |
|---|---|---|---|---|---|
| CI Score | 35.36 (22.14) | 33.59 (23.17) | 33.45 (22.23) | 32.95 (22.12) | 33.84 (21.98) |
| Step Time Cov | 6.89 (4.35) | 7.22 (4.70) | 7.07 (4.46) | 7.10 (4.25) | 7.07 (4.36) |
| Cadence CoV | 6.91 (4.31) | 7.23 (4.74) | 7.15 (4.52) | 7.12 (4.21) | 7.10 (4.36) |
| Stride Time CoV | 2.87 (1.29) | 2.93 (1.51) | 2.99 (1.80) | 3.10 (2.16) | 2.97 (1.69) |
| Stance time CoV | 3.46 (1.69) | 3.80 (2.01) | 3.91 (2.37) | 3.94 (2.67) | 3.78 (2.18) |
| Swing Time CoV | 5.00 (1.66) | 5.11 (2.19) | 5.18 (2.56) | 5.35 (2.99) | 5.16 (2.36) |
| Single Support Time CoV | 4.79 (1.67) | 4.89 (2.05) | 4.97 (2.48) | 5.21 (2.94) | 4.97 (2.29) |
| Double Support Time CoV | 7.36 (2.52) | 7.63 (3.64) | 7.88 (3.45) | 7.63 (3.55) | 7.62 (3.26) |

Table D2. The mean (standard deviation) of the dependent variables for mild, moderate, and severe EDSS levels. CI = composite index; CoV = coefficient of variation.

| Dependent variable | Mild | Moderate | Severe | Overall |
|---|---|---|---|---|
| CI Score | 56.63 (7.72) | 40.40 (17.34) | 12.64 (6.87) | 33.84 (21.98) |
| Step Time Cov | 4.14 (1.53) | 5.76 (4.00) | 10.09 (4.15) | 7.07 (4.36) |
| Cadence CoV | 4.13 (1.51) | 5.79 (3.97) | 10.15 (4.15) | 7.10 (4.36) |
| Stride Time CoV | 1.76 (0.37) | 2.30 (0.51) | 4.30 (1.83) | 2.97 (1.69) |
| Stance time CoV | 2.32 (0.40) | 2.62 (0.56) | 5.60 (2.29) | 3.78 (2.18) |
| Swing Time CoV | 3.35 (0.43) | 4.42 (0.92) | 6.98 (2.54) | 5.16 (2.36) |
| Single Support Time CoV | 3.17 (0.43) | 4.27 (0.93) | 6.75 (2.44) | 4.97 (2.29) |
| Double Support Time CoV | 5.81 (0.98) | 6.00 (0.97) | 10.00 (3.79) | 7.62 (3.26) |

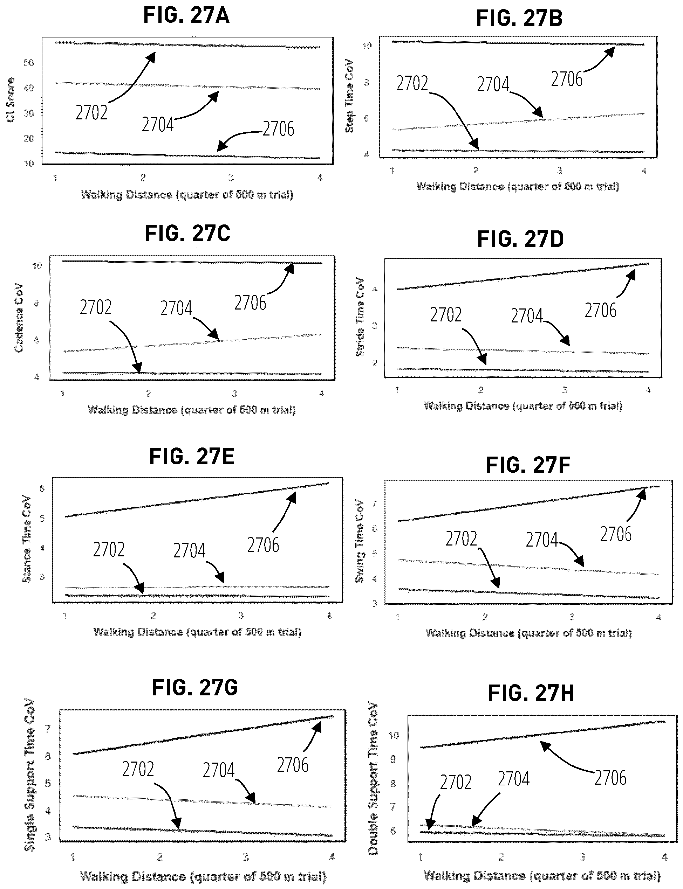

**D3.2 Linear Mixed Models** - A summary of the results of the linear mixed models is presented in **Table D3.** The main effects and interactions are depicted in FIG. 27A to FIG. 27H. As can be seen, the main and interaction effects across each quarter of the 500-metre walking trial. The EDSS levels are indicated as mild (2702), moderate (2704), and severe

(2706). The dependent variables are A) Composite Index (CI) score, B) Step Time Coefficient of Variation (CoV), C) Cadence CoV, D) Stride Time CoV, E) Stance Time CoV, F) Swing Time CoV, G) Single Support Time CoV, and H) Double Support Time CoV. Significant interaction effects at $\alpha$ = 0.05 are indicated with an asterisk.

Table D3. Linear Mixed Model Results. CI = composite index; CoV = coefficient of variation; EDSS = Expanded Disability Status Scale; * = significant at $\alpha$ = 0.05.

| Dependent variable | Predictor | Estimated coefficient | 95% Confidence interval | *p*-value |
|---|---|---|---|---|
| CI Score | Distance | -0.74 | -1.44 - -0.03 | **0.040** * |
| | EDSS Level | -30.86 | -39.80 - -21.93 | **<0.001** * |
| | Interaction | -0.10 | -1.25 - 1.06 | 0.869 |
| Step Time CoV | Distance | 0.07 | -0.13 - 0.28 | 0.488 |
| | EDSS Level | 4.24 | 1.36 - 7.12 | **0.005** * |
| | Interaction | -0.01 | -0.35 - 0.32 | 0.940 |
| Cadence CoV | Distance | 0.08 | -0.13 - 0.29 | 0.435 |
| | EDSS Level | 4.27 | 1.40 - 7.14 | **0.004** * |
| | Interaction | -0.01 | -0.34 - 0.33 | 0.976 |
| Stride Time CoV | Distance | 0.05 | -0.06 - 0.16 | 0.353 |
| | EDSS Level | 1.34 | 0.31 - 2.37 | **0.012** * |
| | Interaction | 0.18 | 0.00 - 0.36 | **0.048** * |
| Stance Time CoV | Distance | 0.12 | -0.01 - 0.26 | 0.065 |
| | EDSS Level | 1.63 | 0.36 - 2.90 | **0.012** * |
| | Interaction | 0.27 | 0.06 - 0.49 | **0.013** * |
| Swing Time CoV | Distance | 0.05 | -0.11 - 0.22 | 0.519 |
| | EDSS Level | 1.52 | 0.09 - 2.95 | **0.038** * |
| | Interaction | 0.42 | 0.15 - 0.69 | **0.003** * |
| Single Support Time CoV | Distance | 0.08 | -0.09 - 0.25 | 0.345 |
| | EDSS Level | 1.50 | 0.12 - 2.89 | **0.034** * |
| | Interaction | 0.41 | 0.14 - 0.68 | **0.004** * |

(continued)

| Dependent variable | Predictor | Estimated coefficient | 95% Confidence interval | *p*-value |
|---|---|---|---|---|
| Double Support Time CoV | Distance | 0.06 | -0.25 - 0.38 | 0.697 |
| | EDSS Level | 2.20 | -0.01 - 4.41 | 0.051 |
| | Interaction | 0.30 | -0.21 - 0.82 | 0.242 |

**D3.2.1 Effect of Distance** - A significant main effect of walking distance was found for the CI score only (p = 0.040). Specifically, as walking distance increased, participants' gait quality tended to decrease, regardless of EDSS level.

**D3.2.2 Effect of EDSS Level** - A significant main effect of EDSS level ($p \leq 0.038$) was found for all variables except double support time CoV (p = 0.051). Specifically, regardless of the distance walked during the trial, participants' gait quality was poorer with greater EDSS levels (i.e., a decrease in CI score); variability of the ST gait metrics tended to be greater with higher EDSS levels.

**D3.2.3 Interaction Between Distance and EDSS Level** - A significant interaction effect between distance walked and EDSS level was observed for stride time CoV, stance time CoV, swing time CoV, and single support time CoV ($p \leq 0.048$). These interaction effects are depicted in Figure 2D, E, F, and G. Throughout the walking trial, these four CoV variables increased in the severe EDSS group, while they showed a minor decrease in the mild and moderate EDSS groups.

**D4.0 DISCUSSION** - MS affects individuals' gait fatigability, which can be assessed by quantifying gait variability and quality. These analyses can provide insight into disease progression, informing treatment and rehabilitation. As these analyses have typically been difficult to implement outside of biomechanics laboratories, the primary objective of this work was to employ an IF to quantify changes in gait variability and quality amongst PwMS during a prolonged walking trial outside a laboratory environment. The secondary objective was to compare gait variability between EDSS levels and examine how this factor modulates the changes in gait variability and quality over a fatiguing trial. The findings showed that the IF captured a significant deterioration in gait quality regardless of EDSS level, while significant differences between EDSS levels were observed for all variables examined except for double support time variability. Furthermore, significant increases in stride time, stance time, swing time, and single support time variability were observed throughout the walking trial amongst individuals in the severe EDSS group, but not amongst individuals in the mild and moderate EDSS groups.

Despite the 500-metre walk being longer than traditional six-minute walk tests (PwMS averaged over 10 min to walk 500 metres), only overall gait quality (CI score) changed significantly throughout the walking trial when averaged across all EDSS levels. A deterioration in gait quality was expected as prolonged walking can induce fatigue, which affects gait quality in PwMS. The CoV of the ST metrics did not change throughout the walking trial when averaged across all EDSS levels, but increases were observed in the severe group (i.e., EDSS > 4) for four of seven ST variables. The IF identified significantly poorer gait quality and greater variability in ST metrics amongst people with greater EDSS severity (i.e., EDSS > 4), which is a finding that is highly supported in the literature (Crenshaw et al., 2006; Kalron 2006; Moon et al., 2016). While gait quality has been quantified using different metrics, literature has shown differences between individuals of differing MS severity in numerous spatiotemporal gait variables, such as stride length, velocity, and double support phase time, stride time, amongst other variables (Comber et al., 2017). As the current CI score accounts for 22 ST gait metrics and uses gait patterns from healthy controls as a reference, it is unsurprising that the present work reflected differences in gait quality between EDSS levels. Overall, these findings support the use of the IF to quantify gait variability and quality amongst PwMS with mild, moderate, or severe EDSS scores, which are important biomarkers associated with fatigability, fall risk, and quality of life.

Many of the functional units described in this specification have been labeled as "engines", or "modules", and/or "buttons" in order to more particularly emphasize their implementation independence. For example, an engine or module may be implemented as a hardware circuit comprising custom VLSI circuits or gate arrays, off-the-shelf semiconductors such as logic chips, transistors, or other discrete components. An engine may also be implemented in programmable hardware devices such as field programmable gate arrays, programmable array logic, programmable logic devices or the like.

Engines and modules may also be implemented in software for execution by various types of processors. An identified engine of executable code may, for instance, comprise one or more physical or logical blocks of computer instructions which may, for instance, be organized as an object, procedure, or function. Nevertheless, the executables of an identified engine need not be physically located together, but may comprise disparate instructions stored in different locations which, when joined logically together, comprise the engine or module and achieve the stated purpose for the module.

Indeed, an engine or module of executable code may be a single instruction, or many instructions, and may even be distributed over several different code segments, among different programs, and across several memory devices. Similarly, operational data may be identified and illustrated herein within engines, and may be embodied in any suitable form and organized within any suitable type of data structure. The operational data may be collected as a single data set, or may be distributed over different locations including over different storage devices, and may exist, at least partially, merely as electronic signals on a system or network. Where an engine or portions of an engine are implemented in software, the software portions are stored on one or more non-transitory, computer readable storage media.

The term "memory", "machine-readable storage medium" or "computer readable medium" as used herein refers to any medium or media that participates in providing instructions to a processor for execution. Such a medium may take many forms, including, but not limited to, non-volatile media, volatile media, and transmission media. Non-volatile media include, for example, optical or magnetic disks, such as data storage devices. Volatile media include dynamic memory, such as random access memory (RAM). Common forms of machine-readable media include, for example, floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM, a FLASH EPROM, any other memory chip or cartridge, or any other medium from which a computer can read. The machine-readable storage medium can be a machine-readable storage device, a machine-readable storage substrate, a memory device, a composition of matter effecting a machine-readable propagated signal, or a combination of one or more of them.

The term "processor" includes general-purpose microprocessors, microcontrollers, a Digital Signal Processors (DSP), Application Specific Integrated Circuits (ASIC), Field Programmable Gate Arrays (FPGA), Programmable Logic Devices (PLD), discrete hardware components, or any other suitable entity that can perform calculations or other manipulations of information. One or more processors in a multi-processing arrangement may also be employed to execute the sequences of instructions contained in a memory. In alternative aspects, hardwired circuitry may be used in place of or in combination with software instructions to implement various aspects of the present disclosure. Thus, aspects of the present disclosure are not limited to any specific combination of hardware circuitry and software.

Various aspects of the subject matter described in this specification can be implemented in a computing system that includes a back end component, e.g., such as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. The communication network can include, for example, any one or more of a LAN, a WAN, the Internet, and the like. Further, the communication network can include, but is not limited to, for example, any one or more of the following network topologies, including a bus network, a star network, a ring network, a mesh network, a star-bus network, tree or hierarchical network, or the like. The communications modules can be, for example, modems or Ethernet cards.

While the present disclosure describes various embodiments for illustrative purposes, such description is not intended to be limited to such embodiments. On the contrary, the applicant's teachings described and illustrated herein encompass various alternatives, modifications, and equivalents, without departing from the embodiments, the general scope of which is defined in the appended claims. Information as herein shown and described in detail is fully capable of attaining the above-described object of the present disclosure, the presently preferred embodiment of the present disclosure, and is, thus, representative of the subject matter which is broadly contemplated by the present disclosure.

## Claims

1. A computer implemented method for evaluating a user's gait, the method comprising:

   receiving, by a computing device having a memory and a processor, from a pair of smart insoles communicatively coupled to the computing device and worn by the user, data measured by one or more types of sensors;
   segmenting, by the processor of the computing device, the data into gait-related segmented data comprising one or more of: gait cycle segmentation or activity type;
   processing the gait-related segmented data, via one or more algorithms stored in the memory of the computing device, to determine one or more of:

      gait patterns associated with the segmented data;
      gait parameters associated with the segmented data;
      gait phenotypes associated with the segmented data;

   calculating, via one or more algorithms a composite gait quality score, based on a combination and interaction of at least two of: the segmented data, the gait patterns, the gait parameters, and the gait phenotypes.

2. The computer implemented method of claim 1, further comprising:

displaying, on a graphical user interface of a user device communicatively coupled to the computing device, the composite gait quality score.

3. The computer implemented method of claim 2, wherein the graphical user interface comprises one or more of: a clinician portal and a patient smart phone app;
   wherein the clinician portal and patient app are further configured to receive raw and processed patient data from the system.

4. The computer implemented method of claim 1, wherein determining, via one or more algorithms a composite movement quality score, further comprises:
   training a support vector machine (SVM), of the one or more algorithms, to classify walking data associated with a plurality of participants in one or more groups.

5. The computer implemented method of claim 4, wherein training the SVM comprises a feature selection step to remove redundant features and identify one or more designated metrics for evaluating gait in a specific patient population.

6. The computer implemented method of claim 1, wherein the one or more sensor types comprise at least one of: an accelerometer, a gyroscope, a magnetometer, a pressure sensor, and a temperature sensor.

7. The computer implemented method of claim 1, wherein the parameters comprise gait metrics from said heel strike, foot on floor, heel raise and toe off data.

8. The computer implemented method of claim 1, wherein the gait patterns comprise a gait signature of the individual from said gait metrics.

9. The computer implemented method of claim 1, wherein determining the composite gait quality score further comprises:
   determining a progression over time of the combination and interaction of at least two of: the segmented data, the gait patterns, the gait parameters, and the gait phenotypes.

10. The computer implemented method of claim 8, wherein determining the composite gait quality score further comprises:
    an assessment of the user's gait signature and recommendations for improvements.

11. The computer implemented method of claim 1, wherein determining the composite gait quality score further comprises:
    an assessment of a change in a patient's neurological disease condition based on measured changes to the patient's gait.

12. The computer implemented method of claim 1, wherein determining the composite gait quality score further comprises:

    determining a baseline objective walking quality score of the individual obtained prior to start of a rehabilitation program or immediately following an injury;
    determining, based on the identified changes in gait pattern, an effectiveness of said rehabilitation program.

13. The computer-implemented method of claim 1, wherein said processing is optimized, by the processor, based on a type of assistive device being used by the user.

14. A system for evaluating a user's gait, the system comprising:

    a processor; and
    a memory comprising instructions stored thereon, which when executed by the processor, causes the processor to:

        receive data measured by one or more types of sensors of a pair of smart insoles worn by the user, the smart insoles in communication with the computing device,
        segment the data into gait-related segmented data comprising one or more of: gait cycle segmentation or activity type;
process the gait-related segmented data, via one or more algorithms stored in the memory of the computing device, to determine one or more of:

gait patterns associated with the segmented data;
gait parameters associated with the segmented data;
gait phenotypes associated with the segmented data;

calculate, via one or more algorithms, a composite gait quality score, based on a combination and interaction of at least two of: the segmented data, the gait patterns, the gait parameters, and the gait phenotypes.

15. A non-transitory computer-readable storage medium comprising instructions stored thereon, which when executed by one or more processors, cause the one or more processors to perform operations for evaluating a user's gait, the operations comprising:

receiving data, measured by one or more sensors of a pair of smart insoles worn by the user, the smart insoles in communication with the computing device,
segmenting, by a processor of the computing device, the data into gait-related segmented data comprising one or more of: heel strike, foot on floor, heel raise, toe off data, and activity type;
processing the gait-related segmented data, via one or more algorithms stored in the memory of the computing device, to determine one or more of:

gait patterns associated with the segmented data;
gait parameters associated with the segmented data;
gait phenotypes associated with the segmented data;

calculating, via one or more algorithms a composite gait quality score, based on a combination and interaction of at least two of: the segmented data, the gait patterns, the gait parameters, and the gait phenotypes.

**FIG. 1**

102

230
COLLECTED DATA

232
CLOUD-BASED OPERATIONS

258
OUTPUTS

116

Detect the use of Assistive Devices

126    264

DATABASE
118

242    246

240    244

250

276

234    236

248

238

252

264    268

**FIG. 2**

304

Daily distance
Spatiotemporal variables
Gait velocity
Centre of pressure location
Fall events
Gait deviation %
EDSS score
Ambulatory index
Balance
500m walk completion time/distance to stop

.
.
.

Gait Phenotype

250

276

Composite Score

314

**FIG. 3A**

312

The
Normal
Distribution

Probability

Values

-1.96σ

95% of values

+1.96σ

-2.58σ

99% of values

+2.58σ

Probability of Cases
in portions of the curve

= 0.0013    = 0.0214    = 0.1359    = 0.3413    = 0.3413    = 0.1359    = 0.0214    = 0.0013

Z Scores    -4.0    -3.0    -2.0    -1.0    0    +1.0    +2.0    +3.0    +4.0

**FIG. 3B**

FIG. 4

**FIG. 5**

SENSOR INPUTS
604

GAIT
ALGORITHMS
606

OUTPUTS 608

DATABASE 118

PROGRESSION
ALGORITHMS 610

USER INTERFACE 225

**FIG. 6**

702

Left Foot

**FIG. 7A**

Left Foot

**FIG. 7B**

FIG. 8

Unassisted – Left Circumduction

FIG. 9A

FIG. 9B

## Detect the use of Assistive Devices

**FIG. 10A**

**FIG. 10B**

1102

## Balance Analysis

Centre of pressure location while quietly standing with eyes open and closed

**FIG. 11**

1102

## BALANCE ANALYSIS

Centre of pressure displacement velocity while quietly standing with eyes open and closed

**FIG. 12**

**FIG. 13A**

6DOF Animation (Sample 12765 of 12766)

**FIG. 13B**

**FIG. 14**

FIG. 15

FIG. 16

FIG. 17A

FIG. 17B

**FIG. 17C**

FIG. 18

**FIG. 19**

**FIG. 20A**

**FIG. 20B**

**FIG. 22**

**FIG. 23A**

**FIG. 23B**

**FIG. 23C**

**FIG. 23D**

FIG. 24

FIG. 25

FIG. 26

EP 4 623 819 A1

FIG. 27A

FIG. 27B

FIG. 27C

FIG. 27D

FIG. 27E

FIG. 27F

FIG. 27G

FIG. 27H

76

| | Europäisches Patentamt<br>European Patent Office<br>Office européen des brevets | **PARTIAL EUROPEAN SEARCH REPORT**<br>under Rule 62a and/or 63 of the European Patent Convention.<br>This report shall be considered, for the purposes of<br>subsequent proceedings, as the European search report | **Application Number**<br>EP 25 16 0661 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2024/197383 A1 (UNIV OTTAWA [CA])<br>3 October 2024 (2024-10-03)<br><br>* the whole document *<br>----- | 1-3,<br>6-10,<br>12-15 | INV.<br>A61B5/11<br>A61B5/103<br>A61B5/00 |
| X | WO 2019/213399 A1 (BAYLOR COLLEGE MEDICINE [US]) 7 November 2019 (2019-11-07)<br>* the whole document *<br>* figures 1-12 *<br>* paragraph [0033] - paragraph [0060] *<br>----- | 1-10,<br>12-15 | |
| X | EP 3 468 450 B1 (PORTABILES HEALTHCARE TECH GMBH [DE])<br>15 February 2023 (2023-02-15)<br>* the whole document *<br>* figures 1-7 *<br>* paragraph [0089] - paragraph [0093] *<br>----- | 2-10,<br>12-15 | |
| X | US 2023/397841 A1 (HUANG CHENHUI [JP] ET AL) 14 December 2023 (2023-12-14)<br>* the whole document *<br>* figures 1-31 *<br>* paragraph [0047] - paragraph [0098] *<br>----- | 1-10,<br>12-15 | TECHNICAL FIELDS SEARCHED (IPC)<br><br>A61B |
| | -/-- | | |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

 see sheet C

| Place of search | Date of completion of the search | Examiner |
|---|---|---|
| The Hague | 19 August 2025 | Knoop, Jan |

EPO FORM 1503 03.82 (P04E07)

Europäisches
Patentamt

European
Patent Office

Office européen
des brevets

**PARTIAL EUROPEAN SEARCH REPORT**

Application Number

EP 25 16 0661

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | ZHANG HUANGHE ET AL:  "Accurate Ambulatory Gait Analysis in Walking and Running Using Machine Learning Models", IEEE TRANSACTIONS ON NEURAL SYSTEMS AND REHABILITATION ENGINEERING, IEEE, USA, vol. 28, no. 1, 10 December 2019 (2019-12-10), pages 191-202, XP011766400, ISSN: 1534-4320, DOI: 10.1109/TNSRE.2019.2958679 [retrieved on 2020-01-07] * the whole document * * abstract * * figures 1, 6 * ----- | 1-10, 12-15 | |

**TECHNICAL FIELDS SEARCHED (IPC)**

EPO FORM 1503 03.82 (P04C10)

page 2 of 2

Europäisches
Patentamt

European
Patent Office

Office européen
des brevets

**INCOMPLETE SEARCH**
**SHEET C**

**Application Number**

EP 25 16 0661

Claim(s) not searched:
        11


Reason for the limitation of the search (non-patentable invention(s)):

1.1        Diagnostic method (Art. 53(c) EPC)
The method of claim 11 is not patentable since it is a diagnostic method
within the meaning of Article 53(c) EPC.
1.1.1      According to the decision of the Enlarged Board of Appeal G
1/04 (see also the Guidelines G-II, 4.2 and 4.2.1.3) a method qualifies
as a diagnostic method in the sense of Article 53(c) if the method
comprises the following steps (G 1/04, point 5 of the reasons):
1.2        (a) the examination phase involving the collection of data,
1.3        (b) the comparison of these data with standard values,
1.4        (c) the finding of any significant deviation, i.e. a symptom,
during the comparison, and
1.5        (d) the attribution of the deviation to a particular clinical
picture, i.e. the deductive medical or veterinary decision phase,
1.6        wherein the steps of a technical nature belonging to steps (a)
to (c) must satisfy the criterion "practised on the human or animal body"
(see conclusion, point 3).
1.7        The criterion "practised on the human or animal body" is to be
considered only in respect of method steps which are of a technical
nature (see points 6.4.1 and 6.4.4 of G 1/04). Thus, it neither applies
to the deductive decision phase, nor to the above-mentioned steps b) and
c) which consist in comparing the data collected in the examination phase
with standard values and in finding a significant deviation resulting
from the comparison. These activities are predominantly of a
non-technical nature and normally not practised on the human or animal
body (see point 6.4.1 of G 1/04).
1.7.1      Present claim  11 relates to a method for evaluating a user's
gait.
1.8        Step (a) mentioned as above is disclosed in claim  1 by the
step of "receiving, by a computing device having a memory and a
processor, from a pair of smart insoles communicatively coupled to the
computing device and worn by the user, data measured by one or more types
of sensors;". This step is of technical character since it inherently
involves technical means. Further, this step obviously requires the
presence of the human body, since no information can be obtained
otherwise.
1.9        Step (b) which refers to the comparison of the data collected
with standard values is to be found in the following feature: (Claim  11)
"an assessment of a change in a patient's neurological disease condition
based on measured changes to the patient's gait.".
1.10       Step (c) which refers to the finding of any significant
deviation, i.e. a symptom, during the comparison and step (d) which
refers to the attribution of the deviation to a particular clinical
picture, i.e. the diagnosis  stricto sensu, is to be found in the same
step.
1.10.1     Consequently, the method according to claim  11 includes all
the features of a diagnostic method practised on the human or animal body
as defined in the decision G 1/04. Such a method is exempted from
patentability by Article 53(c) EPC.

Europäisches
Patentamt
European
Patent Office
Office européen
des brevets

**INCOMPLETE SEARCH
SHEET C**

**Application Number**

EP 25 16 0661

- - - - -

Further limitation of the search

Claim(s) searched incompletely:
     1-10, 12-15

Reason for the limitation of the search:

see items 1 and 4 of the European Search Opinion

## ANNEX TO THE EUROPEAN SEARCH REPORT
## ON EUROPEAN PATENT APPLICATION NO.

EP 25 16 0661

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2025

| Patent document cited in search report | | Publication date | Patent family member(s) | | Publication date |
|---|---|---|---|---|---|
| WO 2024197383 | A1 | 03-10-2024 | US | 2024324907 A1 | 03-10-2024 |
| | | | WO | 2024197383 A1 | 03-10-2024 |
| WO 2019213399 | A1 | 07-11-2019 | EP | 3787502 A1 | 10-03-2021 |
| | | | US | 2021236021 A1 | 05-08-2021 |
| | | | WO | 2019213399 A1 | 07-11-2019 |
| EP 3468450 | B1 | 15-02-2023 | EP | 3257437 A1 | 20-12-2017 |
| | | | EP | 3468450 A1 | 17-04-2019 |
| | | | EP | 4197438 A1 | 21-06-2023 |
| | | | US | 2019150793 A1 | 23-05-2019 |
| | | | WO | 2017216103 A1 | 21-12-2017 |
| US 2023397841 | A1 | 14-12-2023 | JP | 2023179872 A | 20-12-2023 |
| | | | US | 2023397841 A1 | 14-12-2023 |
| | | | US | 2024138713 A1 | 02-05-2024 |
| | | | US | 2024172966 A1 | 30-05-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHAH V V.** ; **MCNAMES J** ; **MANCINI M** ; **CARLSON-KUHTA P** ; **SPAIN RI** ; **NUTT JG et al.** Quantity and quality of gait and turning in people with multiple sclerosis, Parkinson's disease and matched controls during daily living.. *J Neurol.*, 2020, vol. 267 (4), 1188-96